(19) Europäisches Patentamt
European Patent Office
Office européen des brevets

(11) EP 1 733 715 B1

(12) FASCICULE DE BREVET EUROPEEN

(45) Date de publication et mention de la délivrance du brevet:
**27.01.2010 Bulletin 2010/04**

(51) Int Cl.:
***A61K 8/49*** *(2006.01)* ***A61Q 5/10*** *(2006.01)*

(21) Numéro de dépôt: **06114655.1**

(22) Date de dépôt: **29.05.2006**

(54) **Composition pour la teinture des fibres kératiniques comprenant un dérivé de diamino-N,N-dihydro-pyrazolone, un coupleur et un colorant direct hétérocyclique**

Mittel zum Färben von Keratinfasern enthaltend ein Diamino-N,N-Dihydropyrazolonderivat, einen Kuppler und einen heterozyklischen Direktfarbstoff

Composition for dyeing keratinic fibres comprising a diamino-N,N-dihydro-pyrazolone derivative, a coupling agent and a heterocyclic direct dye

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **31.05.2005 FR 0551446**

(43) Date de publication de la demande:
**20.12.2006 Bulletin 2006/51**

(73) Titulaire: **L'Oréal**
**75008 Paris (FR)**

(72) Inventeur: **Hercouet, Leïla**
**93360 Neuilly Plaisance (FR)**

(74) Mandataire: **Prevel, Estelle Nicole**
**L'Oréal**
**D.I.P.I.**
**25-29, Quai Aulagnier**
**92665 Asnières-sur-Seine Cedex (FR)**

(56) Documents cités:
**EP-A- 1 437 123**
**EP-A- 1 550 656**
**EP-A- 1 598 047**
**DE-A1- 1 617 893**
**DE-A1- 10 118 271**
**DE-A1- 10 148 847**

EP 1 733 715 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

Printed by Jouve, 75001 PARIS (FR)

## Description

L'invention a pour objet une composition pour la teinture des fibres kératiniques, et en particulier des fibres kératiniques humaines telles que les cheveux, comprenant au moins une base d'oxydation du type diamino-N,N-dihydropyrazolone ou un de ses sels d'addition, au moins un coupleur et au moins un colorant direct hétérocyclique, ainsi que le procédé de coloration mettant en oeuvre une telle composition.

Il est connu de teindre les fibres kératiniques, et en particulier les fibres kératiniques humaines telles que les cheveux, avec des compositions tinctoriales comprenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidine, des dérivés de pyridine, des dérivés d'indole, des dérivés d'indoline appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés ou colorants. On obtient ainsi des colorations permanentes.

On sait également que l'on peut faire varier les nuances obtenues avec ces bases d'oxydation en les associant à des coupleurs ou modificateurs de coloration, ces derniers étant choisis notamment parmi les méta-phénylènediamines, les méta-aminophénols, les méta-hydroxyphénols et certains composés hétérocycliques.

La variété des molécules mises en jeu au niveau des bases d'oxydation et des coupleurs permet l'obtention d'une riche palette de couleurs.

L'utilisation de bases d'oxydation telle que les dérivés de para-phénylènediamine et de para-aminophénol permettent d'obtenir une gamme de couleurs assez large à pH basique sans toutefois atteindre des nuances de bonne chromaticité tout en conférant aux cheveux d'excellentes propriétés d'intensité de couleur, de variété de nuances, d'uniformité de la couleur et de la ténacité aux agents extérieurs.

L'utilisation de ces bases à pH neutre ne permet pas d'atteindre une gamme de nuances variées, en particulier pour les nuances chaudes telles que les rouges et les orangés.

Le but de la présente invention est de fournir de nouvelles compositions de coloration des fibres kératiniques qui permettent d'obtenir une coloration aux nuances variées, en particulier naturelles, puissante, chromatique, esthétique, peu sélective et résistant bien aux diverses agressions que peuvent subir les cheveux tels que les shampooings, la lumière, la sueur et les déformations permanentes.

La présente invention a donc pour objet une composition de coloration des fibres kératiniques comprenant, dans un milieu approprié :

- au moins une base d'oxydation choisie parmi un dérivé de la diamino-N,N-dihydro-pyrazolone de formule (I) ou l'un de ses sels d'addition :

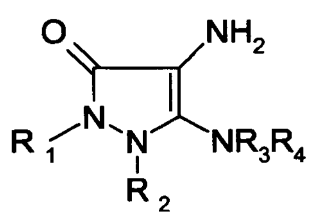

(I)

dans laquelle :

$R_1$, $R_2$, $R_3$ et $R_4$, identiques ou différents, représentent :

- un radical alkyle en $C_1$-$C_{10}$, de préférence en $C_1$-$C_6$, linéaire ou ramifié éventuellement substitué par un ou plusieurs radicaux choisis dans le groupe constitué par un radical $OR_5$, un radical $NR_6R_7$, un radical carboxy, un radical sulfonique, un radical carboxamido $CONR_6R_7$, un radical sulfonamido $SO_2NR_6R_7$, un hétéroaryle, un aryle éventuellement substitué par un ou plusieurs groupes ($C_1$-$C_4$)alkyle, hydroxy, alcoxy en $C_1$-$C_2$, amino, (di)alkyl($C_1$-$C_2$)amino ;
- un radical aryle éventuellement substitué par un ou plusieurs radicaux ($C_1$-$C_4$)alkyle, hydroxy, alcoxy en $C_1$-$C_2$, amino, (di)alkyl($C_1$-$C_2$)amino ;
- un radical hétéroaryle à 5 ou 6 chaînons, éventuellement substitué par un ou plusieurs radicaux choisis

parmi un radical $(C_1-C_4)$alkyle, $(C_1-C_2)$alcoxy ;

$R_3$ et $R_4$ peuvent représenter également un atome d'hydrogène ;

$R_5$, $R_6$ et $R_7$, identiques ou différents, représentent :

- un atome d'hydrogène ;
- un radical alkyle linéaire ou ramifié en $C_1-C_4$ éventuellement substitué par un ou plusieurs radicaux choisis parmi un radical hydroxy, alcoxy en $C_1-C_2$, carboxamido $CONR_8R_9$, sulfonyle $SO_2R_8$, aryle éventuellement substitué par un radical $(C_1-C_4)$alkyle, hydroxy, alcoxy en $C_1-C_2$, amino, (di)alkyl$(C_1-C_2)$amino; aryle éventuellement substitué par un radical $(C_1-C_4)$alkyle, hydroxy, alcoxy en $C_1-C_2$, amino, (di)alkyl$(C_1-C_2)$amino ;

$R_6$ et $R_7$, identiques ou différents, peuvent représenter également un radical carboxamido $CONR_8R_9$; un radical sulfonyle $SO_2R_8$;

$R_8$ et $R_9$, identiques ou différents, représentent un atome d'hydrogène; un radical alkyle en $C_1-C_4$ linéaire ou ramifié éventuellement substitué par un ou plusieurs radicaux hydroxy, alcoxy en $C_1-C_2$ ;

$R_1$ et $R_2$ d'une part, et $R_3$ et $R_4$ d'autre part, peuvent former avec le ou les atomes d'azote auxquels ils sont rattachés un hétérocycle saturé ou insaturé, comportant 5 à 7 chaînons, éventuellement substitué par un ou plusieurs radicaux choisis dans le groupe constitué par les atomes d'halogène, les radicaux amino, (di)alkyl $(C_1-C_4)$amino, hydroxy, carboxy, carboxamido, $(C_1-C_2)$alcoxy, les radicaux alkyle en $C_1-C_4$ éventuellement substitués par un ou plusieurs radicaux hydroxy, amino, (di)-alkylamino, alcoxy, carboxy, sulfonyle ;

$R_3$ et $R_4$ peuvent également former ensemble avec l'atome d'azote auquel ils sont rattachés un hétérocycle à 5 ou 7 chaînons dont les atomes de carbone peuvent être remplacés par un atome d'oxygène ou d'azote éventuellement substitué ; et

- au moins un coupleur ; et
- au moins un colorant direct hétérocyclique.

La présente invention permet en particulier d'obtenir une coloration des fibres kératiniques aux nuances variées, puissante, esthétique, peu sélective et résistant bien aux diverses agressions que peuvent subir les cheveux tels que les shampooings, la lumière, la sueur et les déformations permanentes. Elle permet notamment d'obtenir des nuances naturelles. Elle permet de plus d'obtenir des colorations intenses et variées à pH neutre.

Un autre objet de l'invention est un procédé de teinture des fibres kératiniques mettant en oeuvre la composition de la présente invention, ainsi que l'utilisation de cette composition pour la teinture des fibres kératiniques.

L'invention a enfin pour objet un kit de coloration comprenant d'une part une composition de coloration contenant une base d'oxydation de formule (I), un coupleur et un colorant direct hétérocyclique et d'autre part une composition contenant un agent oxydant.

Dans le cadre de l'invention, on entend par radical alkyle des radicaux alkyle linéaires ou ramifiés en $C_1-C_{10}$ sauf indication contraire, préférentiellement en $C_1-C_6$, encore plus préférentiellement en $C_1-C_4$, tels que le radical méthyle, éthyle, propyle, isopropyle, isobutyle, tertiobutyle, pentyle, hexyle.

Un radical hydroxyalkyle est un alkyle mono ou polysubstitué par un radical hydroxyle, c'est-à-dire un radical alkyle qui peut être substitué par un ou plusieurs radicaux hydroxyle.

Plus particulièrement, dans la formule (I), les radicaux $R_1$ et $R_2$, identiques ou différents, sont choisis parmi :

- un radical alkyle en $C_1-C_6$ éventuellement substitué par un radical hydroxy, $(C_1-C_2)$alcoxy, amino, (di)alkyl$(C_1-C_2)$ amino ;
- un radical phényle, méthoxyphényle, éthoxyphényle, benzyle,.

De préférence, les radicaux $R_1$ et $R_2$, identiques ou non, sont choisis parmi un radical méthyle, éthyle, 2-hydroxyéthyle, 3-hydroxypropyle, 2-hydroxypropyle, phényle.

Selon un autre mode de réalisation, les radicaux $R_1$ et $R_2$ forment ensemble avec les atomes d'azote auxquels ils sont rattachés un cycle à 5 ou 6 chaînons, saturé ou insaturé, éventuellement substitué.

De préférence, les radicaux $R_1$ et $R_2$ forment ensemble avec les atomes d'azote auxquels ils sont rattachés un cycle pyrazolidine, pyridazolidine, éventuellement substitué par un ou plusieurs radicaux alkyle en $C_1-C_4$, hydroxy, $(C_1-C_2)$alcoxy, carboxy, carboxamido, amino, (di)alkyl$(C_1-C_2)$amino.

De manière encore plus avantageuse, les radicaux $R_1$ et $R_2$ forment ensemble avec les atomes d'azote auxquels ils sont rattachés un cycle pyrazolidine, pyridazolidine.

En ce qui concerne les radicaux $R_3$ et $R_4$, ces derniers, identiques ou différents, sont plus particulièrement choisis parmi un atome d'hydrogène ; un radical alkyle en $C_1$-$C_6$ linéaire ou ramifié, éventuellement substitué par un ou plusieurs radicaux hydroxy, ($C_1$-$C_2$)alcoxy, amino, (di)alkyl($C_1$-$C_2$)amino; un radical phényle éventuellement substitué par un ou plusieurs radicaux hydroxy, amino, ($C_1$-$C_2$)alcoxy.

De préférence, les radicaux $R_3$ et $R_4$, identiques ou non, sont choisis parmi un atome d'hydrogène, un radical méthyle, éthyle, isopropyle, 2-hydroxyéthyle, 3-hydroxypropyle, 2-hydroxypropyle, 2-carboxyéthyle. Selon un mode de réalisation particulier, les radicaux $R_3$ et $R_4$ représentent un atome d'hydrogène.

Selon un autre mode de réalisation, les radicaux $R_3$ et $R_4$ forment ensemble avec l'atome d'azote auquel ils sont rattachés un cycle à 5 ou 7 chaînons choisi parmi les hétérocycles pyrrolidine, pipéridine, homopipéridine, pipérazine, homopipérazine ; lesdits cycles pouvant être substitués par un ou plusieurs radicaux hydroxy, amino, (di)alkyl($C_1$-$C_2$) amino, carboxy, carboxamido, alkyle en $C_1$-$C_4$ éventuellement substitué par un ou plusieurs radicaux hydroxy, amino, (di)alkylamino en $C_1$-$C_2$.

Plus particulièrement, les radicaux $R_3$ et $R_4$ forment ensemble avec l'atome d'azote auquel ils sont rattachés un cycle à 5 ou 7 chaînons choisi parmi la pyrrolidine, le 2,5-diméthylpyrrolidine, l'acide pyrrolidine-2-carboxylique, l'acide 3-hydroxypyrrolidine-2-carboxylique, l'acide 4-hydroxypyrrolidine-2-carboxylique, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 3-diméthylamino-pyrrolidine, la 4-amino-3-hydroxy pyrrolidine, la 3-hydroxy-4-(2-hydroxyéthyl)amino- pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, la homopipéridine, la 2-carboxyhomopipéridine, la 2-carboxamidohomopipéridine, l'homopipérazine, le N-méthyl-homopipérazine, le N-(2-hydroxyéthyl)-homopipérazine.

De préférence, les radicaux $R_3$ et $R_4$ forment ensemble avec l'atome d'azote auquel ils sont rattachés un cycle à 5 ou 7 chaînons choisi parmi la pyrrolidine, la 3-hydroxypyrrolidine, la 3-aminopyrrolidine, la 3-diméthylamino-pyrrolidine, l'acide pyrrolidine-2-carboxylique, l'acide 3-hydroxypyrrolidine-2-carboxylique, la pipéridine, l'hydroxypipéridine, l'homopipéridine, le diazépane, la N-méthyl homopipérazine, la N β-hydroxyéthylhomopipérazine.

Conformément à un mode de réalisation encore plus préféré de l'invention, les radicaux $R_3$ et $R_4$ forment ensemble avec l'atome d'azote auquel ils sont rattachés un cycle à 5 chaînons tels que la pyrrolidine, la 3-hydroxypyrrolidine, la 3-aminopyrrolidine, la 3-diméthylamino-pyrrolidine.

Les composés de formule (I) peuvent être éventuellement salifiés par des acides minéraux forts tels que par exemple HCl, HBr, HI, $H_2SO_4$, $H_3PO_4$, ou des acides organiques tels que, par exemple, l'acide acétique, lactique, tartrique, citrique ou succinique, benzènesulfonique, para-toluènesulfonique, formique, méthanesulfonique.

Ils peuvent aussi être sous forme de solvates par exemple un hydrate ou un solvate d'alcool linéaire ou ramifié tel que l'éthanol ou l'isopropanol.

A titre d'exemples de dérivés de formule (I), on peut citer les composés présentés ci-dessous ou leurs sels d'addition :

4,5-diamino-1,2-diméthyl-1,2-dihydro-pyrazol-3-one ;
4-amino-5-méthylamino-1,2-diméthyl-1,2-dihydro-pyrazol-3-one ;
4-amino-5-diméthylamino-1,2-diméthyl-1,2-dihydro-pyrazol-3-one ;
4-amino-5-(2-hydroxyéthyl)amino-1,2-diméthyl-1,2-dihydro-pyrazol-3-one ;
4-amino-5-(pyrrolidin-1-yl)-1,2-diméthyl-1,2-dihydro-pyrazol-3-one ;
4-amino-5-(pipéridin-1-yl)-1,2-diméthyl-1,2-dihydro-pyrazol-3-one ;

4,5-diamino-1,2-di-(2-hydroxyéthyl)-1,2-dihydro-pyrazol-3-one ;
4-amino-5-méthylamino-1,2-di-(2-hydroxyéthyl)-1,2-dihydro-pyrazol-3-one ;
4-amino-5-diméthylamino-1,2-di-(2-hydroxyéthyl)-1,2-dihydro-pyrazol-3-one ;
4-amino-5-(2-hydroxyéthyl)amino-1,2-di-(2-hydroxyéthyl)-1,2-dihydro-pyrazol-3-one ;
4-amino-5-(pyrrolidin-1-yl)-1,2-di-(2-hydroxyéthyl)-1,2-dihydro-pyrazol-3-one ;
4-amino-5-(pipéridin-1-yl)-1,2-di-(2-hydroxyéthyl)-1,2-dihydro-pyrazol-3-one ;

4,5-diamino-1,2-diéthyl-1,2-dihydro-pyrazol-3-one ;
4,5-diamino-1,2-phényl-1,2-dihydro-pyrazol-3-one ;
4,5-diamino-1-éthyl-2-méthyl-1,2-dihydro-pyrazol-3-one ;
4,5-diamino-2-éthyl-1-méthyl-1,2-dihydro-pyrazol-3-one ;

4,5-diamino-1-phényl-2-méthyl-1,2-dihydro-pyrazol-3-one ;
4,5-diamino-2-phényl-1-méthyl-1,2-dihydro-pyrazol-3-one ;
4,5-diamino-1-(2-hydroxyéthyl)-2-méthyl-1,2-dihydro-pyrazol-3-one ;
4,5-diamino-2-(2-hydroxyéthyl)-1-méthyl-1,2-dihydro-pyrazol-3-one ;

2,3-diamino-6,7-dihydro-1H,5H-pyrazolo[1,2-a]pyrazol-1-one ;
2-amino-3-méthylamino-6,7-dihydro-1H,5H-pyrazolo[1,2-a]pyrazol-1-one ;
2-amino-3-diméthylamino-6,7-dihydro-1H,5H-pyrazolo[1,2-a]pyrazol-1-one ;
2-amino-3-éthylamino-6,7-dihydro-1H,5H-pyrazolo[1,2-a]pyrazol-1-one ;
2-amino-3-isopropylamino-6,7-dihydro-1H,5H-pyrazolo[1,2-a]pyrazol-1-one ;
2-amino-3-(2-hydroxyéthyl)amino-6,7-dihydro-1H,5H-pyrazolo[1,2-a]pyrazol-1-one ;
2-amino-3-(2-hydroxypropyl)amino-6,7-dihydro-H,5H-pyrazolo[1,2-a]pyrazol-1-one ;
2-amino-3-bis(2-hydroxyéthyl)amino-6,7-dihydro-1H,5H-pyrazolo[1,2-a]pyrazol-1-one ;

2-amino-3-(pyrrolidin-1-yl)-6,7-dihydro-1H,5H-pyrazolo[1,2-a]pyrazol-1-one ;
2-amino-3-(3-hydroxy-pyrrolidin-1-yl)-6,7-dihydro-1H,5H-pyrazolo[1,2-a]pyrazol-1-one ;
2-amino-3-(pipéridin-1-yl)-6,7-dihydro-1H,5H-pyrazolo[1,2-a]pyrazol-1-one ;

2,3-diamino-6-hydroxy-6,7-dihydro-1H,5H-pyrazolo[1,2-a]pyrazol-1-one;
2,3-diamino-6-méthyl-6,7-dihydro-1H,5H-pyrazolo[1,2-a]pyrazol-1-one ;
2,3-diamino-6-diméthyl-6,7-dihydro-1H,5H-pyrazolo[1,2-a]pyrazol-1-one ;

2,3-diamino-5,6,7,8-tétrahydro-1H,6H-pyridazino[1,2-a]pyrazol-1-one ;
2,3-diamino-5,8-dihydro-1H,6H-pyridazino[1,2-a]pyrazol-1-one ;

4-Amino-5-dimethylamino-1,2-diethyl-1,2-dihydro-pyrazol-3-one ;
4-Amino-1,2-diethyl-5-ethylamino-1,2-dihydro-pyrazol-3-one ;
4-Amino-1,2-diethyl-5-isopropylamino-1,2-dihydro-pyrazol-3-one ;
4-Amino-1,2-diethyl-5-(2-hydroxy-ethylamino)-1,2-dihydro-pyrazol-3-one ;
4-Amino-5-(2-dimethylamino-ethylamino)-1,2-diethyl-1,2-dihydro-pyrazol-3-one ;
4-Amino-5-[bis-(2-hydroxy-ethyl)-amino]-1,2-diethyl-1,2-dihydro-pyrazol-3-one ;
4-Amino-1,2-diethyl-5-(3-imidazol-1-yl-propylamino)-1,2-dihydro-pyrazol-3-one ;
4-Amino-5-dimethylamino-1,2-diethyl-1,2-dihydro-pyrazol-3-one ;
4-Amino-1,2-diethyl-5-ethylamino-1,2-dihydro-pyrazol-3-one ;
4-Amino-1,2-diethyl-5-isopropylamino-1,2-dihydro-pyrazol-3-one ;
4-Amino-1,2-diethyl-5-(2-hydroxy-ethylamino)-1,2-dihydro-pyrazol-3-one ;
4-Amino-5-(2-dimethylamino-ethylamino)-1,2-diethyl-1,2-dihydro-pyrazol-3-one ;
4-Amino-5-[bis-(2-hydroxy-ethyl)-amino]-1,2-diethyl-1,2-dihydro-pyrazol-3-one ;
4-Amino-1,2-diethyl-5-(3-imidazol-1-yl-propylamino)-1,2-dihydro-pyrazol-3-one ;
4-Amino-1.2-diethyl-5-(3-hydroxy-pyrrolidin-1-yl)-1,2-dihydro-pyrazol-3-one ;
4-Amino-1.2-diethyl-5-pyrrolidin-1-yl-1,2-dihydro-pyrazol-3-one ;
4-Amino-5-(3-dimethylamino-pyrrolidin-1-yl)-1,2-diethyl-1,2-dihydro-pyrazol-3-one ;
4-Amino-1,2-diethyl-5-(4-methyl-piperazin-1-yl)-pyrazolidin-3-one ;
2,3-Diamino-6-hydroxy-6,7-dihydro-5H-pyrazolo[1,2-a]pyrazol-1-one ;

dont certains sont figurés ci-dessous pour illustrer les noms par des structures chimiques :

| | |
|---|---|
| 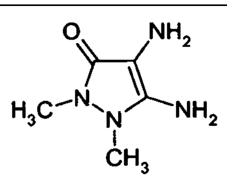 | 4,5-Diamino-1,2-diméthyl-1,2-dihydro-pyrazol-3-one |

(suite)

| | |
|---|---|
| 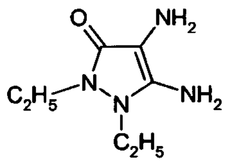 | 4,5-Diamino-1,2-diéthyl-1,2-dihydro -pyrazol-3-one |
| 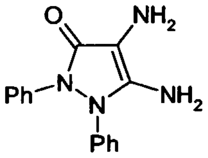 | 4,5-Diamino-1,2-diphényl-1,2-dihydro-pyrazol-3-one |
| 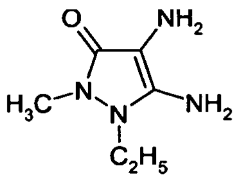 | 4,5-Diamino-1-éthyl-2-méthyi-1,2-dihydro-pyrazol-3-one |
| 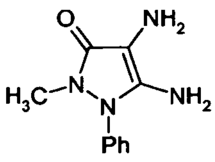 | 4,5-Diamino-1-phényl-2-méthyl-1,2-dihydro-pyrazol-3-one |
| 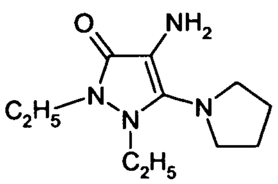 | 4-Amino-5-(pyrrolidin-1-yl)-1,2-diethyl-1,2-dihydro-pyrazol-3-one |
| 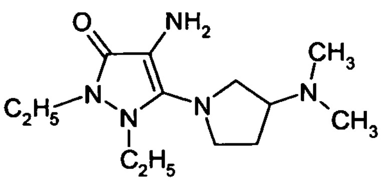 | 4-Amino-5-(3-dimethylamino-pyrrolidin-1-yl)-1,2-diethyl-1,2-dihydro-pyrazol-3-one |
| 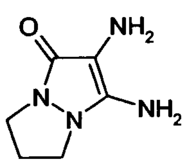 | 2,3-diamino-6,7-dihydro-1H,5H-pyrazolo[1,2-a]pyrazol-1-one |
| 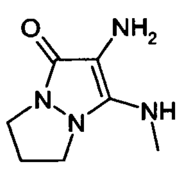 | 2-Amino-3-méthylamino-6,7-dihydro-1H,5H-pyrazolo[1,2-a]pyrazol-1-one |

(suite)

| | |
|---|---|
| 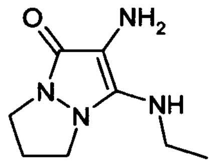 | 2-Amino-3-éthylamino-6,7-dihydro-1H,5H-pyrazolo[1,2-a]pyrazol-1-one |
| 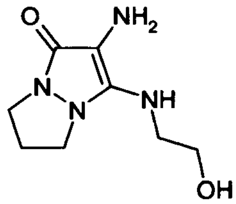 | 2-amino-3-(2-hydroxyéthyl)amino-6,7-dihydro-1H,5H-pyrazolo[1,2-a] pyrazol-1-one |
| 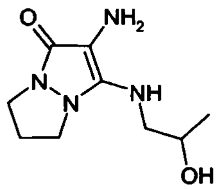 | 2-amino-3-(2-hydroxypropyl)amino-6,7-dihydro-1H,5H-pyrazolo[1,2-a] pyrazol-1-one |
| 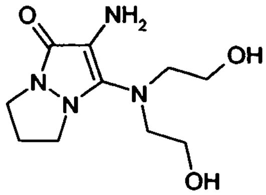 | 2-amino-3-bis(2-hydroxyéthyl)amino-6,7-dihydro-1H,5H-pyrazolo[1,2-a] pyrazol-1-one |
| 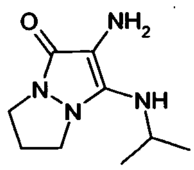 | 2-amino-3-isopropylamino-6,7-dihydro-1H,5H-pyrazolo[1,2-a]pyrazol-1-one |
| 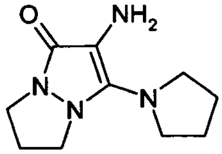 | 2-amino-3-(pyrrolidin-1-yl)-6,7-dihydro-1H,5H-pyrazolo[1,2-a]pyrazol-1-one |
| 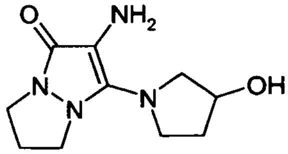 | 2-amino-3-(3-hydroxy-pyrrolidin-1-yl)-6,7-dihydro-1H,5H-pyrazolo[1,2-a] pyrazol-1-one |

(suite)

| | |
|---|---|
| 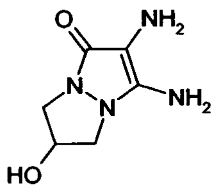 | 2,3-diamino-6-hydroxy-6,7-dihydro-1H,5H-pyrazolo[1,2-a]pyrazol-1-one |
| 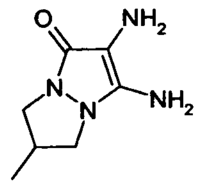 | 2,3-diamino-6-méthyl-6,7-dihydro-1H,5H-pyrazolo[1,2-a]pyrazol-1-one |
| 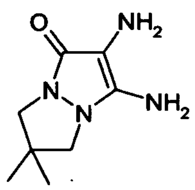 | 2,3-diamino-6,6-diméthyl-6,7-dihydro-1H,5H-pyrazolo[1,2-a]pyrazol-1-one |
| 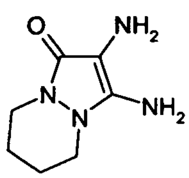 | 2,3-diamino-5,6,7,8-tétrahydro-1H,6H-pyridazino[1,2-a]pyrazol-1-one |
| 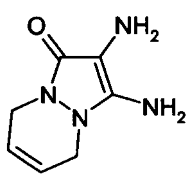 | 2,3-diamino-5,8-dihydro-1H,6H-pyridazino[1,2-a]pyrazol-1-one |
| 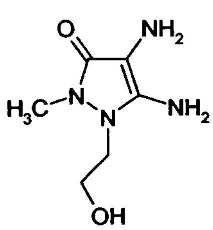 | 4,5-diamino-1-(2-hydroxyéthyl)-2-méthyl-1,2-dihydro-pyrazol-3-one |
| 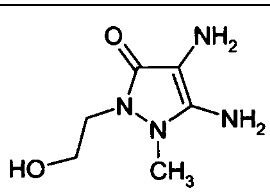 | 4,5-diamino-2-(2-hydroxyéthyl)-1-méthyl-1,2-dihydro-pyrazol-3-one. |

Parmi ces composés, les dérivés de diamino-N,N-dihydropyrazolone de formule (I) ou leurs sels d'addition particulièrement préférés sont les suivants:

2,3-Diamino-6,7-dihydro-1H,5H-pyrazolo[1,2-a]pyrazol-1-one ;

2-Amino-3-éthylamino-6,7-dihydro-1H,5H-pyrazolo[1,2-a]pyrazol-1-one ;
2-Amino-3-isopropylamino-6,7-dihydro-1H,5H-pyrazolo[1,2-a]pyrazol-1-one ;
2-Amino-3-(pyrrolidin-1-yl)-6,7-dihydro-1H,5H-pyrazolo[1,2-a]pyrazol-1-one ;
4,5-diamino-1,2-diméthyl-1,2-dihydro-pyrazol-3-one ;
4,5-diamino-1,2-diéthyl-1,2-dihydro-pyrazol-3-one ;
4,5-diamino-1,2-di-(2-hydroxyéthyl)-1,2-dihydro-pyrazol-3-one ;
2-amino-3-(2-hydroxyéthyl)amino-6,7-dihydro-1H,5H-pyrazolo[1,2-a]pyrazol-1-one ;
2-amino-3-diméthylamino-6,7-dihydro-1H,5H-pyrazolo[1,2-a]pyrazol-1-one ;
2,3-diamino-5,6,7,8-tétrahydro-1H,6H-pyridazino[1,2-a]pyrazol-1-one
4-Amino-1.2-diethyl-5-pyrrolidin-1-yl-1,2-dihydro-pyrazol-3-one ;
4-Amino-5-(3-dimethylamino-pyrrolidin-1-yl)-1,2-diethyl-1,2-dihydro-pyrazol-3-one ;
2,3-diamino-6-hydroxy-6,7-dihydro-1H,5H-pyrazolo[1,2-a]pyrazol-1-one.

La ou les bases d'oxydation de formule (I) sont en général présentes chacune en quantité comprise entre 0,001 et 10 % en poids environ du poids total de la composition tinctoriale, de préférence entre 0,005 et 6 %.

Le ou les coupleurs utiles dans le cadre de l'invention peuvent être choisis parmi les méta-phénylènediamines, les méta-aminophénols, les méta-diphénols, les coupleurs naphtaléniques, les coupleurs hétérocycliques et leurs sels d'addition.

A titre d'exemples, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont en général chacun présents en quantité comprise entre 0,001 et 10 % en poids environ du poids total de la composition tinctoriale, de préférence entre 0,005 et 6 %.

Au sens de la présente invention on entend par colorant direct hétérocyclique tout colorant direct présentant dans sa structure au moins un cycle saturé ou insaturé porteur d'au moins un hétéroatome choisi parmi oxygène, soufre, azote et phosphore, éventuellement condensé avec un cycle hydrocarboné saturé ou insaturé. Cet hétérocycle peut être substitué ou non et peut être chargé ou non chargé et comporter ou non un ou plusieurs groupements carbonyle.

A titre d'exemples de tels hétérocycles on peut citer les cycles thiophène, thianthrène, furane, 1-4 pyrane, 1-2 pyrane, isobenzofurane, chromène, xanthène, 2Hpyrrole, pyrrole, imidazole, pyrazole, isothiazole, isoxazole, pyridine, pyrazine, pyrimidine, pyridazine , indolizine, isoindole, 3Hindole, indole, 1Hindazole, purine, 4Hquinolizine, isoquinoline, quinoline, phtalazine, naphtyridine, quinoxaline, cinnoline, ptéridine, carbazole, 4aHcarbazole , carboline, phénanthridine, acridine, périmidine, phénanthroline, phénazine, phénothiazine, furazane, phénoxazine, phenoxathiine, pyrrolidine, isochromane, chromane, pyrroline, imidazoline, imidazolidine, pyrazoline, pyrazolidine, morpholine, benzisoquinoline, imidazothiazole, benzothiazole, benzofurane, 1-2-3 triazole, 1-2-4 triazole, isoazole, 1-4 oxazine, o ou p isoxazine, 1-2-5 oxathiazine, 1-2-6 oxathiazine, 1-4-2 oxadiazine, 1-3-5-2 oxadiazine, 3 isopyrrole, indène, isoindène, indoline, isoindoline, 1-2-3-4 oxatriazole, 1-2-3-5 oxatriazole, pipérazine, pipéridine, 1-3-5 triazine, 1-2-4 triazine, 1-2-3 triazine, 1-3-2 benzoxazine, 1-4-2 benzoxazine, isocoumarine, 1-2-3 dioxazole, 1-2-4 dioxazole, 1-3-2 dioxazole, 1-3-4 dioxazole, 1-2-5 oxathiazole, 1-3 oxathiole, 1-2-3-4 tétrahydro quinoxaline, quinazoline, pyrazolotriazole, thiazole, indolénine, et leurs homologues incluant un ou plusieurs groupements carbonyle comme les cycles pyrazolone, indolinedione,1-2 pyrone, 1-4 pyrone, quinolinone. Ces cycles peuvent être éventuellement substitués par un ou plusieurs substituants. De manière non limitative, le ou les substituants peuvent être choisis parmi les radicaux alkyle linéaire ou ramifié en $C_1$-$C_{10}$, amino, hydroxy, halogène, alcényle linéaire ou ramifié en $C_2$-$C_{10}$, mono ou polyhydroxyalkyle en $C_1$-$C_{10}$, mono ou polyaminoalkyle en $C_1$-$C_{10}$, mono ou dialkyl($C_1$-$C_6$)amino, mono ou dihydroxyalkyl($C_1$-$C_6$)amino, monoalkyl($C_1$-$C_6$) monohydroxyalkyl($C_1$-$C_6$)amino, mono ou dialkyl($C_1$-$C_6$)aminoalkyle($C_1$-$C_{10}$), mono ou dihydroxyalkyl($C_1$-$C_6$)aminoalkyle($C_1$-$C_{10}$), monoalkyl($C_1$-$C_6$)monohydroxyalkyl($C_1$-$C_6$)aminoalkyle($C_1$-$C_{10}$), nitro, carboxyle, carboxyalkyle($C_1$-$C_{10}$), alcoxy($C_1$-$C_6$)carbonyle, sulfo, sulfoalkyle($C_1$-$C_{10}$), alcoxy($C_1$-$C_{10}$), uréido, trialkyl($C_1$-$C_6$)ammonium, trialkyl($C_1$-$C_6$)ammoniumalkyle($C_1$-$C_{10}$), aryle($C_6$-$C_{30}$) éventuellement substitué.

Ces colorants directs hétérocycliques peuvent être sous forme de monomères ou être intégrés dans les motifs répétitifs de polymères de nature non ionique, mono ou polyanionique, mono ou polycationique.

Les colorants hétérocycliques de l'invention peuvent être neutres, acides ou basiques, la portion organique porteuse du chromophore étant neutre ou porteuse d'une charge globale négative ou positive.

Selon un mode de réalisation particulier de l'invention, le ou les colorants directs hétérocycliques neutres sont choisis parmi ceux comportant au moins un cycle choisi parmi les cycles pyridine, quinoxaline, pyrazoline, pyrazole,

oxadiazole, thiazole, pyrrole, indole, pyrazolotriazole, quinoline, indoline, phénazine, coumarine, benzopyrane et leurs homologues incluant un ou plusieurs groupements carbonyles.

A titre d'exemples de colorants hétérocycliques neutres selon l'invention on peut citer la 2,5 diamino 6 nitropyridine, la 5 amino 2(2'hydroxyéthyl)amino 6 nitropyridine, la 2 amino 5(2'hydroxyéthyl)amino 6 nitropyridine, la 5 amino 2 éthylamino 6 nitropyridine, la 2 éthylamino 5 (2'hydroxyéthyl)amino 6 nitropyridine, la 2 méthylamino 5(2'hydroxyéthyl) amino 6 nitropyridine, les nitroquinoxalines décrites dans les demandes DE3825212 et DE4404564 et en particulier la 1,2,3,4 tétrahydro 6 nitroquinoxaline, les composés pyridiniques décrits dans la demande FR2845387, les composés à cycles pyrazolines décrits dans la demande FR2746392, les composés à cycles pyrazoles décrits dans la demande DE19730412, les composés à cycles oxadiazoles décrits dans la demande WO200222093, les composés à cycles thiazoles décrits dans la demande DE10118271, les composés à cycles pyrroles décrits dans la demande FR2760010, les composés à cycles indoles décrits dans la demande FR2978931, les composés à cycles pyrazolotriazoles décrits dans la demande DE20104441, les composés à cycles quinolines et quinoxalines décrits dans la demande EP984010, la 2,3 indolinedione, le Vat Blue 6 (phénazine), le Disperse Yellow 184 (coumarine), la brasiline et l'hématoxyline (benzopyranes).

Selon un autre mode de réalisation particulier de l'invention, le ou les colorants directs hétérocycliques anionique sont choisis parmi ceux comportant au moins un cycle pyrazole, xanthène ,quinoline, benzotriazole, benzoquinoline, indoline, naphtotriazole, et leurs homologues incluant un ou plusieurs groupements carbonyle.

A titre d'exemples de colorants hétérocycliques anioniques selon l'invention on peut citer, l'Acid Yellow 23 (composé à cycle pyrazolone), l'Acid Yellow 73 (composé à cycle xanthène), l'Acid Red 92 (composé à cycle xanthène), l'Acid Yellow 3 (composé à cycle quinoline), le Food Yellow 4 (composé à cycle pyrazolone), l'Acid Red 51 (composé à cycle benzothiazole), l'Acid Red 52 (composé à cycle xanthène), l'Acid Red 87(composé à cycle benzoquinoline), l'Acid Red 95 (composé à cycle xanthène), l'Acid Red 92 (composé à cycle xanthène), l'Acid Blue 74 (composé à cycle indolinone), l'Acid Red 195 (composé à cycle pyrazole), l'Acid Orange 92 (composé à cycle pyrazolone), l'Acid Yellow 5 (composé à cycle benzoquinoline), l'Acid Black 70 (composé à cycle quinolinone), le Direct Yellow 106 (composé à cycle naphtotriazole), le Direct Yellow 59 (composé à cycle benzothiazole), l'Acid Yellow 14 (composé à cycle pyrazolone).

Selon un autre mode de réalisation particulier de l'invention, le ou les colorants directs hétérocycliques cationiques possèdent au moins une charge cationique appartenant à un hétérocycle.

Selon un autre mode de réalisation particulier de l'invention, le ou les colorants directs hétérocycliques sont choisi parmis les colorants cationiques à cycles xanthèniques, les colorants cationiques à cycles acridiniques, les colorants cationiques à cycles benzothiazoles, les colorants cationiques à cycles phénothiazines, les colorants cationiques à cycles pyrazoles, les colorants cationiques à cycles triazoles, les colorants cationiques à cycles thiazoles, les colorants cationiques à cycles phénazines, les colorants cationiques à cycles indolénines, les colorants cationiques à cycles phénoxazines, les colorants cationiques à cycles imidazoles, les colorants cationiques à cycles pyridines et leurs homologues incluant un ou plusieurs groupements carbonyle.

A titre d'exemples de colorants hétérocycliques cationiques selon l'invention on peut citer les colorants suivants :

- les colorants cationiques à cycles xanthèniques comme le Basic Red 1, le Basic Red 3, le Basic Red 4, le Basic Violet 10 et le Basic Violet 11
- les colorants cationiques à cycles acridiniques comme le Basic Orange 15, le Basic Orange 16, le Basic Orange 17.
- les colorants cationiques à cycles benzothiazoles comme le Basic Blue 41 et le Basic Blue 67.
- les colorants cationiques à cycles phénothiazines comme le Basic Blue 9.
- les colorants cationiques à cycles pyrazolones comme le Basic Yellow 57.
- les colorants cationiques à cycles triazoles comme le Basic Red 22 et le Basic Red 46.
- les colorants cationiques à cycles thiazoles comme le Basic Red 29.
- les colorants cationiques à cycles phénazines comme le Basic Red 2.
- les colorants cationiques à cycles indolénines comme le Basic Red 14, le Basic Yellow 13 , le Basic Yellow 28 et le Basic Yellow 29.
- les colorants cationiques à cycles phénoxazines comme le Basic Blue 6.

ainsi que les colorants décrits dans la demande de brevet EP 1 025 834 suivants :

• De formule (II)

$$G\text{-}N\text{=}N\text{-}J \qquad (II)$$

dans laquelle :

le symbole G représente un groupement choisi parmi les structures $G_1$ à $G_3$ suivantes :

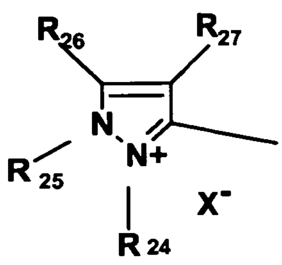

$G_1$

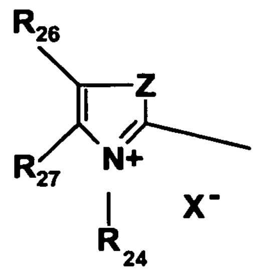

$G_2$

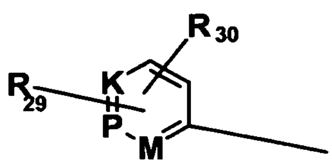

$G_3$

dans lesquelles :

$R_{24}$ désigne un radical alkyle en $C_1$-$C_4$, un radical phényle pouvant être substitué par un radical alkyle en $C_1$-$C_4$ ou un atome d'halogène choisi parmi le chlore, le brome, l'iode et le fluor;
$R_{25}$ désigne un radical alkyle en $C_1$-$C_4$ ou un radical phényle ;
$R_{26}$ et $R_{27}$, identiques ou différents, représentent un radical alkyle en $C_1$-$C_4$, un radical phényle, ou forment ensemble dans $G_1$ un cycle benzénique substitué par un ou plusieurs radicaux alkyle en $C_1$-$C_4$, alcoxy en $C_1$-$C_4$, ou $NO_2$, ou forment ensemble dans $G_2$ un cycle benzénique éventuellement substitué par un ou plusieurs radicaux alkyle en $C_1$-$C_4$, alcoxy en $C_1$-$C_4$, ou $NO_2$ ;
$R_{26}$ peut désigner en outre un atome d'hydrogène ;
Z désigne un atome d'oxygène, de soufre ou un groupement $-NR_{25}$ ;
M représente un groupement -CH, -CR (R désignant alkyle en $C_1$-$C_4$), ou $-NR_{28}(X^-)_r$ ;
K représente un groupement -CH, -CR (R désignant alkyle en $C_1$-$C_4$), ou $-NR_{28}(X^-)_r$ ;
P représente un groupement -CH, -CR (R désignant alkyle en $C_1$-$C_4$), ou $-NR_{28}(X^-)_r$ ;
r désigne zéro ou 1 ;
$R_{28}$ représente un atome $O^-$, un radical alcoxy en $C_1$-$C_4$, ou un radical alkyle en $C_1$-$C_4$ ;
$R_{29}$ et $R_{30}$, identiques ou différents, représentent un atome d'hydrogène ou d'halogène choisi parmi le chlore, le brome, l'iode et le fluor, un radical alkyle en $C_1$-$C_4$, alcoxy en $C_1$-$C_4$, un radical $-NO_2$ ;
$X^-$ représente un anion de préférence choisi parmi le chlorure, l'iodure, le méthyl sulfate, l'éthyl sulfate, l'acétate et le perchlorate ;

le symbole J représente :

- (a) un groupement de structure $J_1$ suivante :

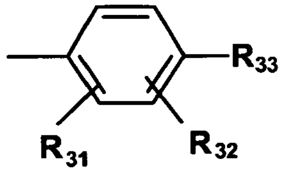

$J_1$

dans laquelle :

$R_{31}$ représente un atome d'hydrogène, un atome d'halogène choisi parmi le chlore, le brome, l'iode et le fluor, un radical alkyle en $C_1$-$C_4$, alcoxy en $C_1$-$C_4$, un radical -OH, - $NO_2$, -$NHR_{34}$, -$NR_{35}R_{36}$, -NHCOalkyle en $C_1$-$C_4$, ou forme avec $R_{32}$ un cycle à 5 ou 6 chaînons contenant ou non un ou plusieurs hétéroatomes choisis parmi l'azote, l'oxygène ou le soufre ;
$R_{32}$ représente un atome d'hydrogène, un atome d'halogène choisi parmi le chlore, le brome, l'iode et le fluor, un radical alkyle en $C_1$-$C_4$, alcoxy en $C_1$-$C_4$ ;
ou forme avec $R_{33}$ ou $R_{34}$ un cycle à 5 ou 6 chaînons contenant ou non un ou plusieurs hétéroatomes choisis parmi l'azote, l'oxygène ou le soufre ;
$R_{33}$ représente un atome d'hydrogène, un radical -OH, un radical -$NHR_{34}$, un radical - $NR_{35}R_{36}$ ;
$R_{34}$ représente un atome d'hydrogène, un radical alkyle en $C_1$-$C_4$, un radical monohydroxyalkyle en $C_1$-$C_4$, polyhydroxyalkyle en $C_2$-$C_4$, un radical phényle ;
$R_{35}$ et $R_{36}$, identiques ou différents, représentent un radical alkyle en $C_1$-$C_4$, un radical monohydroxyalkyle en $C_1$-$C_4$, polyhydroxyalkyle en $C_2$-$C_4$ ;

- (b) un groupement hétérocyclique azoté à 5 ou 6 chaînons susceptible de renfermer d'autres hétéroatomes et / ou des groupements carbonylés et pouvant être substitué par un ou plusieurs radicaux alkyle en $C_1$-$C_4$, amino ou phényle,
et notamment un groupement de structure $J_2$ suivante :

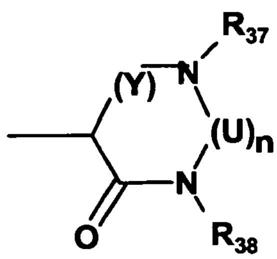

$J_2$

dans laquelle :

$R_{37}$ et $R_{38}$, identiques ou différents, représentent un atome d'hydrogène, un radical alkyle en $C_{13}$-$C_{10}$, un radical phényle ;
Y désigne le radical -CO- ou le radical -$C(CH_3)$= ;
n représente 0 ou 1, avec, lorsque n désigne 1, U désigne le radical -CO- ;
au moins un des K,P,M désignant $NR_{28}(X^-)_r$ si J désigne $J_1$.

- de formule (III) suivante :

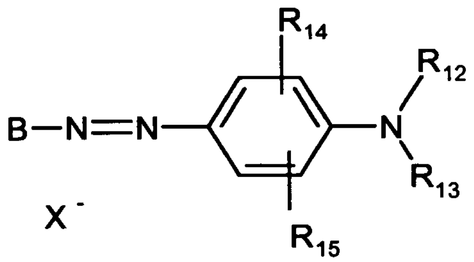

(III)

dans laquelle :

$R_{12}$ représente un atome d'hydrogène ou un radical alkyle en $C_1$-$C_4$,
$R_{13}$ représente un atome d'hydrogène, un radical alkyle pouvant être substitué par un radical -CN ou par un groupement amino, un radical 4'-aminophényle ou forme avec $R_{12}$ un hétérocycle éventuellement oxygéné et / ou azoté pouvant être substitué par un radical alkyle en $C_1$-$C_4$,
$R_{14}$ et $R_{15}$, identiques ou différents, représentent un atome d'hydrogène, un atome d'halogène tel que le brome, le chlore, l'iode ou le fluor, un radical alkyle en $C_1$-$C_4$ ou alcoxy en $C_1$-$C_4$, un radical -CN,
$X^-$ représente un anion de préférence choisi parmi le chlorure, le méthyl sulfate et l'acétate,
B représente un groupement choisi par les structures B1 à B6 suivantes :

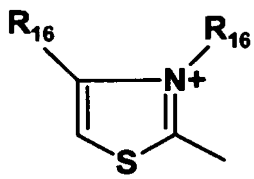

;

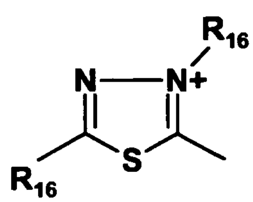

;

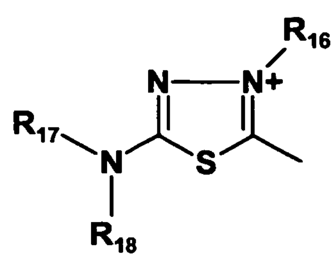

;

B1 B2 B3

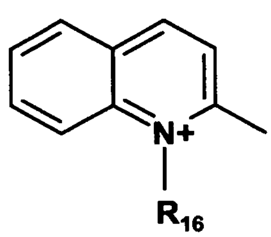

;

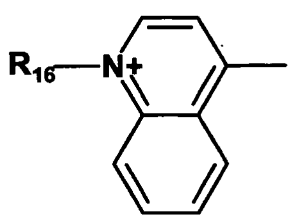

et

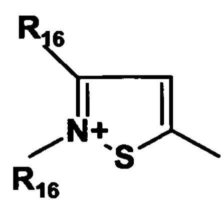

;

B4 B5 B6

dans lesquelles $R_{16}$ représente un radical alkyle en $C_1$-$C_4$,
$R_{17}$ et $R_{18}$, identiques ou différents, représentent un atome d'hydrogène ou un radical alkyle en $C_1$-$C_4$,

- les composés de formules (IV) et (V) suivantes :

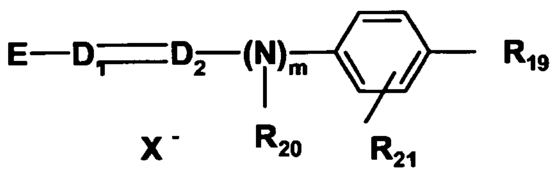

(IV)

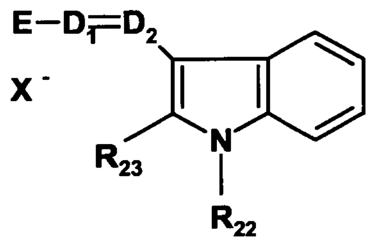

(V)

dans lesquelles :

$R_{19}$ représente un atome d'hydrogène, un radical alcoxy en $C_1$-$C_4$, un atome d'halogène tel que le brome, le chlore, l'iode ou le fluor ou un radical amino,
$R_{20}$ représente un atome d'hydrogène, un radical alkyle en $C_1$-$C_4$ ou forme avec un atome de carbone du cycle benzénique un hétérocycle éventuellement oxygéné et /ou substitué par un ou plusieurs groupements alkyle en $C_1$-$C_4$,
$R_{21}$ représente un atome d'hydrogène ou d'halogène tel que le brome, le chlore, l'iode ou le fluor,
$R_{22}$ et $R_{23}$, identiques ou différents, représentent un atome d'hydrogène ou un radical alkyle en $C_1$-$C_4$,
$D_1$ et $D_2$, identiques ou différents, représentent un atome d'azote ou le groupement - CH,
m représente 0 ou 1,
étant entendu que lorsque $R_{19}$ représente un groupement amino non substitué, alors $D_1$ et $D_2$ représentent simultanément un groupement -CH et m = 0,
$X^-$ représente un anion de préférence choisi parmi le chlorure, le méthyl sulfate et l'acétate,
E représente un groupement choisi par les structures E1 à E8 suivantes :

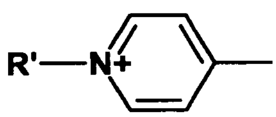

E1

;

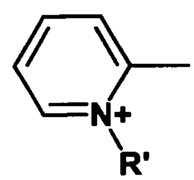

E2

;

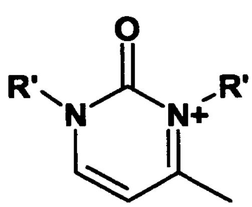

E3

;

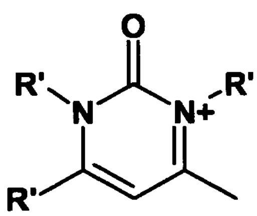

E4

;

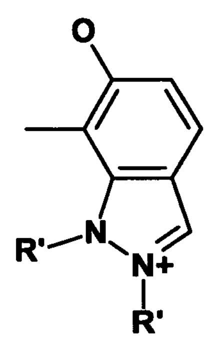

E5

;

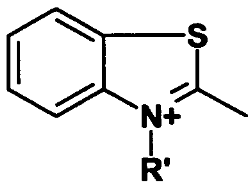 ; 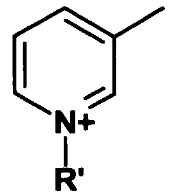 et 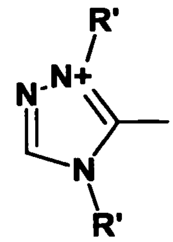

E6 E7 E8

dans lesquelles R' représente un radical alkyle en $C_1$-$C_4$ ;

lorsque m représente 0 et que $D_1$ représente un atome d'azote, alors E peut également désigner un groupement de structure E9 suivante :

E9 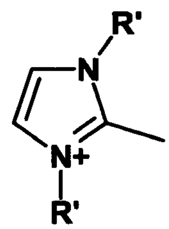

dans laquelle R' représente un radical alkyle en $C_1$-$C_4$.

- les composés de formule (VI) suivante :

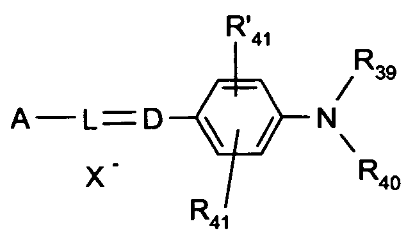

(VI)

dans laquelle :

L et D représentent, identiques ou différents, un atome d'azote ou le groupement - CH,
$R_{39}$ et $R_{40}$, identiques ou différents, représentent un atome d'hydrogène ; un radical alkyle en $C_1$-$C_4$ pouvant être substitué par un radical -CN, -OH ou -$NH_2$ ou forment avec un atome de carbone du cycle benzénique un hétérocycle éventuellement oxygéné ou azoté, pouvant être substitué par un ou plusieurs radicaux alkyle en $C_1$-$C_4$ ; un radical 4'-aminophényle,
$R_{41}$ et $R'_{41}$, identiques ou différents, représentent un atome d'hydrogène ou d'halogène choisi parmi le chlore, le brome, l'iode et le fluor, un radical cyano, alkyl en $C_1$-$C_4$, alcoxy en $C_1$-$C_4$ ou acétyloxy,
$X^-$ représente un anion de préférence choisi parmi le chlorure, le méthyl sulfate et l'acétate,
A représente un groupement choisi par les structures $A_1$ à $A_{19}$ suivantes :

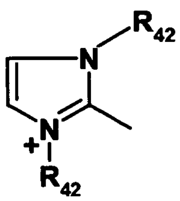 ; 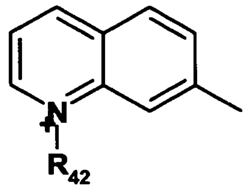 ; 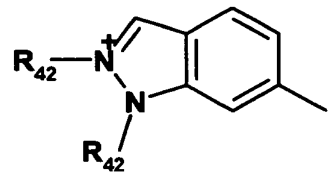 ;

A1 A2 A3

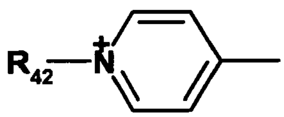 ; 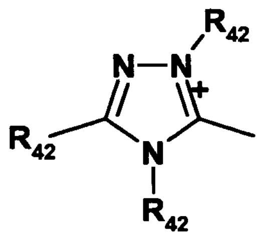 ; 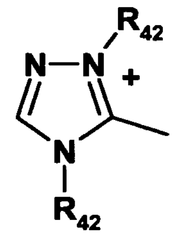 ;

A4 A5 A6

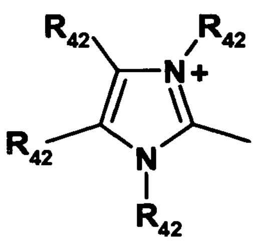 ; 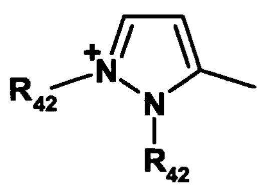 ; 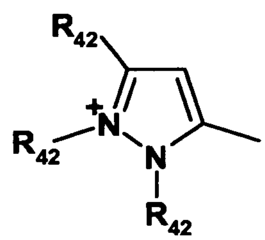 ;

A7 A8 A9

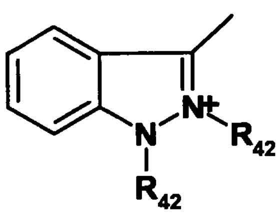 ; 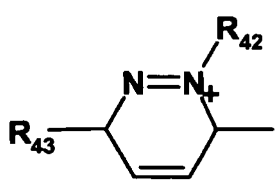 ; 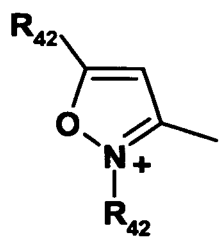 ;

A10 A11 A12

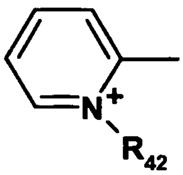 ; 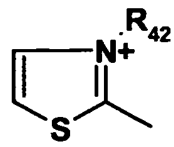 ; 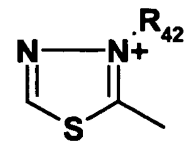 ;

$A_{13}$ $A_{14}$ $A_{15}$

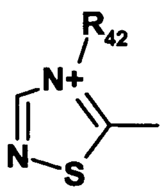 ; 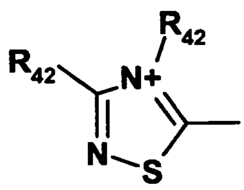 ; 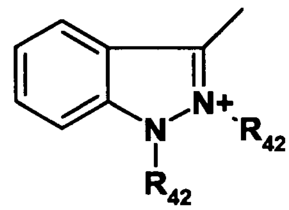

$A_{16}$ $A_{17}$ $A_{18}$

et

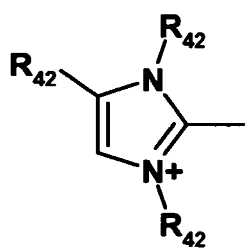 $A_{19}$

dans lesquelles $R_{42}$ représente un radical alkyle en $C_1$-$C_4$ pouvant être substitué par un radical hydroxyle et $R_{43}$ représente un radical alcoxy en $C_1$-$C_4$,

- les colorants décrits dans la demande de brevet EP 714954 de formules :

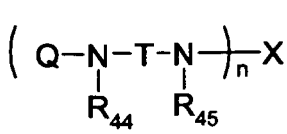

(VII)

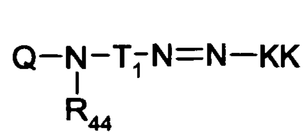

(VIII)

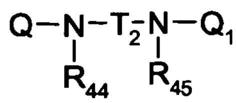

(IX)

dans lesquelles :

Q et $Q_1$, indépendamment l'un de l'autre, sont les restes de formule :

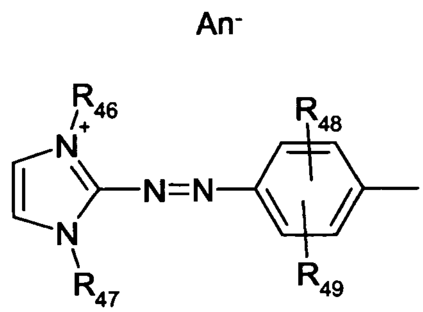

T représente un reste de diamine aliphatique ou aromatique,
$R_{44}$ et $R_{45}$, indépendamment l'un de l'autre, représentent un atome d'hydrogène, un groupe alkyle en $C_1$-$C_4$ ou peuvent former ensemble avec 2 atomes d'azote auxquels ils sont rattachés ou avec T et $T_2$ un cycle à 5, 6 ou 7 chaînons,
X représente le reste d'un chaînon formant un pont,
n représente un nombre entier 2, 3 ou 4,
$T_1$ représente un reste de diamines aromatique,
$T_2$ représente un reste de diamines aliphatique,
KK représente un reste de composé coupleur,
$R_{46}$ et $R_{47}$, indépendamment l'un de l'autre, représentent un atome d'hydrogène ou un groupe alkyle en $C_1$-$C_4$,
$R_{48}$ et $R_{49}$, indépendamment l'un de l'autre, représentent un atome d'hydrogène, un groupe alkyle en $C_1$-$C_4$ ou un groupe alkoxy en $C_1$-$C_4$,
$An^-$ représente un anion incolore,

ainsi que les colorants cationiques décrits dans les demandes de brevet WO 95/01 772, WO 95/15 144, EP 714 954, EP 1 170 000 EP 1 166 753, EP 1 166 754 et EP 1 170 001, différents des colorants ci-dessus. Le passage de ces demandes consacré aux colorants cationiques est incorporé dans la présente demande.

De manière préférée, on utilisera les colorants Basic Red 51 de formule (X) :

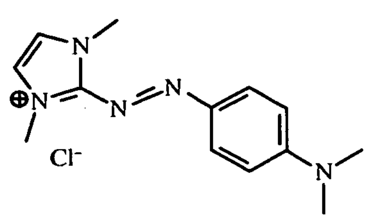

(X)

le Basic Yellow 87 de formule (XI) :

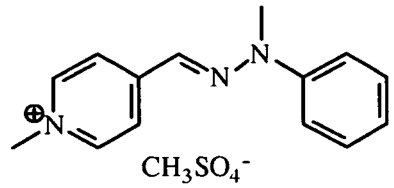

**(XI)**

ainsi que le Basic Orange 31 de formule (XII) :

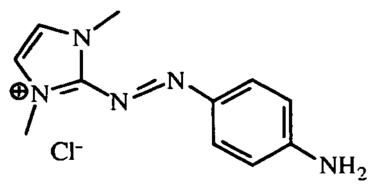

**(XII)**

De préférence, le ou les colorants directs hétérocycliques utiles dans le cadre de l'invention sont cationiques.

Il convient de noter que dans le cadre de l'invention on peut utiliser les colorants tels que décrits ci-dessus et leurs formes mésomères ainsi que dans le cadre de colorants chargés les colorants ne différant de ceux cités que par la nature du ou des contre ions.

Le ou les colorants directs hétérocycliques sont chacun généralement présents en quantité comprise entre 0,0001 et 30 % en poids par rapport au poids total de la composition, de préférence entre 0,001 et 20 % en poids par rapport au poids total de la composition, et encore plus préférentiellement entre 0,01 et 15 % en poids par rapport au poids total de la composition.

La composition tinctoriale de l'invention peut contenir d'autres bases d'oxydation différentes de celles utiles dans la présente invention et conventionnellement utilisées pour la teinture de fibres kératiniques.

La composition de la présente invention peut par exemple comprendre des bases d'oxydation additionnelles choisies parmi les para-phénylènediamines, les bis-phénylalkylènediamines, les para-aminophénols, les bis-para-aminophénols, les ortho-aminophénols, les ortho-phénylènediamines, les bases hétérocycliques différentes des dérivés de formule (I) tels que définis précédemment et leurs sels d'addition.

Parmi les para-phénylènediamines, on peut citer à titre d'exemple, la para-phénylènediamine, la para-toluylènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyi para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluylènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2 801 308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a] pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; le 3-amino-pyrazolo[1,5-a]pyridine-5-ol ; le 3-amino-pyrazolo[1,5-a]pyridine-4-ol ; le 3-amino-pyrazolo[1,5-a]pyridine-6-ol ; le 3-amino-pyrazolo[1,5-a] pyridine-7-ol ; ainsi que leurs d'addition avec un acide ou avec une base.

Parmi les dérivés pyrimidiniques, on peut citer les composés décrits par exemple dans les brevets DE 23 59 399 ; JP 88-169571 ; JP 05-63124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 38 43 892, DE 41 33 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyi)amino 1-méthyl pyrazole, et leurs sels d'addition avec un acide.

La ou les bases d'oxydation additionnelles présentes dans la composition de l'invention sont en général présentes chacune en quantité comprise entre 0,001 à 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

Le milieu approprié pour la teinture appelé aussi support de teinture est un milieu cosmétique généralement constitué par de l'eau ou par un mélange d'eau et d'au moins un solvant organique pour solubiliser les composés qui

ne seraient pas suffisamment solubles dans l'eau. A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition tinctoriale.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (XIII) suivante :

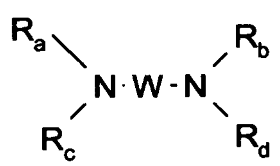
(XIII)

dans laquelle W est un reste propylène éventuellement substitué par un groupement hydroxyle ou un radical alkyle en $C_1$-$C_4$; $R_a$, $R_b$, $R_c$ et $R_d$, identiques ou différents, représentent un atome d'hydrogène, un radical alkyle en $C_1$-$C_4$ ou hydroxyalkyle en $C_1$-$C_4$.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, et on révèle la couleur à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention. De préférence, cette coloration est révélée à pH neutre.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases

à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus à l'exception de l'agent oxydant et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

La présente invention a également pour objet l'utilisation pour la coloration d'oxydation des fibres kératiniques, et en particulier des fibres kératiniques humaines telles que les cheveux, d'une composition telle que définie précédemment.

Les dérivés de diamino-N,N-dihydropyrazolone de formule (I) peuvent être obtenus à partir d'intermédiaires et de voies de synthèse décrites dans la littérature et notamment dans les références suivantes : J. Het. Chem., 2001, 38(3), 613-616 , Helvetica Chimica Acta, 1950, 33, 1183-1194, J.Org.Chem., 23, 2029 (1958), J.Am.Chem.Soc., 73, 3240 (1951), J.Am.Chem.Soc., 84, 590 (1962), Justus Liebig Ann.Chem., 686, 134 (1965), Tetrahedron. Lett., 31, 2859-2862 (1973), les brevets US4128425 et US 2841584 et les références citées.

Suivant ces références, les composés de formule (I) ayant les radicaux $R_3$ et $R_4$ égaux à des atomes d'hydrogène peuvent être obtenus à partir de la voie de synthèse représentée sur le schéma A ci-dessous :

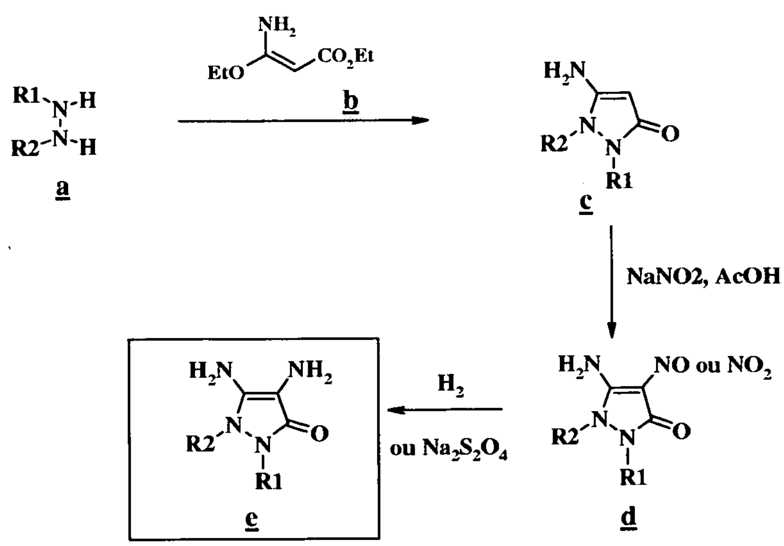


**schéma A**

Les composés dont les radicaux $R_1$ et $R_2$ représentent simultanément un groupe méthyle et les radicaux $R_3$ et $R_4$ des atomes d'hydrogène peuvent être obtenus en s'inspirant de la méthode décrite dans Justus Lieb.Ann.Chem., 686, 134 (1965) (schéma B) :

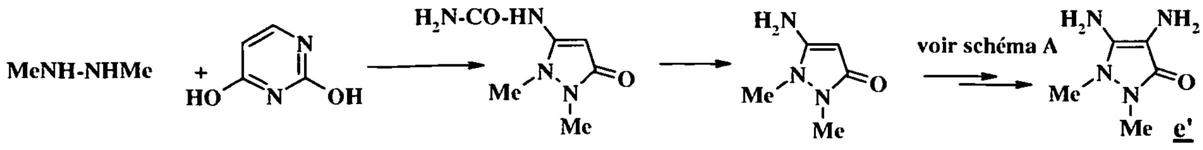


schéma B

Les composés dont le radical $R_1$ représente un groupe méthyle, $R_2$ un radical phényle, et les radicaux $R_3$ et $R_4$ des atomes d'hydrogène peuvent être obtenus en s'inspirant de la méthode décrite dans, J.Org.Chem., 23, 2029 (1958), J.Am.Chem.Soc., 73, 3240 (1951) (schéma C) :

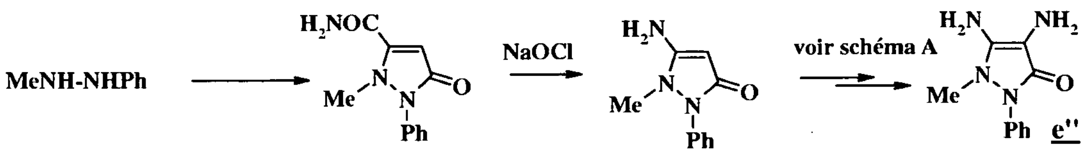


schéma C

Les composés dont les radicaux $R_1$ et $R_2$ forment ensemble un cycle à 5 chaînons et dont les radicaux $R_3$ et $R_4$ représentent des atomes d'hydrogène peuvent être obtenus en s'inspirant de la méthode décrite dans J. Het. Chem., 2001, 38(3), 613-616 (schéma D) :

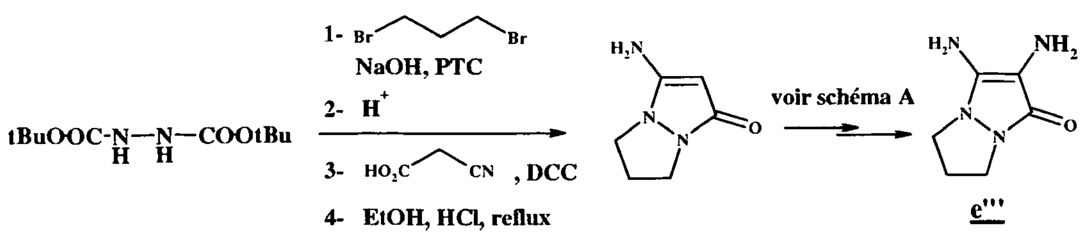


schéma D

Selon un procédé différent, les composés de formule (I) peuvent être obtenus selon la synthèse illustrée dans le schéma E :

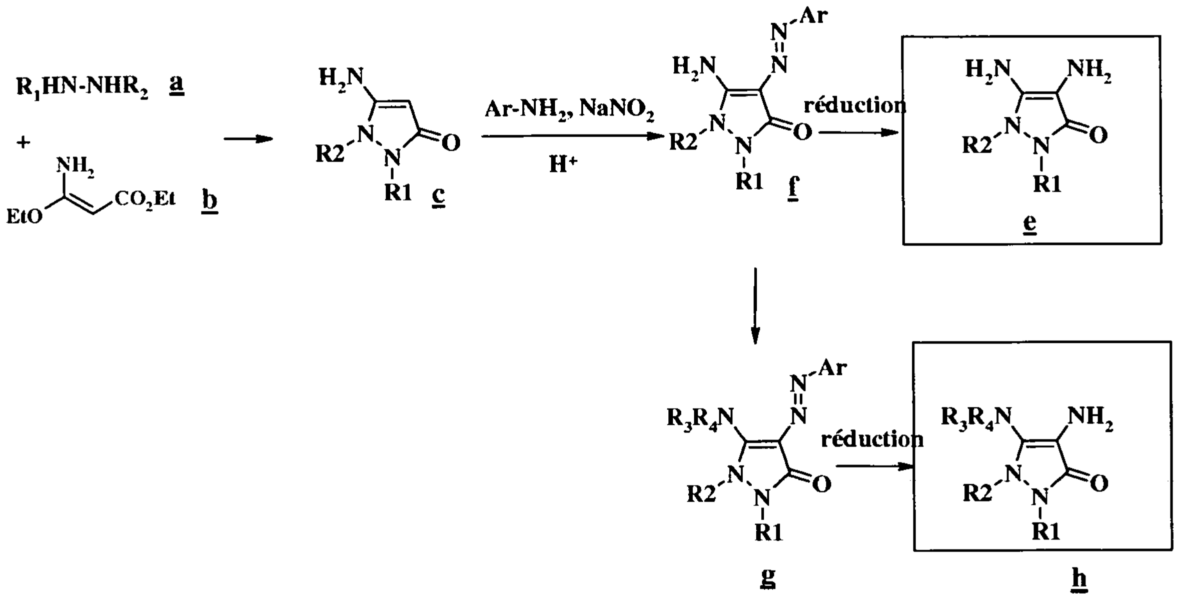


schéma E

Selon ce procédé, on met en oeuvre les étapes suivantes :

a) étape 1 :on fait réagir un composé **a**

**$R_1HN\text{-}NHR_2$** a

avec un composé **b** :

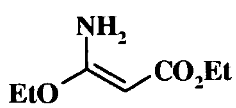

**b**

pour obtenir un composé **5-amino-1,2-dihydro-pyrazol-3-one c :**

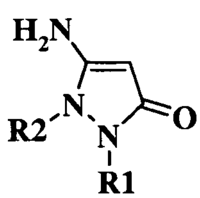

**c**

b) étape 2 : on fait réagir le dérivé **c** ainsi obtenu un sel d'aryldiazonium (**Ar-**$NH_2$, $NaNO_2$, $H^+$) pour obtenir un composé azoïque **f** :

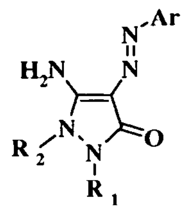

f

c) étape 3 : on effectue éventuellement une étape de fonctionalisation du groupement amine primaire du composé azoïque résultant **f** pour obtenir un composé **g** suivant :

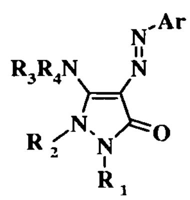

g

d) étape 4 : on effectue une réaction de réduction du composé azoïque **f** ou **g** pour obtenir, respectivement, un composé **e** ou **h** aminé :

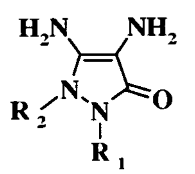

e

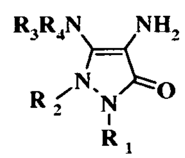

h

L'étape éventuelle de fonctionalisation du groupement amine primaire en position 5 en amine secondaire et tertiaire $NR_3R_4$, pour obtenir les composés **g**, est réalisée selon les méthodes classiques de synthèse organique (halogénure d'alkyle, O-sulfonate d'alkyle, trialkylammonium d'alkyle, amination réductrice, etc... voir par exemple Advanced Organic Chemistry, 3ème édition, 1985, J. March, Willey Interscience).
La réduction du groupe azoïque conduit aux composés **e** et **h** conformes à l'invention.
L'étape de réduction est réalisée de manière classique, par exemple en effectuant une réaction d'hydrogénation par catalyse hétérogène en présence de Pd /C, Pd(II) / C, Ni / Ra, etc... ou encore en effectuant une réaction de réduction par un métal, par exemple par du zinc, fer, étain, etc. (voir Advanced Organic Chemistry, 3ème édition, J. March, 1985, Willey Interscience et Reduction in organic Chemistry, M . Hudlicky, 1983 , Ellis Horwood Series Chemical Science).
Selon un autre procédé, les dérivés 2,3-diamino-6,7-dihydro-1H,5H-pyrazolo[1,2-a]-pyrazol-1-one et 2,3-diamino-5,6,7,8-tétrahydro-1H,6H-pyridazino[1,2-a]-pyrazol-1-one sont obtenus selon la synthèse illustrée par le schéma F :

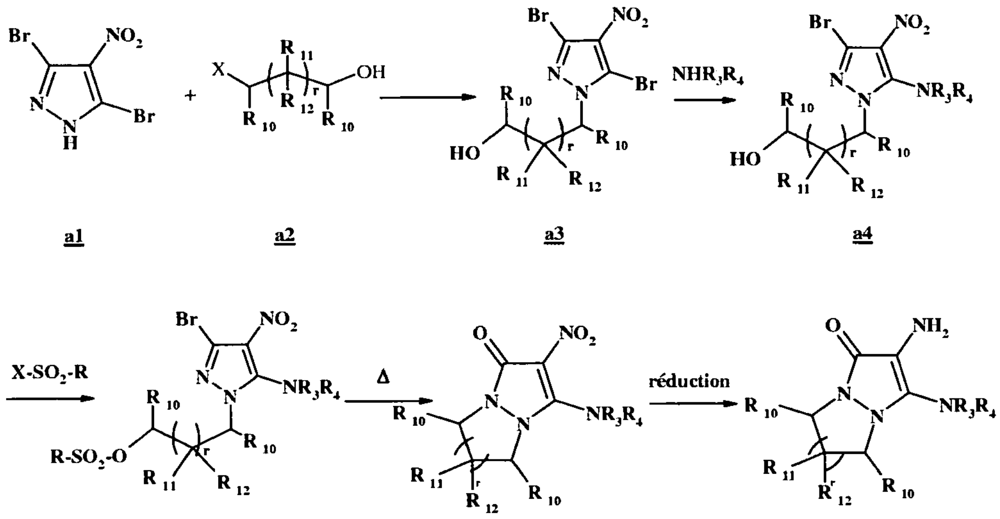


a1 a2 a3 a4

a5 a6 a7

schéma F

Selon ce procédé, on met en oeuvre les étapes suivantes :

a) étape 1 : on fait réagir un composé **a1** suivant :

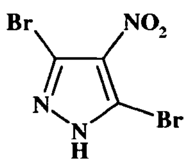

a1

avec un composé **a2** :

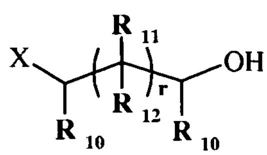

a2

pour obtenir un composé a3 :

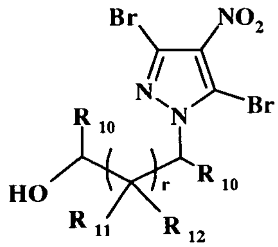

**a3**

dans lesquelles :

le radical $R_{10}$ représente un atome d'hydrogène, un carboxy ; un carboxamido ; un radical alkyle en $C_1$-$C_4$ éventuellement substitués par un ou plusieurs radicaux hydroxy, amino, (di)-alkylamino, alcoxy, carboxy, sulfonyle ;
les radicaux $R_{11}$ et $R_{12}$ représentent indépendamment les uns des autres des atomes d'hydrogène, d'halogène ; des radicaux amino ; (di)alkyl($C_1$-$C_4$)amino ; hydroxy ; carboxy ; carboxamido ; ($C_1$-$C_2$)alcoxy ; radical alkyle en $C_1$-$C_4$ éventuellement substitués par un ou plusieurs radicaux hydroxy, amino, (di)-alkylamino, alcoxy, carboxy, sulfonyle ;
X représente un atome d'halogène ou un alkyle sulfonate ;
r est un entier compris entre 1 et 3.

b) étape 2 : on fait réagir le composé **a3** avec une amine de formule $NHR_3R_4$ pour obtenir un composé **a4** :

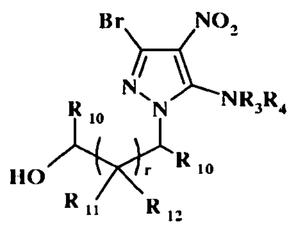

**a4**

c) étape 3 : on fait réagir le composé **a4** avec au moins un halogénure d'alkylsulfonyle, d'arylsulfonyle ou de perfluoroalkylsulfonyle $R-O_2S-X_1$ (R représente un alkyle, un aryle ou un perfluoroalkyle, $X_1$ représente un halogène), dans un solvant de point d'ébullition compris entre 60 °C et 190 °C pour obtenir un composé **a5** :

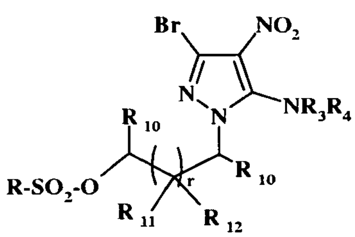

**a5**

d) étape 4 : le composé **a5** résultant est ensuite chauffé dans un solvant de point d'ébullition compris entre 60 °C et 190 °C pour obtenir un composé **a6** :

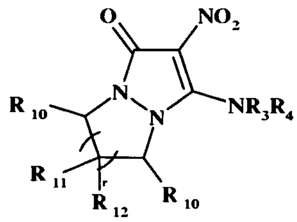

**a6**

e) étape 5 : Le composé **a6** obtenu est réduit pour obtenir le composé **a7** de formule ci-dessous (XIV) :

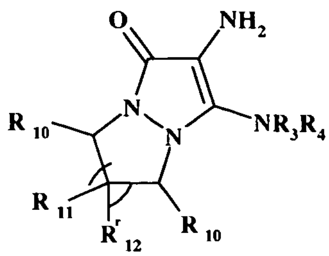

**a7**

**formule (XIV)**

Plus particulièrement, suivant ce procédé, le 3,5-dibromo-4-nitropyrazole **a1,** obtenu par exemple selon la méthode décrite dans le DE 4234885, réagit avec le réactif **a2**, de préférence dans un solvant de point d'ébullition compris entre 60 °C et 190 °C. A titre d'exemple, on peut citer le pentanol, le diméthylformamide, la N-méthylpyrrolidine. Plus particulièrement, la réaction est effectuée en présence d'une base organique ou minérale, telle que par exemple le carbonate de sodium, l'hydroxyde de sodium, l'acétate de sodium, ou la triéthylamine. La température du milieu réactionnel est avantageusement maintenue entre 60 °C et 160 °C, de préférence entre 80 °C et 120 °C.

Le 1-hydroxyalkyl-3,5-dibromo-4-nitropyrazole **a3** est de préférence isolé par précipitation ou cristallisation après ajout de glace au milieu réactionnel.

Dans l'étape 2, le dérivé **a3** est mis en réaction avec une amine $NHR_3R_4$, de préférence dans un solvant de point d'ébullition compris entre 60 °C et 190 °C, tel que par exemple le butanol, le pentanol, le diméthylformamide. La température est plus particulièrement comprise entre 60 °C et 160 °C, de préférence entre 80 °C et 120 °C. Après consommation des réactifs, le composé 5-amino-4-nitro-3-bromo-1-hydroxyalkylpyrazole **a4** est isolé par précipitation ou cristallisation à l'aide d'eau.

Conformément à l'étape 3, le dérivé **a5** est obtenu par réaction de l'alcool **a4** et d'un halogénure d'alkylsulfonyle, d'arylsulfonyle ou de perfluoroalkylsulfonyle. La réaction a lieu de préférence dans un solvant aprotique tel que par exemple le tétrahydrofurane, le dioxane. La réaction a lieu avantageusement à une température comprise entre -20 °C et 60 °C, de préférence entre 0 °C et 25 °C. De plus, cette étape a lieu en présence d'une base organique ou minérale telle que par exemple le carbonate de potassium, la triéthylamine, la N-méthylmorpholine. Après disparition des réactifs, le composé **a5** est isolé par précipitation ou cristallisation dans l'eau.

Le sulfonate **a5** obtenu à l'issue de l'étape 3 est mis, dans l'étape 4, en solution ou en dispersion dans un solvant de point d'ébullition compris entre 60 °C et 190 °C, de préférence entre 90 °C et 140 °C. La température du milieu réactionnel est ensuite amenée entre 90 °C et 140 °C, de préférence entre 105 °C et 125 °C jusqu'à consommation totale du sulfonate **a5**. Après retour à la température ambiante, le composé perhydro-pyrazolo[1,2-a]pyrazol-1-one (r = 1), perhydro-pyridazino[1,2-a]pyrazol-1-one (r = 2) ou perhydro-diazépino[1,2-a]pyrazolone (r = 3) **a6** cristallise et est isolé par les méthodes classiques de synthèse organique.

Le composé final **a7** conforme à l'invention est obtenu, lors d'une étape 5 par réduction du dérivé nitré **a6**, les méthodes de réduction utilisées étant par exemple une hydrogénation par catalyse hétérogène en présence de Pd / C, Pd(II) / C, Ni / Ra, etc... ou encore telles une réaction de réduction par un métal, par exemple par du zinc, fer, étain, etc, (voir

Advanced Organic Chemistry, 3ème édition, J. March, 1985, Willey Interscience et Reduction in organic Chemistry, M. Hudlicky, 1983, Ellis Horwood Series Chemical Science).

Les exemples qui suivent servent à illustrer l'invention sans toutefois présenter un caractère limitatif.

## EXEMPLES

**Exemple 1 : synthèse du dichlorhydrate de 2,3-diamino-6,7-dihydro-1H,5H-pyrazolo[1,2-a]pyrazol-1-one 5**

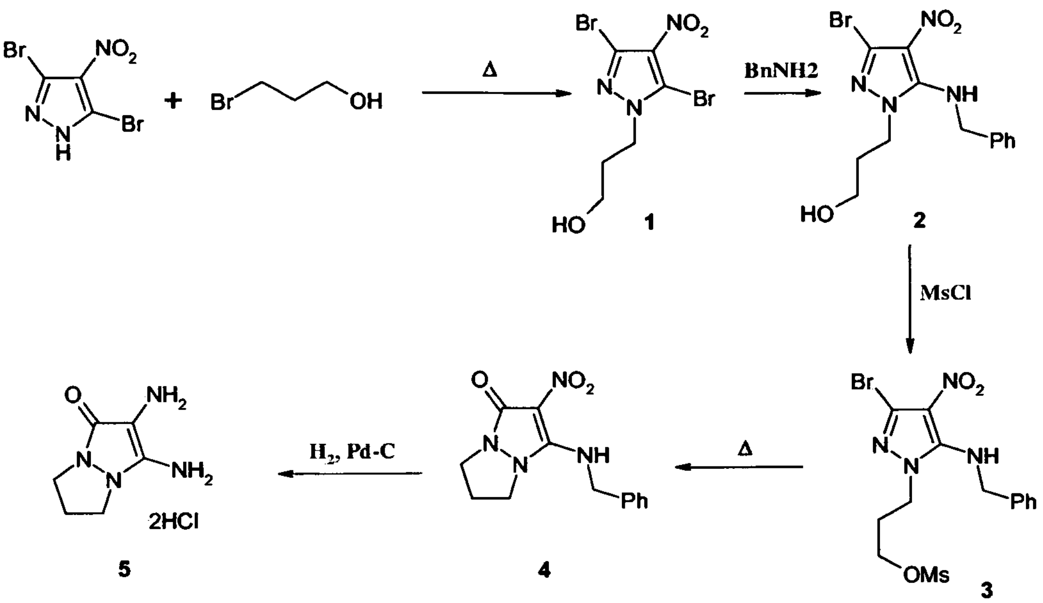


-Etape 1 : synthèse du 3-(3,5-dibromo-4-nitro-1H-pyrazol-1-yl)propan-1-ol **1**

Dans un tricol de 500 ml, on introduit 0,369 mole d'acétate de sodium à une solution de 0,184 mole de dibromonitropyrazole dans 250 ml de N-méthyl pyrrolidone et le milieu réactionnel est porté à 80 °C.

A cette température, on ajoute au goutte à goutte 0,369 mole de 3-bromo propanol. Cette température est maintenue pendant 5 heures.

Après refroidissement à température ambiante, le milieu est versé sur de la glace sous agitation.

Le 3-(3,5-dibromo-4-nitro-1H-pyrazol-1-yl)propan-1-ol **1** précipite. Il est essoré, séché et obtenu avec un rendement de 75 %.

La masse du composé attendu $C_6H_7Br_2N_3O_3$ est détectée en spectrométrie de masse.

Les analyses RMN ($^1H$ 400 MHz et $^{13}C$ 100,61 MHz DMSO $d_6$) sont conformes à la structure attendue.

-Etape 2 : synthèse du 3-[5-(benzylamino)-3-bromo-4-nitro-1H-pyrazol-1-yl]propan-1-ol **2**

Dans un tricol de 500 ml contenant 150 ml d'éthanol, on disperse 0,135 mole de 3-(3,5-dibromo-4-nitro-1H-pyrazol-1-yl)propan-1-ol **1**, chauffe à 60 °C, puis additionne 0,825 mole de benzylamine en 30 minutes.

Après 6 heures à 60 °C, le milieu réactionnel est refroidi à température ambiante.

Le 3-[5-(benzylamino)-3-bromo-4-nitro-1H-pyrazol-1-yl]propan-1-ol 2 est précipité en versant le milieu réactionnel sur 1 litre de glace sous agitation. Après essorage et séchage sous vide en présence de $P_2O_5$, le composé 2 est isolé avec un rendement de 90 %.

Les analyses RMN ($^1H$ 400 MHz et $^{13}C$ 100,61 MHz DMSO $d_6$) sont conformes à la structure attendue.

Analyse élémentaire :

| | | | | | |
|---|---|---|---|---|---|
| théorique : | C43,96 | H4,26 | N15,77 | O13,51 | Br22,50 |
| mesuré : | C44,09 | H4,22 | N15,44 | O14,37 | Br21,50 |

-Etape 3 : synthèse du 3-[5-(benzylamino)-3-bromo-4-nitro-1H-pyrazol-1-yl]propyl méthanesulfonate **3**

Dans un tricol de 500 ml contenant 200 ml de THF, on introduit, sous agitation, 0,126 mole de 3-[5-(benzylamino)-3-bromo-4-nitro-1H-pyrazol-1-yl]propan-1-ol **2** et 15,82 ml de triéthylamine. Le mélange obtenu est ensuite refroidi à 5 ˚C et 0,126 mole de chlorure de mésyle sont coulés en 45 minutes.
Le milieu réactionnel est maintenu à cette température pendant 2 heures, puis le 3-[5-(benzylamino)3-bromo-4-nitro-1H-pyrazol-1-yl]propylmethanesulfonate **3** est précipité en versant le milieu réactionnel sur 800 ml de la glace.
Après filtration, le solide est lavé abondamment à l'eau et à l'éther diisopropylique. Le séchage est réalisé sous vide en présence de $P_2O_5$. Le rendement de cette étape est de 94 %.
La masse du composé attendu $C_{14}H_{17}BrN_4O_5S$ est détectée en spectrométrie de masse.
Les analyses RMN ($^1H$ 400 MHz et $^{13}C$ 100,61 MHz DMSO $d_6$) sont conformes à la structure attendue.

Analyse élémentaire :

| | | | | | | |
|---|---|---|---|---|---|---|
| Théorie : | C38,81 | H3,96 | N12,93 | O18,46 | S7,40 | Br18,44 |
| Mesuré : | C39,03 | H3,91 | N12,83 | O18,52 | S7,29 | Br18,26 |

-Etape 4: synthèse de la 3-(benzylamino)-2-nitro-6,7-dihydro-1H,5H-pyrazolo[1,2-a]pyrazol-1-one **4**

Dans un tricol de 500 ml contenant 300 ml de pentanol, on disperse sous agitation 0,1 mole de 3-[5-(benzylamino)-3-bromo-4-nitro-1H-pyrazol-1-yl]propyl méthanesulfonate 3 et porte le milieu réactionnel à 130 ˚C pendant 2 heures.
Après refroidissement à température ambiante, le solide formé est essoré sur fritté, lavé à l'éther diisopropylique et séché sous vide en présence de $P_2O_5$. Le 3-(benzylamino)-2-nitro-6,7-dihydro-1H,5H-pyrazolo[1,2-a]pyrazol-1- one **4** est obtenu avec un rendement de 86 %.
Les analyses RMN ($^1H$ 400 MHz et $^{13}C$ 100,61 MHz DMSO $d_6$) sont conformes à la structure attendue.
La masse du composé attendu $C_6H_{11}N_4O$ est détectée en spectrométrie de masse.

Analyse élémentaire :

| | | | | |
|---|---|---|---|---|
| Théorie : | C56,72 | H5,49 | N20,36 | O17,44 |
| Mesuré : | C56,68 | H5,13 | N20,38 | O17,69 |

-Etape 5 : synthèse du dichlorhydrate de 2,3-diamino-6,7-dihydro-1H,5H-pyrazolo[1,2-a]pyrazol-1-one **5**

Dans un autoclave de 1 litre contenant 800 ml d 'éthanol, on introduit 20 g de 3-(benzylamino)-2-nitro-6,7-dihydro-1H,5H-pyrazolo[1,2-a]pyrazol-1-one **4** et 4 g de palladium sur charbon à 5 %. La réduction est ensuite réalisée sous une pression d'hydrogène de 8 Bars et à une température comprise entre 50 ˚C et 100 ˚C (agitation comprise entre 1000 et 2500 tr / min).
Au bout de 4 heures de réaction, il n'a plus consommation d'hydrogène et le milieu est refroidi à 20 ˚C.
Le catalyseur est éliminé sous azote par filtration, puis de l'éthanol chlorhydrique est ajouté au filtrat. Le produit cristallisé est essoré, lavé à l'éther diisopropylique, puis séché sous vide en présence de $P_2O_5$. Le dichlorhydrate de 2,3-diamino-6,7-dihydro-1H,5H-pyrazolo[1,2-a]pyrazol-1-one **5** est obtenu avec un rendement de 89 %.
La masse du composé attendu est détectée en spectrométrie de masse.
Les analyses RMN ($^1H$ 400 MHz et $^{13}C$ 100,61 MHz DMSO $d_6$) sont conformes à la structure attendue.

Analyse élémentaire :

| | | | | | |
|---|---|---|---|---|---|
| Théorique: | C31,73 | H5,33 | N24,67 | O7,07 | Cl31,22 |
| Mesuré : | C31,45 | H5,20 | N24,62 | O7,24 | Cl30,86 |

**Exemple 2 : synthèse du dichlorhydrate de 2-amino-3-(éthylamino)-6,7-dihydro-1H,5H-pyrazolo[1,2-a]pyrazol-1-one 9**

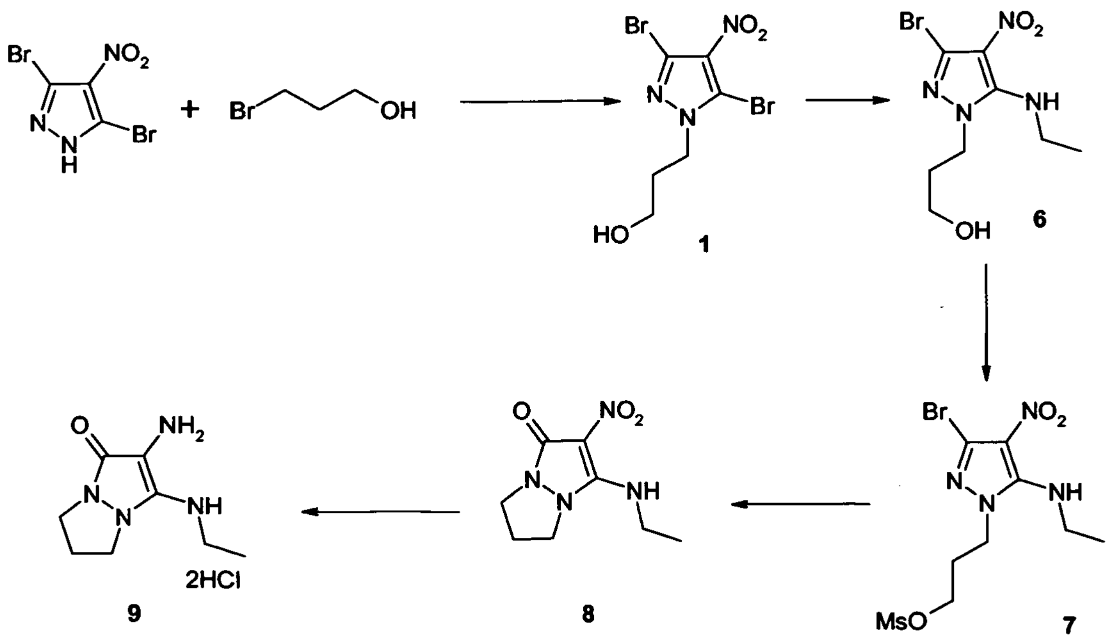

1 6 7 8 9

-Etape 2 :synthèse du 3-[3-bromo-5-(éthylamino)-4-nitro-1H-pyrazol-1-yl]propan-1-ol **6**

Dans un tricol, sous agitation, on introduit 15 mmoles de 3-(3,5-dibromo-4-nitro-1H-pyrazol-1-yl)propan-1-ol dans 30 ml d'éthanol. Le milieu homogène est chauffé à 75 ˚C puis 93 mmoles d'éthylamine sont coulées au goutte à goutte et l'agitation est maintenue quatre heures.

Après refroidissement à température ambiante, le milieu est versé sur de la glace et le 3-[3-bromo-5-(éthylamino)-4-nitro-1H-pyrazol-1-yl]propan-1-ol **6** précipite.

Le solide jaune est essoré, puis lavé abondamment à l'eau et à l'éther diisopropylique. Le séchage est réalisé sous vide en présence de $P_2O_5$. La masse récupérée est de 3,6 g.

Les analyses RMN ($^1H$ 400 MHz et $^{13}C$ 100,61 MHz DMSO $d_6$) sont conformes à la structure attendue. La masse du composé attendu $C_8H_{13}BrN_4O_3$ est détectée en spectrométrie de masse.

-Etape 3: synthèse du 3-[5-(éthylamino)-3-bromo-4-nitro-1H-pyrazol-1-yl]propyl methanesulfonate **7**

Dans un tricol de 100 ml contenant 30 ml de THF, sous agitation, on introduit 11,2 mmoles de 3-[3-bromo-5-(éthylamino)-4-nitro-1H-pyrazol-1-yl]propan-1-ol 6 et 1,6 ml de triéthylamine. Le mélange homogène orange obtenu est refroidi à 0 ˚C et 1,44 ml de chlorure de mésyle sont coulés en 20 minutes.

Le milieu réactionnel est maintenu à cette température pendant 2 heures puis le 3-[5-(éthylamino)-3-bromo-4-nitro-1H-pyrazol-1-yl]propylméthanesulfonate 7 est précipité en versant le milieu réactionnel sur 500 ml de glace.

Le solide jaune est essoré, puis lavé abondamment à l'eau et à l'éther diisopropylique ; le séchage est réalisé sous vide en présence de $P_2O_5$. La masse récupérée est de 3,1 g.

Les analyses RMN ($^1H$ 400 MHz et $^{13}C$ 100,61 MHz DMSO $d_6$) sont conformes à la structure attendue.
La masse du composé attendu $C_9H_{15}BrN_4O_5S$ est détectée en spectrométrie de masse.

-Etape 4 : synthèse de la 3-(éthylamino) 2-nitro-6,7-dihydro-1H,5H-pyrazolo[1,2-alpyrazol-1-one **8**

Dans un tricol de 50 ml contenant 20 ml de pentanol, on disperse sous agitation 8 mmoles de 3-[5-(éthylamino)-3-bromo-4-nitro-1H-pyrazol-1-yl]propyl méthanesulfonate **7,** et porte le milieu réactionnel à 130 ˚C pendant 2 heures. Après refroidissement à température ambiante, le solide formé est essoré, puis lavé à l'éther diisopropylique.
Après séchage sous vide en présence de $P_2O_5$, on obtient 1,46 g de 3-(éthylamino)-2-nitro-6,7-dihydro-1H, 5H-pyrazolo[1,2-a]pyrazol-1one **8.**
Les analyses RMN ($^1H$ 400 MHz et $^{13}C$ 100,61 MHz DMSO $d_6$) sont conformes à la structure attendue.
La masse du composé attendu est détectée en spectrométrie de masse.

-Etape 5 : synthèse du dichlorhydrate de 2-amino-3-(éthylamino 6,7-dihydro-1H,5H-pyrazolo[1,2-a]pyrazol-1-one **9**

Dans un autoclave de 300 ml contenant 200 ml d'éthanol, on introduit 1,45 g de 3-(éthylamino)-2-nitro-6,7-dihydro-1H,5H-pyrazolo[1,2-a]pyrazol-1-one **8** et 300 mg de palladium sur charbon à 5 %. La réduction est réalisée sous une pression d'hydrogène de 8 Bars à une température de 60 ˚C (agitation de 1700 tr / min).
Au bout de 2 heures de réaction, il n'y a plus consommation d'hydrogène et le milieu est refroidi à 20 ˚C.
Le catalyseur est éliminé par filtration sous azote et le filtrat est dilué avec 100 ml d'éther isopropylique chlorhydrique. La solution jaune pale est évaporée à sec puis le solide est repris avec un mélange éthanol / éther isopropylique. Le dichlorhydrate de 2-amino-3-(éthylamino)-6,7-dihydro-1H,5H-pyrazolo[1,2-a]pyrazol-1-one **9** précipite ; il est essoré et après séchage sous vide en présence de $P_2O_5$, on récupère 1,18 g de dichlorhydrate de 2-amino-3-(éthylamino)-6,7-dihydro-1H,5H-pyrazolo[1,2-a]pyrazol-1-one **9**.
Les analyses RMN ($^1H$ 400 MHz et $^{13}C$ 100,61 MHz DMSO $d_6$) sont conformes à la structure attendue.
La masse du composé attendu $C_8H_{14}N_4O$ est détectée en spectrométrie de masse.

**Exemple 3 : dichlorhydrate de 2-amino-3-(isopropylamino)-6,7-dihydro-1H,5H-pyrazolo[1,2-a]pyrazol-1-one 13**

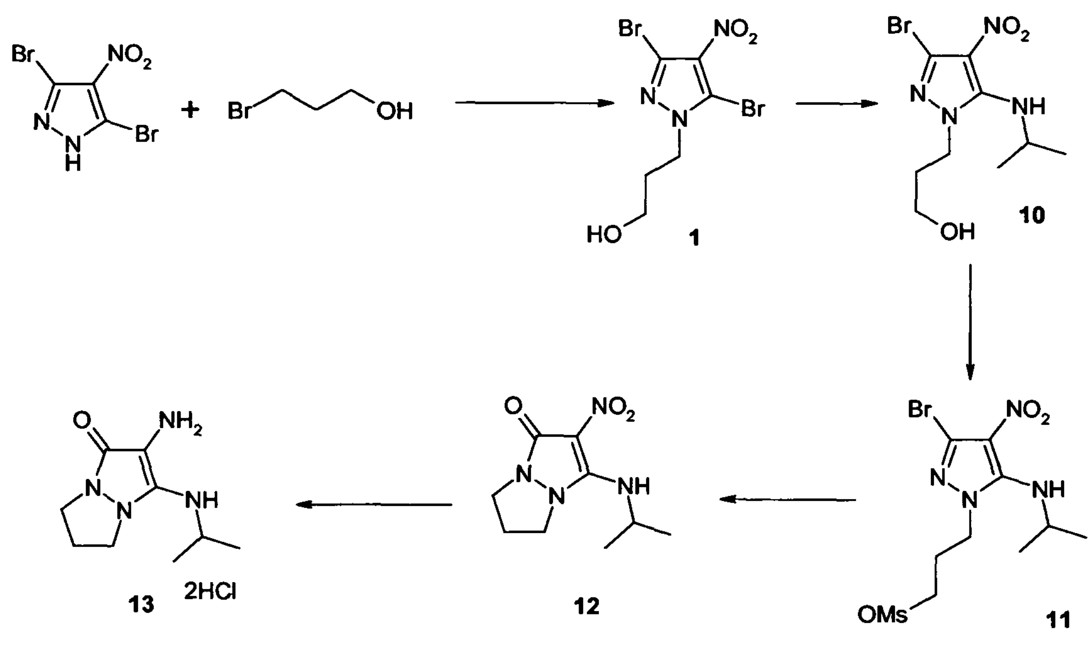

-Etape 2 : 3-[3-bromo-5-(isopropylamino)-4-nitro-1H-pyrazol-1-yl]propan-1-ol **10**

Dans un tricol, sous agitation, on introduit 15 mmoles de 3-(3,5-dibromo-4-nitro-1H-pyrazol-1-yl)propan-1-ol dans 30 ml d'éthanol. Le milieu homogène est chauffé à 75 ˚C, puis 93 mmoles d'isopropylamine sont coulées au goutte

à goutte tout en maintenant l'agitation quatre heures.

Après refroidissement à température ambiante, le milieu est versé sur de la glace, puis neutralisé à l'acide chlorhydrique. On extrait le 3-[3-bromo-5-(isopropylamino)-4-nitro-1H-pyrazol-1-yl]propan-1-ol **10** au dichlorométhane.

Après séchage de la phase organique sur sulfate de sodium et élimination du solvant par évaporation sous vide, on obtient 4,37 g de 3-[3-bromo-5-(isopropylamino)-4-nitro-1H-pyrazol-1-yl]propan-1-ol **10**.

Les analyses RMN ($^1H$ 400 MHz et $^{13}C$ 100,61 MHz DMSO $d_6$) sont conformes à la structure attendue. La masse du composé attendu $C_9H_{15}BrN_4O_3$ est détectée en spectrométrie de masse.

-Etape 3: synthèse du 3-15-(isopropylamino)-3-bromo-4-nitro-1H-pyrazol-1-yl]propyl méthanesulfonate **11**

Dans un tricol de 50 ml contenant 20 ml de THF, on introduit, sous agitation, 13,7 mmoles de 3-[3-bromo-5-(isopropylamino)-4-nitro-1H-pyrazol-1-yl]propan-1-ol **10** et 1,94 ml de triéthylamine. Le mélange homogène orange ainsi obtenu est refroidi à 0 °C et 1,76 ml de chlorure de mésyle sont coulés en 20 minutes.
Le milieu réactionnel est maintenu à cette température pendant 2 heures, puis le 3-[5-(éthylamino)-3-bromo-4-nitro-1H-pyrazol-1-yl]propylméthanesulfonate **11** est précipité en versant le milieu réactionnel sur 500 ml de glace.
Le solide jaune est essoré, puis lavé abondamment à l'eau et à l'éther de pétrole, le séchage est réalisé sous vide en présence de $P_2O_5$. La masse récupérée est de 4,2 g.

Les analyses RMN ($^1H$ 400 MHz et $^{13}C$ 100,61 MHz DMSO $d_6$) sont conformes à la structure attendue. La masse du composé attendu est détectée en spectrométrie de masse.

-Etape 4 : synthèse de la 3-(isopropylamino)-2-nitro-6,7-dihydro-1H,5H-pyrazolo[1,2-a]pyrazol-1-one **12**

Dans un tricol de 50 ml, on disperse, sous agitation, 10 mmoles de 3-[5-(isopropylamino)-3-bromo-4-nitro-1H-pyrazol-1-yl]propylméthanesulfonate **11** dans 20 ml de pentanol et chauffe à 130 °C pendant 2 heures.
Après refroidissement à température ambiante, le solide obtenu est essoré sur fritté, et lavé à l'éther diisopropylique.

Après séchage sous vide en présence de $P_2O_5$, 1,71 g de 3-(isopropylamino)-2-nitro-6,7-dihydro-1H,5H-pyrazolo[1,2-a]pyrazol-1one **12** sont obtenus.

Les analyses RMN ($^1H$ 400 MHz et $^{13}C$ 100,61 MHz DMSO $d_6$) sont conformes à la structure attendue. La masse du composé attendu $C_9H_{14}N_4O_3$ est détectée en spectrométrie de masse.

-Etape 5 : synthèse du dichlorhydrate de 2-amino-3-(isopropylamino)-6,7-dihydro-1H,5H-pyrazolo[1,2-a]pyrazol-1-one **13**

Dans un autoclave de 300 ml contenant 200 ml d 'éthanol, on introduit 1,70 g de 3-(isopropylaminoamino)-2-nitro-6,7-dihydro-1H,5H-pyrazolo[1,2-a]pyrazol-1-one **12** et 300 mg de palladium sur charbon à 5 %. La réduction est réalisée sous une température de 60 °C et sous une pression d'hydrogène de 6 Bars (agitation de 2000 tr min).
Au bout de 2 heures de réaction, il n'a plus consommation d'hydrogène et le milieu est refroidi à 20 °C.
Le catalyseur est éliminé par filtration sous azote après refroidissement à température ambiante et de l'éther isopropylique chlorhydrique est additionné.
La solution jaune pale est évaporée à sec, puis le solide est repris avec 50 ml d'éther diisopropylique saturé en acide chlorhydrique, le précipité est récupéré par essorage. Après séchage sous vide en présence de $P_2O_5$, 1,5 g de dichlorhydrate de 2-amino-3-(isopropylamino)-6,7-dihydro-1H,5H-pyrazolo[1,2-a]pyrazol-1-one **13** sont isolés.

Les analyses RMN ($^1H$ 400 MHz et $^{13}C$ 100,61 MHz DMSO $d_6$) sont conformes à la structure attendue. La masse du composé attendu $C_9H_{16}N_4O$ est détectée en spectrométrie de masse.

**Exemple 4 : dichlorhydrate de 2-amino-3-(pyrrolidin-1-yl)-6,7-dihydro-1H,5H-pyrazolo[1,2-a]pyrazol-1-one 17**

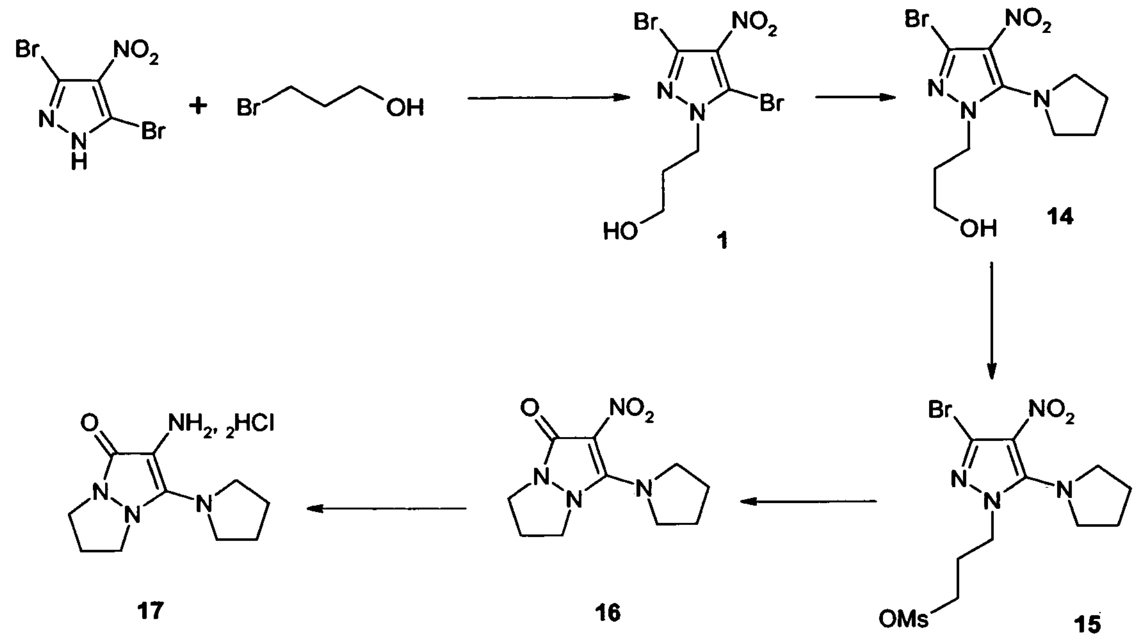

1 14 15 16 17

-Etape 2 : 3-(3-bromo-4-nitro-5-(pyrrolidin-1-yl)-1H-pyrazol-1-ylpropan-1-ol **14**

Dans un tricol, sous agitation, on introduit 15 mmoles de 3-(3,5-dibromo-4-nitro-1H-pyrazol-1-yl)propan-1-ol dans 20 ml d'isopropanol. Le milieu homogène est chauffé à 75 °C puis 90 mmoles de pyrrolidine sont coulées au goutte à goutte et l'agitation est maintenue deux heures.

Après refroidissement à température ambiante, le milieu est versé sur de la glace et neutralisé à l'acide chlorhydrique. On extrait le 3-(3-bromo-4-nitro-5-(pyrrolidin-1-yl)-1H-pyrazol-1-yl)propan-1-ol **14** par du dichlorométhane. Après séchage de la phase organique sur sulfate de sodium et distillation du solvant par évaporation sous vide, on obtient 4,8 g de 3-(3-bromo-4-nitro-5-(pyrrolidin-1-yl)-1H-pyrazol-1-yl)propan-1-ol **14**.

Les analyses RMN ($^1$H 400 MHz et $^{13}$C 100,61 MHz DMSO $d_6$) sont conformes à la structure attendue.

La masse du composé attendu $C_{10}H_{17}BrN_4O$ est détectée en spectrométrie de masse.

-Etape 3: synthèse du 3-(3-bromo-4-nitro-5-(pyrrolidin-1-yl)-1H-pyrazol-1 - yl)propylméthanesulfonate **15**

Dans un tricol de 100 ml contenant 50 ml de THF, on introduit, sous agitation, 30 mmoles 3-(3-bromo-4-nitro-5-(pyrrolidin-1-yl)-1H-pyrazol-1-yl)propan-1-ol **14** et 4,25 ml de triéthylamine. Le mélange homogène orange obtenu est refroidi à 0 °C et 2,32 ml de chlorure de mésyle sont coulés en 20 minutes.
Le milieu réactionnel est maintenu à cette température pendant 2 heures puis le 3-(3-bromo-4-nitro-5-(pyrrolidin-1-yl)-1H-pyrazol-1-yl)propylméthanesulfonate **15** est précipité en versant le milieu réactionnel sur de la glace.
Le solide est essoré, puis séché sous vide en présence de $P_2O_5$. La masse récupérée est de 9,3 g.

Les analyses RMN ($^1$H 400 MHz et $^{13}$C 100,61 MHz DMSO $d_6$) sont conformes à la structure attendue.
La masse du composé attendu $C_{11}H_{19}BrN_4O_3S$ est détectée en spectrométrie de masse.

-Etape 4 : synthèse 2-nitro-3-(pyrrolidin-1-yl)-6,7-dihydro-1H,5H-pyrazolo[1,2-a]pyrazol-1-one **16**

Dans un tricol de 250 ml, on introduit, sous agitation, 22,5 mmoles de 3-(3-bromo-4-nitro-5-(pyrrolidin-1-yl)-1H-pyrazol-1-yl)propylméthanesulfonate **15** dans 100 ml de pentanol. Le milieu ainsi obtenu est porté à 130 °C pendant 2 heures.
Après refroidissement à température ambiante, le 2-nitro-3-(pyrrolidin-1-yl)-6,7-dihydro-1H,5H-pyrazolo[1,2-a]pyrazol-1-one **16** est extrait au dichlorométhane.
Après séchage de la phase organique sur sulfate de sodium et distillation du solvant sous vide, on obtient 1,2 g de 2-nitro-3-pyrrolidin-1-yl-6,7-dihydro-1H,5H-pyrazolo[1,2-a]pyrazol-1-one **16.**

Les analyses RMN ($^1$H 400 MHz et $^{13}$C 100,61 MHz DMSO $d_6$) sont conformes à la structure attendue.
La masse du composé attendu $C_{10}H_{14}N_4O_3$ est détectée en spectrométrie de masse.

-Etape 5 : synthèse du dichlorhydrate de 2-amino-3-(pyrrolidin-1-yl)-6,7-dihydro-1H,5H-pyrazolo[1,2-a]pyrazol-1-one **17**

Dans un autoclave de 300 ml contenant 200 ml d'éthanol, on introduit 1,1 g de 2-nitro-3-(pyrrolidin-1-yl)-6,7-dihydro-1H,5H-pyrazolo[1,2-a]pyrazol-1-one **16** et 300 mg de palladium sur charbon à 5 %. La réduction est réalisée sous une agitation de 2000 tr / min, sous une température de 60 °C et sous une pression d'hydrogène de 6 Bars.
Au bout de 2 heures de réaction, il n'a plus consommation d'hydrogène et le milieu est refroidit à 20 °C.
Le catalyseur est éliminé par filtration sous azote après refroidissement à température ambiante et de l'éther isopropylique chlorhydrique est additionné.
La solution jaune pale est évaporée à sec, puis le solide est repris avec 50 ml d'éther diisopropylique saturé en acide chlorhydrique, le précipité est récupéré par essorage. Après séchage sous vide en présence de $P_2O_5$, on obtient 1,5 g de dichlorhydrate de 2-amino-3-(pyrrolidin-1-yl)-6,7-dihydro-1H,5H-pyrazolo[1,2-a]pyrazol-1-one 5 **17.**
Les analyses RMN ($^1$H 400 MHz et $^{13}$C 100,61 MHz DMSO $d_6$) sont conformes à la structure attendue.
La masse du composé attendu $C_{10}H_{16}N_4O$ est détectée en spectrométrie de masse.

**Exemple 5 : Synthèse du diméthanesulfonate de 2,3-diamino-6,7-dihydro-1H,5H-pyrazolo[1,2-a]pyrazol-1-one**

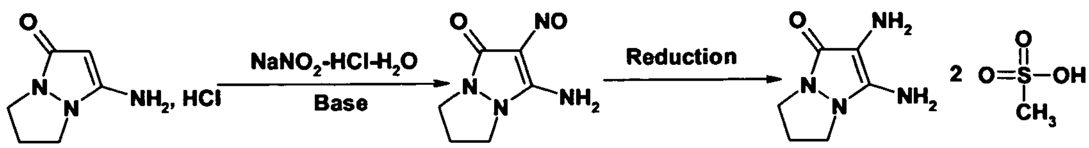


1 2 3

Synthèse du 3-amino-2-nitroso-6,7-dihydro-1H,5H-pyrazolo[1,2-a]pyrazol-1-one : 2

Dans un tricol de 500 ml, on dissout, sous agitation, à température ambiante, 43 g (0,245 mole) de chlorhydrate de 3-amino-6,7-dihydro-1H,5H-pyrazolo[1,2-a]pyrazol-1-one dans un mélange de 180 ml d'eau et 35 ml d'acide chlorhydrique à 35 %.
On refroidit à 0 °C et ajoute goutte à goutte, en 30 minutes, une solution de 17,3 g de nitrite de sodium (0,25 mole) dans 20 ml d'eau. La température du milieu réactionnel est maintenue entre 0 et +5 °C pendant toute la durée de l'addition et pendant une heure après la fin de l'addition.
Le milieu réactionnel est amené à pH 8 par addition de soude, sous agitation, tout en maintenant la température entre 0 et 5 °C. La 3-amino-2-nitroso-6,7-dihydro-1H,5H-pyrazolo[1,2-a]pyrazol-1-one 2 précipite sous la forme d'un solide orangé rouge qui est filtré sur verre fritté n° **4**, empâté dans le minimum de 2-propanol, lavé à l'éther diisopropylique et séché sous vide en présence de pentaoxyde de phosphore. On obtient ainsi 35 g de produit rouge orangé (rendement : 85 %).
Les spectres de RMN (1H 400 MHz et 13C 100,61 MHz DMSO $d_6$) et de masse sont conformes à la structure attendue 2.

Synthèse du diméthanesulfonate de 2,3-diamino-6,7-dihydro-1H,5H-pyrazolo[1,2-a]pyrazol-1-one : **3**

Dans un autoclave de 1 litre, on introduit 33,6 g (0,2 mole) de 3-amino-2-nitroso-6,7-dihydro-1H,5H-pyrazolo [1,2-a]pyrazol-1-one 2, 500 ml d'éthanol et 6 g de palladium sur charbon à 5 % contenant 50 % d'eau.
Le milieu est purgé 3 fois à l'azote puis 3 fois à l'hydrogène et la température du mélange est portée à 40 °C.
La réduction est réalisée en deux heures sous une pression de 8 bars. Cette réduction est exothermique et la température atteint d'elle-même 70 °C.
On laisse la température redescendre à 50 °C puis le catalyseur est filtré sur un filtre presse sous un courant d'azote.
Le filtrat est coulé dans un mélange de 50 ml d'éthanol et 40 ml d'acide méthane sulfonique, en refroidissant à 0 °C. Le diméthanesulfonate de 2,3-diamino-6,7-dihydro-1H,5H-pyrazolo[1,2-a]pyrazol-1-one 3 cristallise sous la forme d'un solide jaune pâle qui est essoré sur verre fritté n° 4, lavé à l'éther diisopropylique puis à l'éther de pétrole et enfin séché sous vide en présence de pentaoxyde de phosphore. On obtient ainsi 43 g de solide jaune pâle (rendement : 65 %).
Les spectres de RMN ($^1$H 400 MHz et $^{13}$C 100,61 MHz DMSO $d_6$) et de masse sont conformes à la structure attendue **3.**

Analyse élémentaire :

| | | | | | |
|---|---|---|---|---|---|
| Théorie : | C27,74 | H5,23 | N16,17 | O32,33 | S18,51 |
| Mesuré : | C27,16 | H5,22 | N15,63 | O32,81 | S18,64 |

**Exemple 6 : synthèse du chlorhydrate de 2,3-diamino-5,6,7,8-tetrahydro-1H-pyrazolo[1,2-a]pyridazin-1-one.**

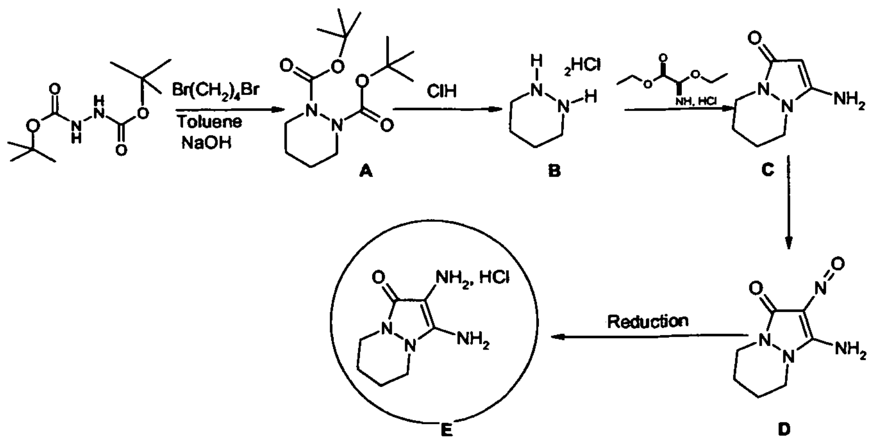


Synthèse du di-tert-butyl tetrahydropyridazine-1,2-dicarboxylate : **A**

Dans un tricol de 250 ml équipé d'un réfrigérant, d'un thermomètre et d'une ampoule de coulée, on introduit sous agitation mécanique 50 ml de toluène, 5 g (21,5 mmoles) de N,N'-di-tert-butoxycarbonylhydrazide, 680 mg de bromure de tétraéthylammonium et 25 ml de soude à 50 %.
Le milieu hétérogène est chauffé à 100 ˚C puis on ajoute goutte à goutte en 15 minutes le 1,4-dibromobutane.
Le milieu réactionnel est chauffé à 100 ˚C pendant 3 jours. Après refroidissement, on ajoute 100 ml d'acétate d'éthyle et on transfère dans une ampoule à décanter. La phase organique est lavée avec 4 fois 70 ml de solution aqueuse saturée en carbonate de sodium, puis avec 4 x 70 ml d'eau et enfin avec 4 x 70ml d'eau salée. La phase organique est séchée sur sulfate de sodium et le solvant est évaporé sous vide. On obtient ainsi une huile incolore qui cristallise en un solide blanc.
On récupère une masse de 6,1 g (rendement : 99 %).
Les spectres RMN ($^1$H 400 MHz et $^{13}$C 100,61 MHz DMSO $d_6$) et de masse sont conformes à la structure attendue **A**.

Synthèse du dichlorhydrate d'hexahydropyridazine : **B**

Dans un tricol de 100 ml équipé d'un réfrigérant et d'un thermomètre, on introduit sous agitation mécanique 5,9 g du composé **A** dans 50 ml de mélange 3 / 1 de dioxanne et d'acide chlorhydrique à 35 %.
La solution incolore obtenue est agitée à température ambiante pendant 3 heures puis le milieu réactionnel est dilué par de l'éther diisopropylique. Les solvants sont évaporés sous vide. Le résidu pâteux obtenu est repris par un mélange éther / éthanol. Après filtration du solide et séchage sous vide, on obtient 1,39 g de solide blanc.
Les spectres de RMN ($^1$H 400 MHz et $^{13}$C 100,61 MHz DMSO $d_6$) et de masse sont conformes à la structure attendue **B**.

Synthèse du 3-amino-5,6,7,8-tetrahydro-1H-pyrazolo[1,2-a]pyridazin-1-one : **C**

Dans un tricol de 25 ml équipé d'un réfrigérant et d'un thermomètre, on introduit sous agitation mécanique 7,5 ml d'éthanol, 1,5 ml de triéthylamine et 0,73 ml d'acide 3-amino-3-ethoxyacrylique. On ajoute ensuite 500 mg de dichlorhydrate d'hexahydropyridazine (composé **B**) et on agite pendant 3 heures à température ambiante.

On filtre l'insoluble et distille le solvant sous vide. Le solide est repris par le minimum d'eau, filtré et séché sous vide. On obtient ainsi 0,9 g de poudre légèrement jaune.

Les spectres de RMN ($^1$H 400 MHz et $^{13}$C 100,61 MHz DMSO $d_6$) et de masse sont conformes à la structure attendue **C**.

Synthèse du 3-amino-2-nitroso-5,6,7,8-tetrahydro-1H-pyrazolo[1,2-a]pyridazin-1-one : **D**

Dans un tricol de 50 ml équipé d'un réfrigérant et d'un thermomètre, on introduit sous agitation mécanique 20 ml d'acide chlorhydrique à 35 % et 1 g de 3-amino-5,6,7,8-tetrahydro-1H-pyrazolo[1,2-a]pyridazin-1-one (composé **C**). On refroidit à 0 °C et on coule une solution de 675 mg nitrite de sodium dans 5 ml d'eau en maintenant cette température. La couleur du mélange réactionnel vire du jaune à l'orange et un précipité commence à se former.
En 30 minutes, la réaction est terminée et le solide orange est filtré sur verre fritté n° 4, lavé à l'eau puis séché sous vide. Le rendement est de 78,3 %.
Les spectres de RMN ($^1$H 400 MHz et $^{13}$C 100,61 MHz DMSO $d_6$) et de masse sont conformes à la structure attendue **D**.

Synthèse du chlorhydrate de 2,3-diamino-5,6,7,8-tetrahydro-1H-pyrazolo[1,2-a]pyridazin-1-one : **E**

Dans un autoclave de 300 ml contenant 250 ml d 'éthanol, on introduit 1,3 g de 3-amino-2-nitroso-5,6,7,8-tetrahydro-1H-pyrazolo[1,2-a]pyridazin-1-one (composé **D**) et 250 mg de palladium sur charbon à 5 %. La réduction est réalisée sous une agitation de 2000 tr / min, à une température de 60 °C et sous une pression d'hydrogène de 6 bars. Au bout de 2 heures de réaction, il n'y a plus de consommation d'hydrogène et le milieu est refroidi à 20 °C.
Le catalyseur est éliminé par filtration sous azote après refroidissement à température ambiante et la solution est versée sur 75 ml de dioxanne chlorhydrique.

La solution ainsi obtenue est évaporée jusqu'à obtention d'une poudre légèrement jaune que l'on reprend dans de l'éther diisopropylique.
Le solide est récupéré par filtration. Après séchage sous vide en présence de pentaoxyde de phosphore, on obtient 1,1 g de dichlorhydrate de 2,3-diamino-5,6,7,8-tetrahydro-1H-pyrazolo[1,2-a]pyridazin-1-one.
Les spectres de RMN ($^1$H 400 MHz et $^{13}$C 100,61 MHz DMSO $d_6$) et de masse sont conformes à la structure attendue **E**.

**Exemple 7 : Synthèse du chlorhydrate de 4-Amino-1,2-diethyl-5-pyrrolidin-1-yl-1,2-dihydro-pyrazol-3-one**

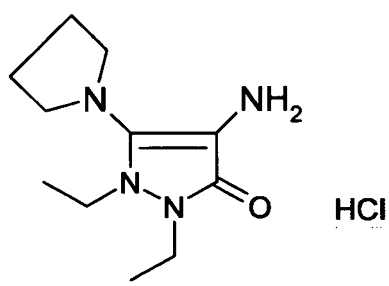

**Etape 1 : Synthèse de la 1,2-diéthylpyrazolidine-3,5-dione**

Dans un tricol de 3000 ml muni d'un thermomètre et sous agitation magnétique, on introduit successivement sous atmosphère d'azote 100 g du dichlorhydrate de diéthylhydrazine (0,63 moles) dans 1000 ml de dichlorométhane, 85,3 g d'acide malonique (0,82 moles ; 1,3 éq.), 196 g de hydroxybenzotriazole (1,45 moles ; 2,3 éq.), 278 g de 1-(3-diméthylaminopropyl)-3-ethyl-carbodiimide, hydrochlorure (EDCI ; 1,45 moles ; 2,3 éq.).

Le milieu réactionnel est ensuite refroidi entre 0 °C et 5 °C. On y additionne alors lentement 407 g de N, N-diisopropylethylamine (3,14 moles ; 520 ml : 5 éq.). A la fin de l'addition, le milieu réactionnel devenu homogène est laissé sous agitation à température ambiante. Après une nuit à température ambiante, la réaction est terminée.

Le milieu réactionnel est lavé par trois fois 600 ml d'eau permutée. La phase organique est séchée sur sulfate de sodium, filtrée et concentrée sous vide pour fournir 46g de produit brut. La pyrazolidine-dione étant soluble en milieu aqueux, la phase aqueuse est donc concentrée à sec puis reprise par 800 ml d'une solution d'acide chlorhydrique 1 N. Le précipité formé est filtré et la phase aqueuse est extraite par trois fois 1300 ml de dichlorométhane. Les phases

organiques rassemblées sont séchées sur sulfate de sodium, filtrées et concentrées sous vide pour fournir 67,5 g de poduit brut.

On obtient ainsi la **1,2-diéthylpyrazolidine-3**,5-dione sous forme de solide jaune avec un rendement de 40 % (39,5 g).

**Etape 2 : Synthèse de la 1,2-diéthyl-3-chloro-5-pyrazolone**

Dans un tricol de 500 ml équipé d'un réfrigérant et d'une agitation magnétique, sont introduits, sous atmosphère d'azote, 30 g de 1,2 g de 1,2-diéthylpyrazolidine-3,5-dione (0,19 moles) en solution dans 200 ml de toluène et 35,8 ml d'oxyde de trichlorophosphine (258,9 g ; 0,38 moles ; 2 éq.).

Le milieu réactionnel est porté au reflux du toluène et la réaction est suivie par CCM (dichlorométhane / méthanol 95 / 5). Le milieu réactionnel initialement sous forme de pâte, s'homogénéise dès le reflux puis devient biphasique.

Après une heure de reflux, la réaction est hydrolysée à 0 °C par addition très lente de 100 ml d'eau permutée. Après décantation, la phase toluène est séparée de la phase aqueuse. La phase aqueuse est lavée par 50 ml de toluène puis amenée à pH 12 par 184 ml d'une solution de soude à 35 %. On observe la formation d'un précipité. La phase aqueuse est portée à 100 °C pendant 10 minutes et le précipité se solubilise. Le milieu réactionnel présente alors deux phases. La phase supérieure de coloration brune est séparée après décantation à chaud. Cette phase supérieure est solubilisée par 200 ml de dichlorométhane, lavée par une fois 50 ml d'eau permutée, séchée sur sulfate de sodium et concentrée sous vide pour fournir 20,5 g d'une huile brune.

Un précipité se forme dans la phase aqueuse inférieure lors du retour à la température ambiante. Après filtration sur fritté, le précipité est rincé à l'eau et le filtrat est extrait par trois fois 300 ml de dichlorométhane. La phase dichlorométhane est séchée sur sulfate de sodium et concentrée sous vide pour fournir 5,5 g de cristaux bruns.

L'huile et les cristaux bruns sont rassemblés, greffés sur silice et chromatographiés sur gel de silice (40-60 µm ; 2000 g) par un gradient d'élution :

1) Dichlorométhane 100 (13 litres)
2) Dichlorométhane / MeOH 99,5 / 0,5 (0,8 litres)
3) Dichlorométhane / MeOH 99 / 1 (8 litres) produit attendu + 15 % impureté m = 6,6 g
4) Dichlorométhane / MeOH 98,5 / 1,5 (35 litres) produit attendu (14,7 g)

La **1,2-diéthyl-3-chloro-5-pyrazolone** est ainsi obtenue sous la forme de cristaux jaune avec un rendement de 44 %.

**Etape 3 : Synthèse du 1,2-Diethyl-5-pyrrolidin-1-yl-1,2-dihydro-pyrazol-3-one**

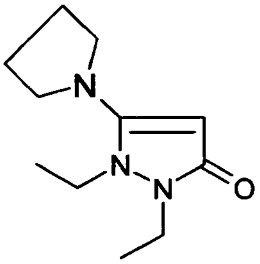

Dans un réacteur 2,5 ml du micro-onde Biotage initiator, on introduit 1 g de 5-chloro-1,2-diéthyl-1,2-dihydro-pyrazol-3-one ($5,7.10^{-4}$ moles), auquel on ajoute 2 ml de pyrrolidine (4,2 éq.).

Conditions opératoires : micro-onde à puissance maximum $\theta$ = 120 °C pendant 17 min.

Après 17 minutes, la réaction est terminée (suivi CCM éluant : 90 / 10 $CH_2Cl_2$ / MeOH).

5 ml d'eau déminéralisée sont alors ajoutés au milieu réactionnel, puis l'ensemble est transféré en ampoule à décanter. La phase aqueuse est extraite avec quatre fois 10 ml de dichlorométhane. Les phases organiques sont ensuite réunies et séchées sur du sulfate de sodium anhydre, puis filtrées et évaporées à sec. 1,2 grammes d'une huile orangée brune sont obtenus avec un rendement de 100 %.

RMN ($^1H$ 400 MHz DMSO $d_6$)
0,81 (1t, 3H), 0,89 (1t, 3H), 1,88 (1m, 1H), 3,22 (1m, 4H), 3,4 (1m, 4H), 4,4 (1s, 1H) Masse : Analyse faite en OpenLynx (FIA/MS).

La masse principalement détectée est en accord avec la structure attendue : M = 20

__Etape 4: Synthèse du 1,2-diéthyl-4-nitroso-5-pyrrolidin-1-yl-1,2-dihydro-3H-pyrazol-3-one__

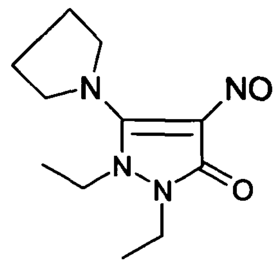

Dans un tricol de 25 ml tout équipé, on introduit 1,2 g de 1,2-diéthyl-5-pyrrolidin-1-yl-1,2-dihydro-pyrazol-3-one que l'on solubilise dans un mélange composé de 0,84 ml d'acide chlorhydrique 37 % et de 4 ml d'eau déminéralisée. Le milieu réactionnel est refroidit entre 0 ˚C et 5 ˚C, à l'aide d'un bain d'eau glacée. Une solution composée de 400 mg de nitrite de sodium ($5{,}7.10^{-4}$ moles) solubilisés dans 0,6 ml d'eau déminéralisée, est alors ajoutée au goutte à goutte. Le milieu réactionnel vire instantanément au rouge vif dès l'ajout de la première goutte du mélange précédent.

Après une heure, la réaction est terminée.
Le pH est ajusté à environ 7-8 à l'aide d'une solution de soude à 30 %, puis le milieu réactionnel est transféré en ampoule à décanter. La phase aqueuse est extraite avec 4 fois 10 ml de dichloromethane. Les phases organiques sont réunies et séchées sur sulfate de sodium anhydre puis évaporées à sec. 1,2 grammes d'une poudre bleu turquoise sont obtenus avec un rendement de 89,6 %.

Les spectres de RMN ($^1$H 400 MHz DMSO $d_6$) et de masse sont conformes à la structure attendue.
RMN ($^1$H 400 MHz DMSO $d_6$)
0,94 (1t, 3H), 1 (1t, 3H), 2,05 (1m, 4H), 3,51 (1q, 4H), 3,76 (1q, 4H), 3,94 (1m, 4H) Analyse faite en OpenLynx (FIA/MS).
La masse principalement détectée est en accord avec la structure attendue. M = 238.

__Etape 5 : Synthèse du chlorhydrate de 4-Amino-1,2-diéthyl-5-pyrrolidin-1-yl-1,2-dihydro-pyrazol-3-one :__

Dans un tricol de 500 ml tout équipé, on introduit 4 grammes de zinc en poudre (0,06 moles) dans 300 ml d'éthanol absolu auxquels on ajoute 1 ml d'acide acétique.
Le milieu réactionnel est chauffé à 40 ˚C, puis 1,15 g ($4{,}8.10^{-3}$ moles) de 1,2-diéthyl-4-nitroso-5-pyrrolidin-1-yl-1,2-dihydro-3H-pyrazol-3-one sont introduits par petites spatules. 4 ml d'acide acétique sont enfin introduits ml par ml et le milieu est porté au reflux. Le milieu est totalement soluble et incolore. Après 30 minutes, la réaction est terminée sur CCM selon l'éluant acétate d'éthyle / MeOH 90 / 10.

Le milieu réactionnel est refroidi puis filtré sur fritté contenant un lit de cellite 545. Les eaux mères sont filtrées dans un ballon contenant 2,5 ml d'isopropanol chlorhydrique 5 N refroidi. Le tout est ensuite évaporé à sec. Le produit obtenu est une poudre rose conforme par RMN et Masse.
RMN ($^1$H 400 MHz DMSO $d_6$)
0,79 (1t, 3H), 0,96 (1t, 3H), 1,87 (1m, 4H), 3,49 (1q, 2H), 3,59 (1m, 6H)
Analyse FIA/MS réalisée via OpenLynx.
Les ions quasi moléculaires [M+H]+, [M+Na]+, [2M+H]+, [2M+Na]+ de la base attendue $C_{11}H_{20}N_4O$ sont principalement détectés.

En reproduisant les étapes précédentes avec les réactifs appropriés, on peut obtenir le 4-amino-5-[3-(diméthylamino)pyrrolidin-1-yl]-1,2-diéthyl-1,2-dihydro-3H-pyrazol-3-one hydrochloride.

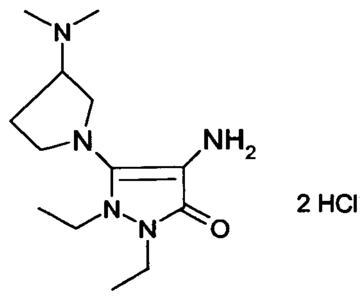

**Exemple 8 : synthèse du colorant comprenant deux chromophores de formule suivante :**

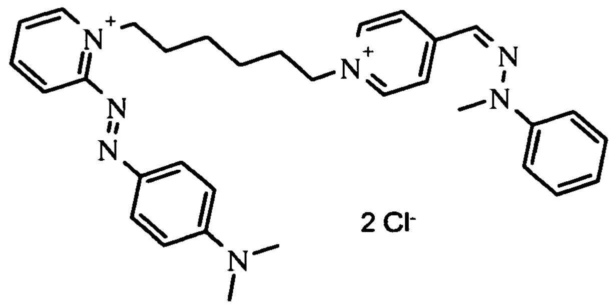

Schéma réactionnel :

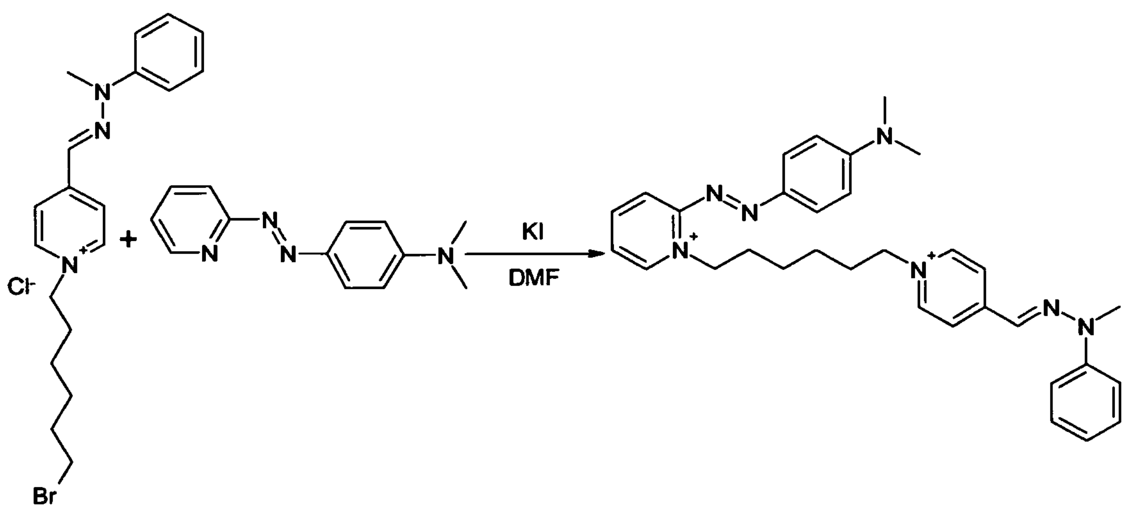


Procédé :

Dans un tricol on introduit 1,06 équivalents du colorant hydrazone (3 g) et environ 100 mg de KI, dans 2 ml de DMF, sous agitation et l'on chauffe à 95˚C.
On ajoute ensuite 1 équivalent du colorant azoïque (1,49 g) dissout dans 5ml de DMF et on laisse réagir pendant 24 heures.
On récupère le produit par précipitation dans l'acétate d'éthyle (50 ml), on filtre et on sèche le produit qui se présente

sous la forme d'une poudre noire.
Le produit est purifié par solubilisation dans le dichlorométhane, puis précipité dans un mélange contenant un mélange isopropanol / acétate d'éthyle (1 / 4) puis filtré.
Le spectre RMN $^{13}C$ et $^{1}H$ sont en accord avec la structure du produit attendu.

**EXEMPLES DE TEINTURE**

**Exemple 1 :**

Les compositions A, B et C suivantes ont été réalisées :

| | **Composition A** (invention) | **Composition B** (comparatif) | **Composition C** (comparatif) |
|---|---|---|---|
| 2,3-diamino-6,7-dihydro-1H,5H-pyrazolo[1,2-a] pyrazol-1-one, 2 HCl | $1.10^{-3}$ mole | $1,5.10^{-3}$ mole | - |
| 5,6 dihydroxyindoline, HBr | $1.10^{-3}$ mole | $1,5.10^{-3}$ mole | - |
| Colorant direct hétérocyclique de formule (XV) | $1.10^{-3}$ mole | - | $3.10^{-3}$ mole |
| Support de teinture commun n°1 | (**) | (**) | (**) |
| Eau déminéralisée q.s.p. | 100 g | 100 g | 100 g |

Colorant direct hétérocyclique de formule (XV) :

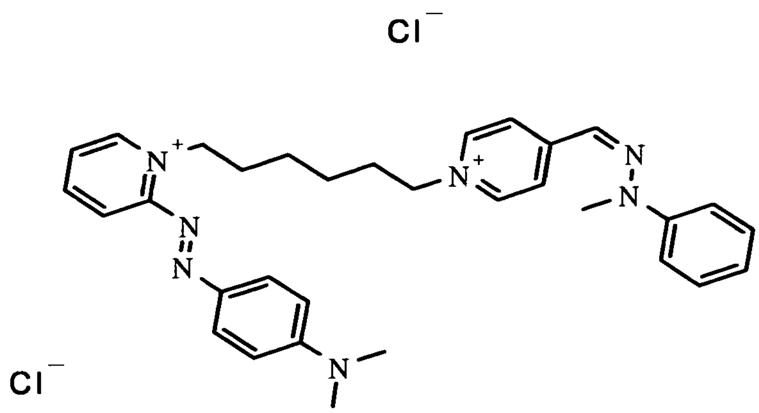

(**) support de teinture commun n° 1 :

| | |
|---|---|
| Alcool oléique polyglycérolé à 2 moles de glycérol | 4 g |
| Alcool oléique polyglycérolé à 4 moles de glycérol (78 % M.A.) | 5,69 g M.A. |
| Acide oléique | 3 g |
| Amine oléique 2 OE commercialisée sous la dénomination ETHOMEEN 012 par la société AKZO | 7 g |
| Laurylamino succinamate de diéthylaminopropyle, sel de sodium à 55 % M.A. | 3 g M.A. |
| Alcool oléique | 5 g |
| Diéthanolamide d'acide oléique | 12 g |
| Propylène glycol | 3,5 g |
| Alcool éthylique | 7 g |
| Dipropylène glycol | 0,5 g |
| Monométhyléther de propylène glycol | 9 g |

(suite)

| | |
|---|---|
| Métabisulfite de sodium en solution aqueuse à 35 % M.A. | 0,455 g |
| Acétate d'ammonium | 0,8 g |
| Antioxydant, séquestrant | q.s. |
| Ammoniaque à 20 % de $NH_3$ | 10 g |

**Mode d'application**

Au moment de l'emploi, les compositions A, B et C sont mélangées poids pour poids avec une solution de peroxyde d'hydrogène à 20 volumes. On obtient un pH final de 9,8.
Le mélange obtenu est appliqué sur des mèches de cheveux gris à 90 % de blancs naturels et des mèches de cheveux gris à 90 % de blancs décolorés à raison de 10 g de mélange pour 1 g de cheveux. Après 30 minutes de pose à température ambiante, les mèches sont rincées, lavées avec un shampooing standard, rincées à nouveau puis séchées.
La couleur des cheveux est mesurée à l'aide d'un spectrocolorimètre MINOLTA CM2002® (illuminant D65 - 10 ° CSI) dans le système CIELab. Dans ce système, L* représente la clarté, a* la teinte et b* la saturation.
ΔE*ab, qui représente la variation de couleur entre une mèche de cheveux naturels et une mèche de cheveux décolorés après coloration, est obtenu à l'aide de la formule suivante :

$$\Delta E = \sqrt{(L^* - L_o{}^*)^2 + (a^* - a_o{}^*)^2 + (b^* - b_o{}^*)^2}$$

dans laquelle L* représente la clarté, a* la teinte et b* la saturation de la mèche de cheveux naturel après coloration et $L_0$* représente la clarté, $a_0$* la teinte et $b_0$* la saturation $L_0$* de la mèche de cheveux décolorés après coloration. Plus la valeur de ΔE est faible, moins la coloration des cheveux est sélective.
Les résultats obtenus sont présentés dans le tableau ci-dessus.

| **Composition** | **ΔE** |
|---|---|
| A | 9,54 |
| B | 19,21 |
| C | 22,59 |

Les résultats ci-dessus montrent que la composition conforme à l'invention conduit à une coloration nettement moins sélective que les compositions de l'art antérieur.

**Exemple 2** :

La composition D suivante a été réalisée :

| | |
|---|---|
| Alcool oléique polyglycérolé à 2 moles de glycérol | **4 g** |
| Alcool oléique polyglycérolé à 4 moles de glycérol (78 % M.A.) | **5,69 g M.A.** |
| Acide oléique | **3 g** |
| *Amine oléique 2 OE commercialisée sous la dénomination ETHOMEEN 012 par la société AKZO | **7 g** |
| Laurylamino succinamate de diéthylaminopropyle, sel de sodium à 55 % M.A. | **3 g M.A.** |
| Alcool oléique | **5 g** |
| Diéthanolamide d'acide oléique | **12g** |
| Propylène glycol | **3,5 g** |
| Alcool éthylique | **7 g** |
| Dipropylène glycol | **0,5 g** |
| Monométhyléther de propylène glycol | **9 g** |
| Métabisulfite de sodium en solution aqueuse à 35 % M.A. | **0,455 g M.A.** |
| Acétate d'ammonium | **0,8 g** |

(suite)

| | |
|---|---|
| 2,3-diamino-6,7-dihydro-1H,5H-pyrazolo[1,2-a]pyrazol-1-one, 2 HCl | **1,36 g** |
| 2-méthyl 5-hydroxyéthylamino phénol | **1,00 g** |
| Basic Yellow 28 | **0,5 g** |
| Antioxydant, séquestrant | q.s. |
| Ammoniaque à 20 % de $NH_3$ | **10 g** |
| Eau déminéralisée | q.s.p **100 g** |

Basic Yellow 28 :

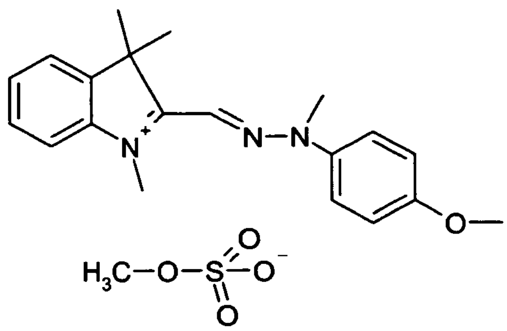

**Mode d'application**

Au moment de l'emploi, la composition D est mélangée poids pour poids avec une solution de peroxyde d'hydrogène à 20 volumes. On obtient un pH final de 9,8.
Le mélange obtenu est appliqué sur des mèches de cheveux gris à 90 % de blancs naturels et des mèches de cheveux gris à 90 % de blancs décolorés à raison de 10 g de mélange pour 1 g de cheveux. Après 30 minutes de pose à température ambiante, les mèches sont rincées, lavées avec un shampooing standard, rincées à nouveau puis séchées. On obtient un reflet cuivré-doré puissant, aussi bien sur cheveu naturel que décoloré.

**Revendications**

**1.** Composition de coloration des fibres kératiniques comprenant, dans un milieu approprié :

- au moins une base d'oxydation choisie parmi un dérivé de la diamino-N,N-dihydro-pyrazolone de formule (I) ou l'un de ses sels d'addition :

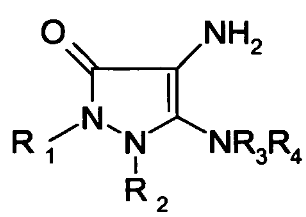

(I)

dans laquelle :

$R_1$, $R_2$, $R_3$ et $R_4$, identiques ou différents, représentent :

- un radical alkyle en $C_1$-$C_{10}$ linéaire ou ramifié éventuellement substitué par un ou plusieurs radicaux

choisis dans le groupe constitué par un radical $OR_5$, un radical $NR_6R_7$, un radical carboxy, un radical sulfonique, un radical carboxamido $CONR_6R_7$, un radical sulfonamido $SO_2NR_6R_7$, un hétéroaryle, un aryle éventuellement substitué par un ou plusieurs groupes ($C_1$-$C_4$)alkyle, hydroxy, alcoxy en $C_1$-$C_2$, amino, (di)alkyl($C_1$-$C_2$)amino ;
- un radical aryle éventuellement substitué par un ou plusieurs radicaux ($C_1$-$C_4$)alkyle, hydroxy, alcoxy en $C_1$-$C_2$, amino, (di)alkyl($C_1$-$C_2$)amino ;
- un radical hétéroaryle à 5 ou 6 chaînons, éventuellement substitué par un ou plusieurs radicaux choisis parmi un radical ($C_1$-$C_4$)alkyle, ($C_1$-$C_2$)alcoxy ;

$R_3$ et $R_4$ peuvent représenter également un atome d'hydrogène;
$R_5$, $R_6$ et $R_7$, identiques ou différents, représentent :

- un atome d'hydrogène ;
- un radical alkyle linéaire ou ramifié en $C_1$-$C_4$ éventuellement substitué par un ou plusieurs radicaux choisis parmi un radical hydroxy, alcoxy en $C_1$-$C_2$, carboxamido $CONR_8R_9$, sulfonyle $SO_2R_8$, aryle éventuellement substitué par un radical ($C_1$-$C_4$)alkyle, hydroxy, alcoxy en $C_1$-$C_2$, amino, (di)alkyl($C_1$-$C_2$) amino; aryle éventuellement substitué par un radical ($C_1$-$C_4$)alkyle, hydroxy, alcoxy en $C_1$-$C_2$, amino, (di)alkyl($C_1$-$C_2$)amino ;

$R_6$ et $R_7$, identiques ou différents, peuvent représenter également un radical carboxamido $CONR_8R_9$; un radical sulfonyle $SO_2R_8$;
$R_8$ et $R_9$, identiques ou différents, représentent un atome d'hydrogène ; un radical alkyle en $C_1$-$C_4$ linéaire ou ramifié éventuellement substitué par un ou plusieurs radicaux hydroxy, alcoxy en $C_1$-$C_2$ ;
$R_1$ et $R_2$ d'une part, et $R_3$ et $R_4$ d'autre part, peuvent former avec le ou les atomes d'azote auxquels ils sont rattachés un hétérocycle saturé ou insaturé, comportant 5 à 7 chaînons, éventuellement substitué par un ou plusieurs radicaux choisis dans le groupe constitué par les atomes d'halogène, les radicaux amino, (di)alkyl($C_1$-$C_4$)amino, hydroxy, carboxy, carboxamido, ($C_1$-$C_2$)alcoxy, les radicaux alkyle en $C_1$-$C_4$ éventuellement substitués par un ou plusieurs radicaux hydroxy, amino, (di)-alkylamino, alcoxy, carboxy, sulfonyle ;
$R_3$ et $R_4$ peuvent également former ensemble avec l'atome d'azote auquel ils sont rattachés un hétérocycle à 5 ou 7 chaînons dont les atomes de carbone peuvent être remplacés par un atome d'oxygène ou d'azote éventuellement substitué ; et

- au moins un coupleur ; et
- au moins un colorant direct hétérocyclique.

**2.** Composition selon la revendication 1 dans laquelle $R_1$ et $R_2$ sont choisis parmi un radical alkyle en $C_1$-$C_6$ éventuellement substitué par un radical hydroxy, ($C_1$-$C_2$)alcoxy, amino, (di)alkyl($C_1$-$C_2$)amino ; un radical phényle, méthoxyphényle, éthoxyphényle, benzyle.

**3.** Composition selon la revendication 2 dans laquelle $R_1$ et $R_2$ sont choisis parmi un radical méthyle, éthyle, 2-hydroxyéthyle, 3-hydroxypropyle, 2-hydroxypropyle, phényle.

**4.** Composition selon la revendication 1 dans laquelle $R_1$ et $R_2$ forment ensemble avec les atomes d'azote auxquels ils sont rattachés un cycle à 5 ou 6 chaînons, saturé ou insaturé, éventuellement substitué.

**5.** Composition selon l'une quelconque des revendications 1 et 4 dans laquelle $R_1$ et $R_2$ forment ensemble avec les atomes d'azote auxquels ils sont rattachés un cycle pyrazolidine, pyridazolidine, éventuellement substitué par un ou plusieurs radicaux alkyle en $C_1$-$C_4$, hydroxy, ($C_1$-$C_2$)alcoxy, carboxy, carboxamido, amino, (di)alkyl($C_1$-$C_2$)amino.

**6.** Composition selon l'une quelconque des revendications 1 et 4 à 5 dans laquelle $R_1$ et $R_2$ forment ensemble avec les atomes d'azote auxquels ils sont rattachés un cycle pyrazolidine, pyridazolidine.

**7.** Composition selon l'une quelconque des revendications précédentes dans laquelle $R_3$ et $R_4$ sont choisis parmi un atome d'hydrogène ; un radical alkyle en $C_1$-$C_6$, linéaire ou ramifié, éventuellement substitué par un ou plusieurs radicaux hydroxy, ($C_1$-$C_2$)alcoxy, amino, (di)alkyl($C_1$-$C_2$)amino ; un radical phényle éventuellement substitué par un ou plusieurs radicaux hydroxy, amino, ($C_1$-$C_2$)alcoxy.

**8.** Composition selon l'une quelconque des revendications 1 à 7 dans laquelle $R_3$ et $R_4$ sont choisis parmi un atome d'hydrogène, un radical méthyle, éthyle, isopropyle, 2-hydroxyéthyle, 3-hydroxypropyle, 2-hydroxypropyle, 2-carboxyéthyle.

**9.** Composition selon la revendication 8 dans laquelle $R_3$ et $R_4$ représentent un atome d'hydrogène.

**10.** Composition selon l'une quelconque des revendications 1 à 6 dans laquelle $R_3$ et $R_4$ forment ensemble avec l'atome d'azote auquel ils sont rattachés un cycle à 5 ou 7 chaînons choisi parmi les hétérocycles pyrrolidine, pipéridine, homopipéridine, pipérazine, homopipérazine ; lesdits cycles pouvant être substitués par un ou plusieurs radicaux hydroxy, amino, (di)alkyl($C_1$-$C_2$)amino, carboxy, carboxamido, alkyle en $C_1$-$C_4$ éventuellement substitué par un ou plusieurs radicaux hydroxy, amino, (di)alkylamino en $C_1$-$C_2$.

**11.** Composition selon l'une quelconque des revendications 1 à 6 et 10 dans laquelle $R_3$ et $R_4$ forment ensemble avec l'atome d'azote auquel ils sont rattachés un cycle à 5 ou 7 chaînons choisi parmi la pyrrolidine, le 2,5-diméthylpyrrolidine, l'acide pyrrolidine-2-carboxylique, l'acide 3-hydroxypyrrolidine-2-carboxylique, l'acide 4-hydroxypyrrolidine-2-carboxylique, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthylpyrrolidine, la 3-hydroxypyrrolidine, la 3,4-dihydroxypyrrolidine, la 3-aminopyrrolidine, la 3-méthylaminopyrrolidine, la 3-diméthylamino-pyrrolidine, la 4-amino-3-hydroxy pyrrolidine, la 3-hydroxy-4-(2-hydroxyéthyl)amino-pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, la homopipéridine, la 2-carboxyhomopipéridine, la 2-carboxamidohomopipéridine, l'homopipérazine, le N-méthyl-homopipérazine, le N-(2-hydroxyéthyl)-homopipérazine.

**12.** Composition selon l'une quelconque des revendications 1 à 6 et 10 à 11 dans laquelle $R_3$ et $R_4$ forment ensemble avec l'atome d'azote auquel ils sont rattachés un cycle à 5 ou 7 chaînons choisi parmi la pyrrolidine, la 3-hydroxypyrrolidine, la 3-aminopyrrolidine, la 3-diméthylamino-pyrrolidine, l'acide pyrrolidine-2-carboxylique, l'acide 3-hydroxypyrrolidine-2-carboxylique, la pipéridine, l'hydroxypipéridine, l'homopipéridine, le diazépane, la N-méthylhomopipérazine, la N β-hydroxyéthylhomopipérazine.

**13.** Composition selon l'une quelconque des revendications 1 à 6 et 10 à 12 dans laquelle $R_3$ et $R_4$ forment ensemble avec l'atome d'azote auquel ils sont rattachés un cycle à 5 chaînons tels que la pyrrolidine, la 3-hydroxypyrrolidine, la 3-aminopyrrolidine, la 3-diméthylamino-pyrrolidine.

**14.** Composition selon la revendication 1 à 13 dans laquelle le composé de formule (I) ou un de ses sels d'addition est choisi parmi :

2,3-Diamino-6,7-dihydro-1H,5H-pyrazolo[1,2-a]pyrazol-1-one ;
2-Amino-3-éthylamino-6,7-dihydro-1H,5H-pyrazolo[1,2-a]pyrazol-1-one ;
2-Amino-3-isopropylamino-6,7-dihydro-1H,5H-pyrazolo[1,2-a]pyrazol-1-one ;
2-Amino-3-(pyrrolidin-1-yl)-6,7-dihydro-1H,5H-pyrazolo[1,2-a]pyrazol-1-one ;
4,5-diamino-1,2-diméthyl-1,2-dihydro-pyrazol-3-one ;
4,5-diamino-1,2-diéthyl-1,2-dihydro-pyrazol-3-one ;
4,5-diamino-1,2-di-(2-hydroxyéthyl)-1,2-dihydro-pyrazol-3-one ;
2-amino-3-(2-hydroxyéthyl)amino-6,7-dihydro-1H,5H-pyrazolo[1,2-a]pyrazol-1-one ;
2-amino-3-diméthylamino-6,7-dihydro-1H,5H-pyrazolo[1,2-a]pyrazol-1-one ;
2,3-diamino-5,6,7,8-tétrahydro-1H,6H-pyridazino[1,2-a]pyrazol-1-one
4-Amino-1.2-diethyl-5-pyrrolidin-1-yl-1,2-dihydro-pyrazol-3-one ;
4-Amino-5-(3-dimethylamino-pyrrolidin-1-yl)-1,2-diethyl-1,2-dihydro-pyrazol-3-one ;
2,3-diamino-6-hydroxy-6,7-dihydro-1H,5H-pyrazolo[1,2-a]pyrazol-1-one.

**15.** Composition selon l'une quelconque des revendications précédentes dans laquelle le ou les coupleurs sont choisis parmi les méta-phénylènediamines, les méta-aminophénols, les méta-diphénols, les coupleurs naphtaléniques, les coupleurs hétérocycliques et leurs sels d'addition.

**16.** Composition selon la revendication 15 dans laquelle le ou les coupleurs sont choisis parmi le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

**17.** Composition selon l'une quelconque des revendications précédentes dans laquelle la quantité de chacun des coupleurs est comprise entre 0,001 et 10 % en poids du poids total de la composition tinctoriale.

**18.** Composition selon l'une quelconque des revendications précédentes dans laquelle le ou les colorants directs hétérocycliques présentent dans leur structure au moins un hétérocycle choisi parmi les cycles thiophène, thianthrène, furane, 1-4 pyrane, 1-2 pyrane, isobenzofurane, chromène, xanthène, 2Hpyrrole, pyrrole, imidazole, pyrazole, isothiazole, isoxazole, pyridine, pyrazine, pyrimidine, pyridazine, indolizine, isoindole, 3Hindole, indole, 1Hindazole, purine, 4Hquinolizine, isoquinoline, quinoline, phtalazine, naphtyridine, quinoxaline, cinnoline, ptéridine, carbazole, 4aHcarbazole , carboline, phénanthridine, acridine, périmidine, phénanthroline, phénazine, phénothiazine, furazane, phénoxazine, phenoxathiine, pyrrolidine, isochromane, chromane, pyrroline, imidazoline, imidazolidine, pyrazoline, pyrazolidine, morpholine, benzisoquinoline, imidazothiazole, benzothiazole, benzofurane, 1-2-3 triazole, 1-2-4 triazole, isoazole, 1-4 oxazine, o ou p isoxazine, 1-2-5 oxathiazine, 1-2-6 oxathiazine, 1-4-2 oxadiazine, 1-3-5-2 oxadiazine, 3 isopyrrole, indène, isoindène, indoline, isoindoline, 1-2-3-4 oxatriazole, 1-2-3-5 oxatriazole, pipérazine, pipéridine, 1-3-5 triazine, 1-2-4 triazine, 1-2-3 triazine, 1-3-2 benzoxazine, 1-4-2 benzoxazine, isocoumarine, 1-2-3 dioxazole, 1-2-4 dioxazole, 1-3-2 dioxazole, 1-3-4 dioxazole, 1-2-5 oxathiazole, 1-3 oxathiole, 1-2-3-4 tétrahydro quinoxaline , quinazoline, pyrazolotriazole, thiazole, indolénine, éventuellement substitués par un ou plusieurs substituants et leurs homologues incluant un ou plusieurs groupements carbonyle.

**19.** Composition selon la revendication 18 dans laquelle le ou les substituants sont choisis parmi les radicaux alkyle linéaire ou ramifié en $C_1$-$C_{10}$, amino, hydroxy, halogène, alcényle linéaire ou ramifié en $C_2$-$C_{10}$, mono ou polyhydroxyalkyle en $C_1$-$C_{10}$, mono ou polyaminoalkyle en $C_1$-$C_{10}$, mono ou dialkyl($C_1$-$C_6$)amino, mono ou dihydroxyalkyl ($C_1$-$C_6$)amino, monoalkyl($C_1$-$C_6$)monohydroxyalkyl($C_1$-$C_6$)amino, mono ou dialkyl($C_1$-$C_6$)aminoalkyle($C_1$-$C_{10}$), mono ou dihydroxyalkyl($C_1$-$C_6$)aminoalkyle($C_1$-$C_{10}$), monoalkyl($C_1$-$C_6$)monohydroxyalkyl($C_1$-$C_6$)aminoalkyle($C_1$-$C_{10}$), nitro, carboxyle, carboxyalkyle($C_1$-$C_{10}$), alcoxy($C_1$-$C_6$)carbonyle, sulfo, sulfoalkyle($C_1$-$C_{10}$), alcoxy($C_1$-$C_{10}$), uréido, trialkyl($C_1$-$C_6$)ammonium, trialkyl($C_1$-$C_6$)ammoniumalkyle($C_1$-$C_{10}$), aryle($C_6$-$C_{30}$) éventuellement substitué.

**20.** Composition selon l'une quelconque des revendications précédentes dans laquelle le colorant direct hétérocyclique est neutre.

**21.** Composition selon la revendication 20 dans laquelle le colorant direct hétérocyclique neutre est choisi parmi ceux comportant au moins un cycle choisi parmi les cycles pyridine, quinoxaline, pyrazoline, pyrazole, oxadiazole, thiazole, pyrrole, indole, pyrazolotriazole, quinoline, indoline, phénazine, coumarine, benzopyrane et leurs homologues incluant un ou plusieurs groupements carbonyle.

**22.** Composition selon la revendication 21 dans laquelle le colorant direct hétérocyclique neutre est choisi parmi la 2,5 diamino 6 nitropyridine, la 5 amino 2(2'hydroxyéthyl)amino 6 nitropyridine, la 2 amino 5(2'hydroxyéthyl)amino 6 nitropyridine, la 5 amino 2 éthylamino 6 nitropyridine, la 2 éthylamino 5 (2'hydroxyéthyl)amino 6 nitropyridine, la 2 méthylamino 5(2'hydroxyéthyl)amino 6 nitropyridine, la 1,2,3,4 tétrahydro 6 nitroquinoxaline, la 2,3 indolinedione , le Vat Blue 6, le Disperse Yellow 184, la brasiline et l'hématoxyline.

**23.** Composition selon l'une quelconque des revendications 1 à 19 dans laquelle le colorant direct hétérocyclique est anionique.

**24.** Composition selon la revendication 23 dans laquelle le colorant direct hétérocyclique anionique est choisi parmi ceux comportant au moins un cycle pyrazole, xanthène ,quinoline, benzotriazole, benzoquinoline, indoline, naphtotriazole, et leurs homologues incluant un ou plusieurs groupements carbonyle.

**25.** Composition selon la revendication 24 dans laquelle le colorant direct hétérocyclique anionique est choisi parmi l'Acid Yellow 23, l'Acid Yellow 73, l'Acid Red 92, l'Acid Yellow 3, le Food Yellow 4, l'Acid Red 51, l'Acid Red 52, l'Acid Red 87, l'Acid Red 95, l'Acid Red 92, l'Acid Blue 74, l'Acid Red 195, l'Acid Orange 92, l'Acid Yellow 5, l'Acid Black 70, le Direct Yellow 106, le Direct Yellow 59, l'Acid Yellow 14.

**26.** Composition selon l'une quelconque des revendications 1 à 19 dans laquelle le colorant direct hétérocyclique est cationique.

**27.** Composition selon la revendication 26 dans laquelle le colorant direct hétérocyclique cationique possède au moins une charge cationique appartenant à un hétérocycle.

**28.** Composition selon la revendication 26 ou 27 dans laquelle le colorant direct hétérocyclique est choisi parmi les colorants cationiques à cycles xanthèniques, les colorants cationiques à cycles acridiniques, les colorants cationiques à cycles benzothiazoles, les colorants cationiques à cycles phénothiazines, les colorants cationiques à cycles pyrazoles, les colorants cationiques à cycles triazoles, les colorants cationiques à cycles thiazoles, les colorants cationiques à cycles phénazines, les colorants cationiques à cycles indolénines, les colorants cationiques à cycles phénoxazines, les colorants cationiques à cycles imidazoles, les colorants cationiques à cycles pyridines et leurs homologues incluant un ou plusieurs groupements carbonyle.

**29.** Composition selon la revendication 28 dans laquelle le colorant direct hétérocyclique cationique est choisi parmi les colorants suivants :

le Basic Red 1, le Basic Red 3, le Basic Red 4, le Basic Violet 10 et le Basic Violet 11.
le Basic Orange 15, le Basic Orange 16, le Basic Orange 17.
le Basic Blue 41 et le Basic Blue 67.
le Basic Blue 9.
le Basic Yellow 57.
le Basic Red 22 et le Basic Red 46.
le Basic Red 29.
le Basic Red 2.
le Basic Red 14, le Basic Yellow 13 , le Basic Yellow 28 et le Basic Yellow 29.
le Basic Blue 6.
le Basic Red 51 et le Basic Orange 31.
le Basic Yellow 87.

**30.** Composition selon l'une quelconque des revendications précédentes dans laquelle le ou les colorants directs hétérocycliques sont chacun généralement présents en quantité comprise entre 0,0001 et 30 % en poids par rapport au poids total de la composition tinctoriale.

**31.** Composition selon l'une quelconque des revendications précédentes comprenant une base d'oxydation additionnelle choisie parmi les para-phénylènediamines, les bis-phénylalkylènediamines, les para-aminophénols, les bis-para-aminophénols, les ortho-aminophénols, les ortho-phénylènediamines, les bases hétérocycliques différentes des dérivés de formule (I) tels que définis dans l'une quelconque des revendications 1 à 14 et leurs sels d'addition, ainsi que leurs mélanges.

**32.** Composition selon l'une quelconque des revendications précédentes dans laquelle la quantité de chacune des bases d'oxydation est comprise entre 0,001 et 10 % en poids du poids total de la composition tinctoriale.

**33.** Composition selon l'une quelconque des revendications précédentes comprenant de plus un agent oxydant.

**34.** Procédé de teinture des fibres kératiniques, **caractérisé en ce qu'**une composition telle que définie dans l'une quelconque des revendications 1 à 32 est appliquée sur les fibres kératiniques en présence d'un agent oxydant pendant un temps suffisant pour développer la coloration désirée.

**35.** Procédé selon la revendication 34 dans lequel l'agent oxydant est choisi parmi le peroxyde d'hydrogène, le peroxyde d'urée, les bromates de métaux alcalins, les persels, les peracides et les enzymes oxydases.

**36.** Dispositif à plusieurs compartiments dans lequel un premier compartiment contient une composition tinctoriale telle que définie dans l'une quelconque des revendications 1 à 32 et un deuxième compartiment contient un agent oxydant.

**37.** Utilisation pour la teinture d'oxydation des fibres kératiniques d'une composition telle que définie dans l'une quelconque des revendications 1 à 33.

## Claims

**1.** Composition for dyeing keratin fibres, comprising, in a suitable medium:

- at least one oxidation base chosen from a diamino-N,N-dihydropyrazolone derivative of formula (I), or an addition salt thereof:

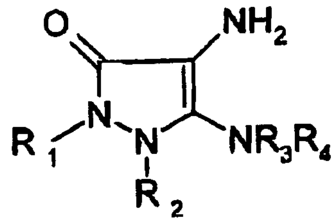

(I)

in which:

$R_1$, $R_2$, $R_3$ and $R_4$, which may be identical or different, represent:

- a linear or branched $C_1$-$C_{10}$ alkyl radical optionally substituted with one or more radicals chosen from the group consisting of a radical $OR_5$, a radical $NR_6R_7$, a carboxyl radical, a sulfonic radical, a carboxamido radical $CONR_6R_7$, a sulfonamido radical $SO_2NR_6R_7$, a heteroaryl or an aryl optionally substituted with one or more ($C_1$-$C_4$) alkyl, hydroxyl, $C_1$-$C_2$ alkoxy, amino or (di) ($C_1$-$C_2$)alkylamino groups;
- an aryl radical optionally substituted with one or more ($C_1$-$C_4$) alkyl, hydroxyl, $C_1$-$C_2$ alkoxy, amino or (di) ($C_1$-$C_2$) alkylamino radicals;
- a 5- or 6-membered heteroaryl radical, optionally substituted with one or more radicals chosen from a ($C_1$-$C_4$) alkyl or ($C_1$-$C_2$) alkoxy radical;

$R_3$ and $R_4$ may also represent a hydrogen atom;
$R_5$, $R_6$ and $R_7$, which may be identical or different, represent:

- a hydrogen atom;
- a linear or branched $C_1$-$C_4$ alkyl radical optionally substituted with one or more radicals chosen from a hydroxyl, $C_1$-$C_2$ alkoxy, carboxamido $CONR_8R_9$, sulfonyl $SO_2R_8$ or aryl radical optionally substituted with a ($C_1$-$C_4$) alkyl, hydroxyl, $C_1$-$C_2$ alkoxy, amino or (di) ($C_1$-$C_2$) alkylamino radical; aryl optionally substituted with a ($C_1$-$C_9$) alkyl, hydroxyl, $C_1$-$C_2$ alkoxy, amino or (di)($C_1$-$C_2$)alkylamino radical;

$R_6$ and $R_7$, which may be identical or different, may also represent a carboxamido radical $CONR_8R_9$; a sulfonyl radical $SO_2R_8$;
$R_8$ and $R_9$, which may be identical or different, represent a hydrogen atom; a linear or branched $C_1$-$C_4$ alkyl radical optionally substituted with one or more hydroxyl or $C_1$-$C_2$ alkoxy radicals;
$R_1$ and $R_2$, on the one hand, and $R_3$ and $R_4$, on the other hand, may form, with the nitrogen atom(s) to which they are attached, a saturated or unsaturated 5- to 7-membered heterocycle optionally substituted with one or more radicals chosen from the group consisting of halogen atoms, amino, (di) ($C_1$-$C_4$)alkylamino, hydroxyl, carboxyl, carboxamido or ($C_1$-$C_2$)alkoxy radicals, or $C_1$-$C_4$ alkyl radicals optionally substituted with one or more hydroxyl, amino, (di)alkylamino, alkoxy, carboxyl or sulfonyl radicals;
$R_3$ and $R_4$ may also form, together with the nitrogen atom to which they are attached, a 5- or 7-membered heterocycle, the carbon atoms of which may be replaced with an oxygen or optionally substituted nitrogen atom; and

- at least one coupler; and
- at least one heterocyclic direct dye.

**2.** Composition according to Claim 1, in which $R_1$ and $R_2$ are chosen from a $C_1$-$C_6$ alkyl radical optionally substituted with a hydroxyl, ($C_1$-$C_2$) alkoxy, amino or (di)($C_1$-$C_2$)alkylamino radical; a phenyl, methoxyphenyl, ethoxyphenyl or

benzyl radical.

3. Composition according to Claim 2, in which $R_1$ and $R_2$ are chosen from methyl, ethyl, 2-hydroxyethyl, 3-hydroxypropyl, 2-hydroxypropyl and phenyl radicals.

4. Composition according to Claim 1, in which $R_1$ and $R_2$ form, together with the nitrogen atoms to which they are attached, a saturated or unsaturated, optionally substituted 5- or 6-membered ring.

5. Composition according to either of Claims 1 and 4, in which $R_1$ and $R_2$ form, together with the nitrogen atoms to which they are attached, a pyrazolidine or pyridazolidine ring, optionally substituted with one or more $C_1$-$C_4$ alkyl, hydroxyl, ($C_1$-$C_2$)alkoxy, carboxyl, carboxamido, amino or (di)($C_1$-$C_2$)alkylamino radicals.

6. Composition according to any one of Claims 1, 4 and 5, in which $R_1$ and $R_2$ form, together with the nitrogen atoms to which they are attached, a pyrazolidine or pyridazolidine ring.

7. Composition according to any one of the preceding claims, in which $R_3$ and $R_4$ are chosen from a hydrogen atom; a linear or branched $C_1$-$C_6$ alkyl radical optionally substituted with one or more hydroxyl, ($C_1$-$C_2$) alkoxy, amino or (di) ($C_1$-$C_2$) alkylamino radicals; a phenyl radical optionally substituted with one or more hydroxyl, amino or ($C_1$-$C_2$) alkoxy radicals.

8. Composition according to any one of Claims 1 to 7, in which $R_3$ and $R_4$ are chosen from a hydrogen atom and a methyl, ethyl, isopropyl, 2-hydroxyethyl, 3-hydroxypropyl, 2-hydroxypropyl or 2-carboxyethyl radical.

9. Composition according to Claim 8, in which $R_3$ and $R_4$ represent a hydrogen atom.

10. Composition according to any one of Claims 1 to 6, in which $R_3$ and $R_4$ form, together with the nitrogen atom to which they are attached, a 5- or 7-membered ring chosen from pyrrolidine, piperidine, homopiperidine, piperazine and homopiperazine heterocycles; the said rings possibly being substituted with one or more hydroxyl, amino, (di) ($C_1$-$C_2$)alkylamino, carboxyl, carboxamido or $C_1$-$C_4$ alkyl radicals optionally substituted with one or more hydroxyl, amino or $C_1$-$C_2$ (di)alkylamino radicals.

11. Composition according to any one of Claims 1 to 6 and 10, in which $R_3$ and $R_4$ form, together with the nitrogen atom to which they are attached, a 5- or 7-membered ring chosen from pyrrolidine, 2,5-dimethyl-pyrrolidine, pyrrolidine-2-carboxylic acid, 3-hydroxypyrrolidine-2-carboxylic acid, 4-hydroxypyrrolidine-2-carboxylic acid, 2,4-dicarboxypyrrolidine, 3-hydroxy2-hydroxymethylpyrrolidine, 2-carboxamidopyrrolidine, 3-hydroxy-2-carboxamidopyrrolidine, 2-(diethylcarboxamido)pyrrolidine, 2-hydroxymethylpyrrolidine, 3,4-dihydroxy-2-hydroxymethylpyrrolidine, 3-hydroxypyrrolidine, 3,4-dihydroxypyrrolidine, 3-aminopyrrolidine, 3-methylaminopyrrolidine, 3-dimethylaminopyrrolidine, 4-amino-3-hydroxypyrrolidine, 3-hydroxy-4-(2-hydroxyethyl)-aminopyrrolidine, piperidine, 2,6-dimethylpiperidine, 2-carboxypiperidine, 2-carboxamidopiperidine, 2-hydroxymethylpiperidine, 3-hydroxy-2-hydroxymethylpiperidine, 3-hydroxypiperidine, 4-hydroxypiperidine, 3-hydroxymethylpiperidine, homopiperidine, 2-carboxyhomopiperidine, 2-carboxamidohomopiperidine, homopiperazine, N-methylhomopiperazine and N-(2-hydroxyethyl)homopiperazine.

12. Composition according to any one of Claims 1 to 6, 10 and 11, in which $R_3$ and $R_4$ form, together with the nitrogen atom to which they are attached, a 5- or 7-membered ring chosen from pyrrolidine, 3-hydroxypyrrolidine, 3-aminopyrrolidine, 3-dimethylaminopyrrolidine, pyrrolidine-2-carboxylic acid, 3-hydroxypyrrolidine-2-carboxylic acid, piperidine, hydroxypiperidine, homopiperidine, diazepane, N-methylhomopiperazine and N-β-hydroxyethylhomopiperazine.

13. Composition according to any one of Claims 1 to 6 and 10 to 12, in which $R_3$ and $R_4$ form, together with the nitrogen atom to which they are attached, a 5-membered ring such as pyrrolidine, 3-hydroxypyrrolidine, 3-aminopyrrolidine or 3-dimethylaminopyrrolidine.

14. Composition according to Claims 1 to 13, in which the compound of formula (I), or an addition salt thereof, is chosen from:

2,3-diamino-6,7-dihydro-1H,5H-pyrazolo[1,2-a]pyrazol-1-one;
2-amino-3-ethylamino-6,7-dihydro-1H,5H-pyrazolo-[1,2-a]pyrazol-1-one;

2-amino-3-isopropylamino-6,7-dihydro-1H,5H-pyrazolo-[1,2-a]pyrazol-1-one;
2-amino-3- (pyrrolidin-1-yl)-6,7-dihydro-1H,5H-pyrazolo-[1,2-a]pyrazol-1-one;
4,5-diamino-1,2-dimethyl-1,2-dihydropyrazol-3-one;
4,5-diamino-1,2-diethyl-1,2-dihydropyrazol-3-one;
4,5-diamino-1,2-di-(2-hydroxyethyl)-1,2-dihydropyrazol-3-one;
2-amino-3-(2-hydroxyethyl)amino-6,7-dihydro-1H,5H-pyrazolo[1,2-a]pyrazol-1-one;
2-amino-3-dimethylamino-6,7-dihydro-1H,5H-pyrazolo-[1,2-a]pyrazol-1-one;
2,3-diamino-5,6,7,8-tetrahydro-1H,6H-pyridazino-[1,2-a]pyrazol-1-one;
4-amino-1,2-diethyl-5-pyrrolidin-1-yl-1,2-dihydropyrazol-3-one;
4-amino-5-(3-dimethylaminopyrrolidin-1-yl)-1,2-diethyl-1,2-dihydropyrazol-3-one;
2,3-diamino-6-hydroxy-6,7-dihydro-1H,5H-pyrazolo-[1,2-a]pyrazol-1-one.

**15.** Composition according to any one of the preceding claims, in which the coupler(s) is(are) chosen from meta-phenylenediamines, meta-aminophenols, metadiphenols, naphthalene-based couplers and heterocyclic couplers, and the addition salts thereof.

**16.** Composition according to Claim 15, in which the coupler(s) is(are) chosen from 2-methyl-5-aminophenol, 5-N-(β-hydroxyethyl)amino-2-methylphenol, 6-chloro-2-methyl-5-aminophenol, 3-aminophenol, 1,3-dihydroxybenzene, 1,3-dihydroxy-2-methylbenzene, 4-chloro-1,3-dihydroxybenzene, 2,4-diamino-l-(p-hydroxyethyloxy)-benzene, 2-amino-4-(β-hydroxyethylamino)-1-methoxybenzene, 1,3-diaminobenzene, 1,3-bis(2,4-diaminophenoxy)propane, 3-ureidoaniline, 3-ureido-1-dimethylaminobenzene, sesamol, 1-p-hydroxyethylamino-3,4-methylenedioxybenzene, α-naphthol, 2-methyl-1-naphthol, 6-hydroxyindole, 4-hydroxyindole, 4-hydroxy-N-methylindole, 2-amino-3-hydroxypyridine, 6-hydroxybenzomorpholine, 3,5-diamino-2,6-dimethoxypyridine, 1-N-(β-hydroxyethyl)amino-3,4-methylenedioxybenzene and 2,6-bis(β-hydroxyethylamino)toluene, and the addition salts thereof with an acid.

**17.** Composition according to any one of the preceding claims, in which the amount of each of the couplers is between 0.001% and 10% by weight relative to the total weight of the dye composition.

**18.** Composition according to any one of the preceding claims, in which the heterocyclic direct dye(s) contain(s) in its (their) structure at least one heterocycle chosen from thiophene, thianthrene, furan, 1,4-pyran, 1,2-pyran, isobenzofuran, chromene, xanthene, 2H-pyrrole, pyrrole, imidazole, pyrazole, isothiazole, isoxazole, pyridine, pyrazine, pyrimidine, pyridazine, indolizine, isoindole, 3H-indole, indole, 1H-indazole, purine, 4H-quinolizine, isoquinoline, quinoline, phthalazine, naphthyridine, quinoxaline, cinnoline, pteridine, carbazole, 4a,H-carbazole, carboline, phenanthridine, acridine, perimidine, phenanthroline, phenazine, phenothiazine, furazan, phenoxazine, phenoxathine, pyrrolidine, isochroman, chroman, pyrroline, imidazoline, imidazolidine, pyrazoline, pyrazolidine, morpholine, benzisoquinoline, imidazothiazole, benzothiazole, benzofuran, 1,2,3-triazole, 1,2,4-triazole, isoazole, 1,4-oxazine, o- or p-isoxazine, 1,2,5-oxathiazine, 1,2,6-oxathiazine, 1,4,2-oxadiazine, 1,3,5,2-oxadiazine, 3-isopyrrole, indene, isoindene, indoline, isoindoline, 1,2,3,4-oxatriazole, 1,2,3,5-oxatriazole, piperazine, piperidine, 1,3,5-triazine, 1,2,4-triazine, 1,2,3-triazine, 1,3,2-benzoxazine, 1,4,2-benzoxazine, isocoumarin, 1,2,3-dioxazole, 1,2,4-dioxazole, 1,3,2-dioxazole, 1,3,4-dioxazole, 1,2,5-oxathiazole, 1,3-oxathiol, 1,2,3,4-tetrahydroquinoxaline, quinazoline, pyrazolotriazole, thiazole and indolenine rings, optionally substituted with one or more substituents, and homologues thereof including one or more carbonyl groups.

**19.** Composition according to Claim 18, in which the substituent(s) is(are) chosen from optionally substituted linear or branched $C_1$-$C_{10}$ alkyl, amino, hydroxyl, halogen, linear or branched $C_2$-$C_{10}$ alkenyl, $C_1$-$C_{10}$ mono- or polyhydroxyalkyl, $C_1$-$C_{10}$ mono- or polyaminoalkyl, mono- or di($C_1$-$C_6$) alkylamino, mono- or dihydroxy ($C_1$-$C_6$) alkylamino, mono ($C_1$-$C_6$) alkylmonohydroxy ($C_1$-$C_6$) alkylamino, mono- or di ($C_1$-$C_6$) alkylamino ($C_1$-$C_{10}$) alkyl, mono- or dihydroxy ($C_1$-$C_6$) alkylamino-($C_1$-$C_{10}$) alkyl, mono ($C_1$-$C_6$) alkylmonohydroxy ($C_1$-$C_6$) alkylamino ($C_1$-$C_{10}$) alkyl, nitro, carboxyl, carboxy ($C_1$-$C_{10}$) - alkyl, ($C_1$-$C_6$) alkoxycarbonyl, sulfo, sulfo ($C_1$-$C_{10}$) alkyl, ($C_1$-$C_{10}$) alkoxy, ureido, tri ($C_1$-$C_6$) alkylammonium, tri ($C_1$-$C_6$) alkylammonium ($C_1$-$C_{10}$) alkyl and ($C_6$-$C_{30}$) aryl radicals.

**20.** Composition according to any one of the preceding claims, in which the heterocyclic direct dye is neutral.

**21.** Composition according to Claim 20, in which the neutral heterocyclic direct dye is chosen from those comprising at least one ring chosen from pyridine, quinoxaline, pyrazoline, pyrazole, oxadiazole, thiazole, pyrrole, indole, pyrazolotriazole, quinoline, indoline, phenazine, coumarin and benzopyran rings, and homologues thereof including one or more carbonyl groups.

**22.** Composition according to Claim 21, in which the neutral heterocyclic direct dye is chosen from 2,5-diamino-6-nitropyridine, 5-amino-2-(2'-hydroxyethyl)-amino-6-nitropyridine, 2-amino-5-(2'-hydroxyethyl)-amino-6-nitropyridine, 5-amino-2-ethylamino-6-nitropyridine, 2-ethylamino-5-(2'-hydroxyethyl)amino-6-nitropyridine, 2-methylamino-5-(2'-hydroxyethyl)amino-6-nitropyridine, 1,2,3,4-tetrahydro-6-nitroquinoxaline, 2,3-indolinedione, Vat Blue 6, Disperse Yellow 184, brasiline and hematoxylin.

**23.** Composition according to any one of Claims 1 to 19, in which the heterocyclic direct dye is anionic.

**24.** Composition according to Claim 23, in which the anionic heterocyclic direct dye is chosen from those comprising at least one pyrazole, xanthene, quinoline, benzotriazole, benzoquinoline, indoline or naphthotriazole ring, and homologues thereof including one or more carbonyl groups.

**25.** Composition according to Claim 24, in which the anionic heterocyclic direct dye is chosen from Acid Yellow 23, Acid Yellow 73, Acid Red 92, Acid Yellow 3, Food Yellow 4, Acid Red 51, Acid Red 52, Acid Red 87, Acid Red 95, Acid Red 92, Acid Blue 74, Acid Red 195, Acid Orange 92, Acid Yellow 5, Acid Black 70, Direct Yellow 106, Direct Yellow 59, Acid Yellow 14.

**26.** Composition according to any one of Claims 1 to 19, in which the heterocyclic direct dye is cationic.

**27.** Composition according to Claim 26, in which the cationic heterocyclic direct dye has at least one cationic charge belonging to a heterocycle.

**28.** Composition according to Claim 26 or 27, in which the heterocyclic direct dye is chosen from cationic dyes containing xanthene rings, cationic dyes containing acridene rings, cationic dyes containing benzothiazole rings, cationic dyes containing phenothiazine rings, cationic dyes containing pyrazole rings, cationic dyes containing triazole rings, cationic dyes containing thiazole rings, cationic dyes containing phenazine rings, cationic dyes containing indolenine rings, cationic dyes containing phenoxazine rings, cationic dyes containing imidazole rings, cationic dyes containing pyridine rings, and homologues thereof including one or more carbonyl groups.

**29.** Composition according to Claim 28, in which the cationic heterocyclic direct dye is chosen from the following dyes:

Basic Red 1, Basic Red 3, Basic Red 4, Basic Violet 10
and Basic Violet 11,
Basic Orange 15, Basic Orange 16 and Basic Orange 17,
Basic Blue 41 and Basic Blue 67,
Basic Blue 9,
Basic Yellow 57,
Basic Red 22 and Basic Red 46,
Basic Red 29,
Basic Red 2,
Basic Red 14, Basic Yellow 13, Basic Yellow 28 and
Basic Yellow 29,
Basic Blue 6,
Basic Red 51 and Basic Orange 31,
Basic Yellow 87.

**30.** Composition according to any one of the preceding claims, in which the heterocyclic direct dye(s) is(are) each generally present in an amount of between 0.0001% and 30% by weight relative to the total weight of the dye composition.

**31.** Composition according to any one of the preceding claims, comprising an additional oxidation base chosen from para-phenylenediamines, bis(phenyl)alkylenediamines, para-aminophenols, bis-para-aminophenols, ortho-aminophenols, ortho-phenylenediamines, heterocyclic bases other than the derivatives of formula (I) as defined in any one of Claims 1 to 14, and the addition salts thereof, and also mixtures thereof.

**32.** Composition according to any one of the preceding claims, in which the amount of each of the oxidation bases is between 0.001% and 10% by weight relative to the total weight of the dye composition.

**33.** Composition according to any one of the preceding claims, also comprising an oxidizing agent.

**34.** Process for dyeing keratin fibres, **characterized in that** a composition as defined in any one of Claims 1 to 32 is applied to the keratin fibres in the presence of an oxidizing agent for a time that is sufficient to develop the desired coloration.

**35.** Process according to Claim 34, in which the oxidizing agent is chosen from hydrogen peroxide, urea peroxide, alkali metal bromates, persalts, peracids and oxidase enzymes.

**36.** Multi-compartment device, in which a first compartment contains a dye composition as defined in any one of Claims 1 to 32 and a second compartment contains an oxidizing agent.

**37.** Use for the oxidation dyeing of keratin fibres of a composition as defined in any one of Claims 1 to 33.

## Patentansprüche

**1.** Zusammensetzung zum Färben von Keratinfasern, die in einem geeigneten Medium enthält:

o mindestens eine Oxidationsbase, die unter einem Diamino-N,N-dihydro-pyrazolonderivat der Formel (I) oder einem seiner Additionssalze ausgewählt ist:

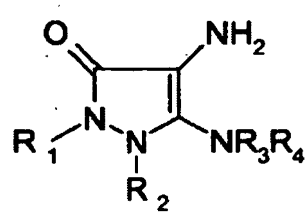

(I)

in der Formel:

$R_1$, $R_2$, $R_3$ et $R_4$, die gleich oder verschieden sind, bedeuten:

- eine geradkettige oder verzweigte $C_{1}$-$_{10}$-Alkylgruppe, die gegebenenfalls mit einer oder mehreren Gruppen substituiert ist, die unter einer Gruppe $OR_5$, einer Gruppe $NR_6R_7$, einer Carboxygruppe, einer Sulfonsäuregruppe, einer Carboxamidogruppe $CONR_6R_7$, einer Sulfonamidogruppe $SO_2NR_6R_7$, einer Heteroarylgruppe und einer Arylgruppe ausgewählt sind, die gegebenenfalls mit einer oder mehreren Gruppen ($C_1$-$_4$)-Alkyl, Hydroxy, ($C_1$-$_2$)Alkoxy, Amino, (Di)alkyl($C_{1-2}$)amino substituiert ist;
- eine Arylgruppe, die gegebenenfalls mit einer oder mehreren Gruppen ($C_{1-4}$)Alkyl, Hydroxy, ($C_{1-2}$) Alkoxy, Amino, (Di)alkyl($C_{1-2}$)amino substituiert ist;
- eine 5- oder 6-gliedrige Heteroarylgruppe, die gegebenenfalls mit einer oder mehreren Gruppen substituiert ist, die unter ($C_{1-4}$)Alkyl und ($C_{1-2}$)Alkoxy ausgewählt sind;

$R_3$ et $R_4$ können auch ein Wasserstoffatom bedeuten;
$R_5$, $R_6$ et $R_7$, die gleich oder verschieden sind, bedeuten:

- ein Wasserstoffatom;
- eine geradkettige oder verzweigte $C_{1-4}$-Alkylgruppe, die gegebenenfalls mit einer oder mehreren Gruppen substituiert ist, die unter Hydroxy, ($C_{1-2}$)Alkoxy, Carboxamido $CONR_8R_9$, Sulfonyl $SO_2R_8$ und einer Arylgruppe ausgewählt sind, die gegebenenfalls mit ($C_{1-4}$)Alkyl, Hydroxy, ($C_{1-2}$)Alkoxy, Amino, (Di)alkyl($C_{1-2}$)amino substituiert ist; eine Arylgruppe, die gegebenenfalls mit ($C_{1-4}$)Alkyl, Hydroxy, ($C_{1-2}$) Alkoxy, Amino, (Di)alkyl($C_{1-2}$)amino substituiert ist;

$R_6$ et $R_7$, die gleich oder verschieden sind, können auch eine Carboxamidogruppe $CONR_8R_9$; eine Sulfo-

nylgruppe $SO_2R_8$ bedeuten;

$R_8$ et $R_9$, die gleich oder verschieden sind, bedeuten ein Wasserstoffatom; eine geradkettige oder verzweigte $C_{1-4}$-Alkylgruppe, die gegebenenfalls mit einer oder mehreren Gruppen Hydroxy, $C_{1-2}$-Alkoxy substituiert ist;

$R_1$ und $R_2$ einerseits und $R_3$ et $R_4$ andererseits können mit dem oder den Stickstoffatom(en), an die sie gebunden sind, einen gesättigten oder ungesättigten, 5- bis 7-gliedrigen Heterocyclus bilden, der gegebenenfalls mit einer oder mehreren Gruppen substituiert ist, die unter den Halogenatomen, den Gruppen Amino, (Di)alkyl($C_{1-4}$)amino, Hydroxy, Carboxy, Carboxamido, ($C_{1-2}$)-Alkoxy und den $C_{1-4}$-Alkylgruppen ausgewählt sind, die gegebenenfalls mit einer oder mehreren Gruppen Hydroxy, Amino, (Di)alkylamino, Alkoxy, Carboxy, Sulfonyl substituiert sind;

$R_3$ et $R_4$ können auch gemeinsam mit dem Stickstoffatom, an das sie gebunden sind, einen 5- oder 7-gliedrigen Heterocyclus bilden, bei dem die Kohlenstoffatome durch ein Sauerstoffatom oder ein Stickstoffatom ersetzt sein können, das gegebenenfalls substituiert ist ; und

o mindestens einen Kuppler; und

o mindestens einen heterocyclischen Direktfarbstoff.

**2.** Zusammensetzung nach Anspruch 1, bei der die Gruppen $R_1$ et $R_2$ unter einer Alkyl($C_{1-6}$)gruppe, die gegebenenfalls mit Hydroxy, ($C_{1-2}$)Alkoxy, Amino, (Di)alkyl($C_{1-2}$)amino substituiert ist; einer Phenylgruppe, einer Methoxyphenylgruppe, einer Ethoxyphenygruppe, einer Benzylgruppe ausgewählt sind.

**3.** Zusammensetzung nach Anspruch 2, bei der die Gruppen $R_1$ et $R_2$ unter Methyl, Ethyl, 2-Hydroxyethyl, 3-Hydroxypropyl, 2-Hydroxypropyl, Phenyl ausgewählt sind.

**4.** Zusammensetzung nach Anspruch 1, bei der die Gruppen $R_1$ et $R_2$ gemeinsam mit den Stickstoffatomen, an die sie gebunden, sind, einen 5- oder 6-gliedrigen, gesättigten oder ungesättigten, gegebenenfalls substituierten Ring bilden.

**5.** Zusammensetzung nach einem der Ansprüche 1 bis 4, bei der die Gruppen $R_1$ et $R_2$ gemeinsam mit den Stickstoffatomen, an die sie gebunden sind, einen Pyrazolidinring oder Pyridazolidinririg bilden, wobei diese Ringe gegebenenfalls mit einer oder mehreren Gruppen ($C_{1-4}$)Alkyl, Hydroxy, ($C_{1-2}$)Alkoxy, Carboxy, Carboxamido, Amino, (Di)alkyl($C_{1-2}$)amino substituiert sein können.

**6.** Zusammensetzung nach einem der Ansprüche 1 und 4 bis 5, bei der die Gruppen $R_1$ et $R_2$ gemeinsam mit den Stickstoffatomen, an die sie gebunden sind, einen Pyrazolidinring oder Pyridazolidinring bilden.

**7.** Zusammensetzung nach einem der vorhergehenden Ansprüche, bei der die Gruppen $R_3$ et $R_4$ unter einem Wasserstoffatom; einer geradkettigen oder verzweigten Alkyl($C_{1-6}$)gruppe, die gegebenenfalls mit einer oder mehreren Gruppen Hydroxy, ($C_{1-2}$) Alkoxy, Amino, (Di)alkyl($C_{1-2}$)amino substituiert ist; einer Phenylgruppe, die gegebenenfalls mit einer oder mehreren Gruppen Hydroxy, Amino, ($C_{1-2}$)Alkoxy substituiert ist, ausgewählt sind.

**8.** Zusammensetzung nach einem der Ansprüche 1 bis 7, bei der die Gruppen $R_3$ et $R_4$ unter einem Wasserstoffatom, Methyl, Ethyl, Isopropyl, 2-Hydroxyethyl, 3-Hydroxypropyl, 2-Hydroxypropyl, 2-Carboxyethyl ausgewählt sind.

**9.** Zusammensetzung nach Anspruch 8, bei der die Gruppen $R_3$ et $R_4$ ein Wasserstoffatom bedeuten.

**10.** Zusammensetzung nach einem der Ansprüche 1 bis 6, bei der die Gruppen $R_3$ et $R_4$ gemeinsam mit dem Stickstoffatom, an das sie gebunden sind, einen 5- oder 7-gliedrigen Ring bilden, der unter den Heterocyclen Pyrrolidin, Piperidin, Homopiperidin, Piperazin, Homopiperazin ausgewählt ist; wobei diese Ringe gegebenenfalls substituiert sein können mit einer oder mehreren Gruppen Hydroxy, Amino, (Di)alkyl($C_{1-2}$)amino, Carboxy, Carboxamido, ($C_{1-4}$) Alkyl, wobei diese Gruppe gegebenenfalls mit einer oder mehreren Gruppen Hydroxy, Amino, (Di)alkyl($C_{1-2}$)amino substituiert sein kann.

**11.** Zusammensetzung nach einem der Ansprüche 1 bis 6 und 10, bei der die Gruppen $R_3$ et $R_4$ gemeinsam mit dem Stickstoffatom, an das sie gebunden sind, einen 5- oder 7-gliedrigen Ring bilden, der unter Pyrrolidin, 2,5-Dimethylpyrrolidin, Pyrrolidin-2-carbonsäure, 3-Hydroxypyrrolidin-2-carbonsäure, 4-Hydroxypyrrolidin-2-carbonsäure, 2,4-Dicarboxypyrrolidin, 3-Hydroxy-2-hydroxymethylpyrrolidin, 2-Carboxamidopyrrolidin, 3-Hydroxy-2-carboxamidopyrrolidin, 2-(Diethylcarboxamido)pyrrolidin, 2-Hydroxymethyl-pyrrolidin, 3,4-Dihydroxy-2-hydroxymethylpyrrolidin, 3-Hydroxypyrrolidin, 3,4-Dihydroxypyrrolidin, 3-Aminopyrrolidin, 3-Methylaminopyrrolidin, 3-Dimethylaminopyrrolidin, 4-Amino-3-hydtoxypyrrolidin, 3-Hydroxy-4-(2-hydroxyethyl)aminopyrrolidin, Piperidin, 2,6-Dimethylpiperidin, 2-Carboxypiperidin, 2-Carboxamidopiperidin, 2-Hydroxymethylpiperidin, 3-Hydroxy-2-hydroxymethylpiperidin, 3-Hydroxypiperidin, 4-Hydroxypiperidin, 3-Hydroxymethylpiperidin, Homopiperidin, 2-Carboxyhomopiperidin, 2-Carboxamidohomopiperidin, Homopiperazin, N-Methyl-homopiperazin, N-(2-Hydroxyethyl)-homopiperazin ausgewählt ist.

**12.** Zusammensetzung nach einem der Ansprüche 1 bis 6 und 10 bis 11, bei der die Gruppen $R_3$ et $R_4$ gemeinsam mit dem Stickstoffatom, an das sie gebunden sind, einen 5- oder 7-gliedrigen Ring bilden, der unter Pyrrolidin, 3-Hydroxypyrrolidin, 3-Aminopyrrolidin, 3-Dimethylamino-pyrrolidin, Pyrrolidin-2-carbonsäure, 3-Hydroxypyrrolidin-2-carbonsäure, Piperidin, Hydroxypiperidin, Homopiperidin, Diazepan, N-Methylhomopiperazin, N-β-Hydroxyethylhomopiperazin ausgewählt ist.

**13.** Zusammensetzung nach einem der Ansprüche. 1 bis 6 und 10 bis 12, bei der die Gruppen' $R_3$ et $R_4$ gemeinsam mit dem Stickstoffatom, an das sie gebunden sind, einen 5-gliedrigen Ring bilden, wie Pyrrolidin, 3-Hydroxypyrrolidin, 3-Aminopyrrolidin, 3-Dimethylaminopyrrolidin.

**14.** Zusammensetzung nach Anspruch 1 bis 13, bei der die Verbindung der Formel (I) oder eines ihrer Additionssalze ausgewählt ist unter:

2,3-Diamino-6,7-dihydro-1H,5H-pyrazolo[1,2-a]pyrazol-1-on;
2-Amino-3-methylamino-6,7-dihydro-1H,5H-pyrazolo[1,2-a]-pyrazol-1-on;
2-Amino-3-isopropylamino-6,7-dihydro-1H,5H-pyrazolo[1,2-a]-pyrazol-1-on;
2-Amino-3-(pyrrolidin-1-yl)-6,7-dihydro-1H,5H-pyrazolo[1,2-a]-pyrazol-1-on;
4,5-Diamino-1,2-dimethyl-1,2-dihydro-pyrazol-3-on;
4,5-Diamino-1,2-diethyl-1,2-dihydro-pyrazol-3-on;
4,5-Diamino-1,2-di-(2-hydroxyethyl)-1,2-dihydro-pyrazol-3-on;
2-Amino-3-(2-hydroxyethyl)amino-6,7-dihydro-1H,5H-pyrazolo[ 1,2-a]pyrazol-1-on;
2-Amino-3-dimethylamino-6,7-dihydro-1H,5H-pyrazolo[ 1,2-a]-pyrazol-1-on;
2,3-Diamino-5,6,7,8-tetrahydro-1H,6H-pyridazino[ 1,2-a]pyrazol-1-on;
4-Amino-1,2-diethyl-5-(pyrrolidin-1-yl)-1,2-dihydro-pyrazol-3-on;
4-Amino-5-(3-dimethylamino-pyrrolidin-1-yl)-1,2-diethyl-1,2-dihydro-pyrazol-3-on;
2,3-Diamino-6-hydroxy-6,7-dihydro-1H,5H-pyrazolo [ 1,2-a]-pyrazol-1-on.

**15.** Zusammensetzung nach einem der vorhergehenden Ansprüche, wobei der oder die Kuppler unter den meta-Phenylendiaminen, meta-Aminophenolen, meta-Dihydroxybenzolen, Naphthalinkupplern, heterocyclischen Kupplern und deren Additionssalzen ausgewählt sind.

**16.** Zusammensetzung nach Anspruch 15, wobei der oder die Kuppler unter 2-Methyl-5-amino-phenol, 5-N-(β-Hydroxyethyl)-amino-2-methyl-phenol, 6-Chlor-2-methyl-5-aminophenol, 3-- Amino-phenol, 1,3-Dihydroxy-benzol, 1,3-Dihydroxy-2-methylbenzol, 4-Chlor-1,3-dihydroxy-benzol, 2,4-Diamino-1-(β-hydroxyethylamino)-benzol, 2-Amino-4-(β-hydroxyethylamino)-1-methoxy-benzol, 1,3-Diamino-benzol, 1,3-Bis(2,4-diaminophenoxy)-propan, 3-Ureidoanilin, 3-Ureido-1-dimethylaminobenzol, Sesamol, 1-β-Hydroxyethylamino-3,4-methylendioxy-benzol, α-Naphthol, 2-Methyl-1-naphthol, 6-Hydroxy-indol, 4-Hydroxy-indol, 4-Hydroxy-N-methyl-indol, 2-Amino-3-hydroxy-pyridin, 6-Hydroxy-benzomorpholin, 3,5-Diamino-2,6-dimethoxypyridin, 1-N-(β-Hydroxyethyl)amino-3,4-methylendioxy-benzol, 2,6-Bis(β-hydroxyethylamino)-toluol und deren Additionssalzen mit einer Säure ausgewählt sind.

**17.** Zusammensetzung nach einem der vorhergehenden-Ansprüche, wobei der Mengenanteil jedes Kupplers im Bereich von 0,001 bis 10 Gew.-% des Gesamtgewichts der Farbmittelzusammensetzung, liegt.

**18.** Zusammensetzung nach einem der vorhergehenden Ansprüche, wobei der oder die heterocyclischen Direktfarbstoffe in ihrer Struktur mindestens einen Heterocyclus aufweisen, der unter den Ringen Thiophen, Thianthren, Furan, 1-4-Pyran, 1-2-Pyran, Isobenzofuran, Chromen, Xanthen, 2H-Pyrrol, Pyrrol, Imidazol, Pyrazol, Isothiazol, Isoxazol, Pyridin, Pyrazin, Pyrimidin, Pyridazin, Indolizin, Isoindol, 3H-Indol, Indol, 1H-Indazol, Purin, 4H-Chinolizin, Isochinolin, Chinolin, Phthalazin, Naphthyridin, Chinoxalin, Cinnolin, Pteridin, Carbazol, 4aHCarbazol, Carbolin, Phenanthridin, Acridin, Perimidin, Phenanthrolin, Phenazin, Phenothiazin, Furazan, Phenoxazin, Phenoxathiin, Pyrrolidin, Isochroman, Chroman, Pyrrolin, Imidazolin, Imidazolidin, Pyrazolin, Pyrazolidin, Morpholin, Benzisochinolin, Imidazothiazol, Benzothiazol, Benzofuran, 1-2-3-Triazol, 1-2-4-Triazol, Isoazol, 1-4-Oxazin, o- oder p-Isoxazin, 1-2-5-Oxathiazin, 1-2-6-Oxathiazin, 1-4-2-Oxadiazin, 1-3-5-2-Oxadiazin, 3-Isopyrrol, Inden, Isoinden, Indolin, Isoindolin, 1-2-3-4-Oxatriazol, 1-2-3-5-Oxatriazol, Piperazin, Piperidin, 1-3-5-Triazin, 1-2-4-Triazin, 1-2-3-Triazin, 1-3-2-Benzoxazin, 1-4-2-Benzoxazin, Isocumarin, 1-2-3-Dioxazol, 1-2-4-Dioxazol, 1-3-2-Dioxazol, 1-3-4-Dioxazol, 1-2-5-Oxathiazol, 1-3-Oxathiol, 1-2-3-4-Tetrahydro-chinoxalin, Chinazolin, Pyrazolotriazol, Thiazol, Indolenin, die gegebenenfalls mit einem oder mehreren Substituenten substituiert sind, und deren Homologen, die eine oder mehrere Carbonylgruppen aufweisen.

**19.** Zusammensetzung nach Anspruch 18, wobei der oder die Substituenten ausgewählt sind unter: geradkettigen oder verzweigten Alkylgruppen mit 1 bis 10 Kohlenstoffatomen, Amino, Hydroxy, Halogen, geradkettigen oder verzweigten Alkenylgruppen mit 2 bis 10 Kohlenstoffatomen, Mono- oder Polyhydroxyalkylgruppen mit 1 bis 10 Kohlenstoffatomen, Mono-oder Polyaminoalkylgrup-pen mit 1 bis 10 Kohlenstoffatomen, Mono- oder Dialkyl($C_{1-6}$)-amino, Mono-oder Dihydroxyalkyl($C_{1-6}$) amino, Monoalkyl($C_{1-6}$)-monohydroxyalkyl($C_{1-6}$)amino, Mono-oder Dialkyl($C_{1-6}$)aminoalkyl($C_{1-10}$), Mono- oder Dihydroxyalkyl($C_{1-6}$)aminoalkyl($C_{1-10}$), Monoalkyl($C_{1-6}$) monohydroxyalkyl($C_{1-6}$)aminoalkyl ($C_{1-10}$), Nitro, Carboxy, Carboxyalkyl($C_{1-10}$), Alkoxy($C_{1-6}$)carbonyl, Sulfo, Sulfoalkyl($C_{1-10}$), Alkoxy($C_{1-10}$), Ureido, Trialkyl($C_{1-6}$)ammonium, Trialkyl($C_{1-6}$) ammoniumalkyl($C_{1-10}$), Aryl($C_{6-30}$), gegebenenfalls substituiert.

**20.** Zusammensetzung nach einem der vorhergehenden Ansprüche, wobei der heterocyclische Direktfarbstoff neutral ist.

**21.** Zusammensetzung nach Anspruch 20, wobei der neutrale heterocyclische Direktfarbstoff unter den Farbstoffen ausgewählt ist, die mindestens einen Ring enthalten, der ausgewählt ist unter: Pyridin, Chinoxalin, Pyrazolin, Pyrazol, Oxadiazol, Thiazol, Pyrrol, Indol, Pyrazolotriazol, Chinolin, Indolin, Phenazin, Cumarin, Benzopyran und deren Homologen, die eine oder mehrere Carbonylgruppen aufweisen.

**22.** Zusammensetzung nach dem Anspruch 21, wobei der neutrale heterocyclische Direktfarbstoff ausgewählt ist unter: 2,5-Diamino-6-nitropyridin, 5-Amino-2-(2'-hydroxyethyl)amino-6-nitropyridin; 2-Amino-5-(2'-hydroxyethyl)amino-6-nitropyridin, 5-Amino-2-ethylamino-6-nitropyridin, 2-Ethylamino-5-(2'-hydroxyethyl)amino-6-nitropyridin, 2-Methylamino-5-(2'-hydroxyethyl)amino-6-nitropyridin, 1,2,3,4-Tetrahydro-6-nitrochinoxalin, 2,3-Indolindion, Vat Blue 6, Disperse Yellow 184, Brasilin und Hämatoxylin.

**23.** Zusammensetzung nach einem der Ansprüche 1 bis 19, wobei der heterocyclische Direktfarbstoff anionisch ist.

**24.** Zusammensetzung nach dem Anspruch 23, wobei der anionische heterocyclische Direktfarbstoff unter den Verbindungen ausgewählt ist, die mindestens einen Ring Pyrazol, Xanthen, Chinolin, Berizotriazol, Benzochinolin, Indolin, Naphthotriazol und deren Homologe, die eine oder mehrere Carbonylgruppen aufweisen, enthalten.

**25.** Zusammensetzung nach dem Anspruch 23, wobei der anionische heterocyclische Direktfarbstoff ausgewählt ist unter: Acid Yellow 23, Acid Yellow 73, Acid Red 92, Acid Yellow 3, Food Yellow 4, Acid Red 51, Acid Red 52, Acid Red 87, Acid Red 95, Acid Red 92, Acid Blue 74, Acid Red 195, Acid Orange 92, Acid Yellow 5, Acid Black 70, Direct Yellow 106, Direct Yellow 59, Acid Yellow 14.

**26.** Zusammensetzung nach einem der Ansprüche 1 bis 19, wobei der heterocyclische Direktfarbstoff kationisch ist.

**27.** Zusammensetzung nach dem Anspruch 26, wobei der kationische heterocyclische Direktfarbstoff mindestens eine kationische Ladung an mindestens einem Heterocyclus aufweist.

**28.** Zusammensetzung nach dem Anspruch 26 oder 27, wobei der kationische heterocyclische Direktfarbstoff unter den kationischen Farbstoffen mit Xanthenringen, kationischen Farbstoffen mit Acridinringen, kationischen Farbstoffen mit Benzothiazolringen, kationischen Farbstoffen mit Phenothiazinringen, kationischen Farbstoffen mit Pyrazolringen, kationischen Farbstoffen mit Triazolringen, kationischen Farbstoffen mit Thiazolringen, kationischen Farbstoffen mit Phehazinringen, kationischen Farbstoffen mit Indoleninringen, kationischen Farbstoffen mit Phenoxazinringen, kationischen Farbstoffen mit Imidazolringen, kationischen Farbstoffen mit Pyridinringen und deren Homologen, die eine oder mehrere Carbonylgruppen aufweisen, enthalten.

**29.** Zusammensetzung nach dem Anspruch 28, wobei der kationische heterocyclische Direktfarbstoff unter den folgenden Direktfarbstoffen ausgewählt ist:

Basic Red 1, Basic Red 3, Basic Red 4, Basic Violet 10 und Basic Violet 11;
Basic Orange 15, Basic Orange 16, Basic Orange 17;

Basic Blue 41 und Basic Blue 67;
Basic Blue 9;
Basic Yellow 57;
Basic Red 22 und Basic Red 46;
Basic Red 29;
Basic Red 2;
Basic Red 14, Basic Yellow 13 , Basic Yellow 28 und Basic Yellow 29;
Basic Blue 6;
Basic Red 51 und Basic Orange 31;
Basic Yellow 87.

**30.** Zusammensetzung nach einem der vorhergehenden Ansprüche, bei der der oder die heterocyclische(n) Direktfarbstoff(e) jeder im Allgemeinen in einem Mengenanteil im Bereich von 0,0001 bis 30 Gew.-%, bezogen auf das Gesamtgewicht der Farbmittelzusammensetzung, vorliegen.

**31.** Zusammensetzung nach einem der vorhergehenden Ansprüche, die eine ergänzende Oxidationsbase enthält, die unter den p-Phenylendiaminen, Bisphenylalkylendiaminen, p-Aminophenolen, Bis-p-aminophenolen, o-Aminophenolen, o-Phenylendiaminen, heterocyclischen Basen, die von den Derivaten der Formel (I), wie sie in einem der Ansprüche 1 bis 14 definiert sind, und deren Additionssalzen verschieden sind, und deren Gemischen ausgewählt ist.

**32.** Zusammensetzung nach einem der vorhergehenden Ansprüche, bei der der Mengenteil jeder Oxidationsbase im Bereich von 0,001 bis 10 Gew.-% des Gesamtgewichts der Farbmittelzusammensetzung liegt.

**33.** Zusammensetzung nach einem der vorhergehenden Ansprüche, die ferner ein Oxidationsmittel enthält.

**34.** Verfahren zum Färben von Keratinfasern, **dadurch gekennzeichnet, dass** eine Zusammensetzung nach einem der Ansprüche 1 bis 32 in Gegenwart eines Oxidationsmittels während einer Zeitspanne, die ausreichend ist, um die gewünschte Farbe zu bilden, auf die Keratinfasern aufgebracht wird.

**35.** Verfahren nach Anspruch 34, wobei das Oxidationsmittel unter Wasserstoffperoxid, Harnstoffperoxid, Alkalimetallbromaten, Salzen von Persäuren, Persäuren und Oxidasen ausgewählt ist.

**36.** Vorrichtung mit mehreren Abteilungen, wobei eine erste Abteilung eine Farbmittelzusammensetzung enthält, wie sie in einem der Ansprüche 1 bis 32 definiert ist, und eine zweite Abteilung ein Oxidationsmittel enthält.

**37.** Verwendung einer Zusammensetzung nach einem der Ansprüche 1 bis 33 zum oxidativen Färben von Keratinfasern.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- DE 3825212
- DE 4404564
- FR 2845387
- FR 2746392
- DE 19730412
- WO 200222093 A
- DE 10118271
- FR 2760010
- FR 2978931
- DE 20104441
- EP 984010 A
- EP 1025834 A
- EP 714954 A
- WO 9501772 A
- WO 9515144 A
- EP 1170000 A
- EP 1166753 A
- EP 1166754 A
- EP 1170001 A
- GB 1026978 A
- GB 1153196 A
- FR 2801308
- DE 2359399
- JP 63169571 A
- JP 5063124 A
- EP 0770375 A
- WO 9615765 A
- FR 2750048 A
- DE 3843892
- DE 4133957
- WO 9408969 A
- WO 9408970 A
- FR 2733749 A
- DE 19543988
- FR 2586913
- US 4128425 A
- US 2841584 A
- DE 4234885


### Littérature non-brevet citée dans la description


- *J. Het. Chem.,* 2001, vol. 38 (3), 613-616
- *Helvetica Chimica Acta,* 1950, vol. 33, 1183-1194
- *J.Org.Chem.,* 1958, vol. 23, 2029
- *J.Am.Chem.Soc.,* 1951, vol. 73, 3240
- *J.Am.Chem.Soc.,* 1962, vol. 84, 590
- *Justus Liebig Ann.Chem.,* 1965, vol. 686, 134
- *Tetrahedron. Lett.,* 1973, vol. 31, 2859-2862
- **J. March.** Advanced Organic Chemistry. Willey Interscience, 1985
- **M . Hudlicky.** Reduction in organic Chemistry. Ellis Horwood Series Chemical Science, 1983
- **M. Hudlicky.** Reduction in organic Chemistry. Ellis Horwood Series Chemical Science, 1983